# EUROPÄISCHE PATENTSCHRIFT

(11) **EP 2 481 762 B1**
(45) Veröffentlichungstag und Bekanntmachung des Hinweises auf die Patenterteilung: **31.12.2014**
(21) Anmeldenummer: 12161487.9
(22) Anmeldetag: 25.08.2010
(51) Int. Cl.: C08F 220/28, C08L 33/14, D06M 13/46, D06M 15/27, D06M 15/29, D06M 23/00, C08F 220/38, D06M 15/263

(54) **Wiederbeladbare Ausrütungen für Textielen**
Reloadable Finishes for Textiles
Equipements rechargeables pour textiles

(30) Priorität: 08.09.2009 CH 13912009
(43) Veröffentlichungstag der Anmeldung: 01.08.2012
(62) Teilanmeldung aus: 10747028.8
(73) Patentinhaber: Schoeller Textil AG, 9475 Sevelen (CH)
(72) Erfinder: Holzdörfer, Uwe, "verstorben" (CH); Gaupp, Theo, 7204 Untervaz (CH); Lottenbach, Roland, 9422 Staad (CH); Hübner, Hans-Jürgen, 6645 Brione s.M. (CH)
(74) Vertreter: Rentsch Partner AG

(56) Entgegenhaltungen:
- DE-A1- 19 608 910
- US-A- 3 459 716
- US-A- 3 705 053

## Beschreibung

### Technisches Gebiet

Die Erfindung betrifft Polymerverbindungen und Ausrüstungsformulierungen zur Ausrüstung von textilen Produkten, und die entsprechenden Ausrüstungsschichten und textilen Produkte.

### Stand der Technik

Aus dem Stand der Technik ist eine Vielzahl von Ausrüstungen von textilen Produkten bekannt, um Textilien mit zusätzlichen funktionellen Eigenschaften zu versehen. Unter dem Begriff "Textilien" und "textile Produkte" versteht man dabei insbesondere sowohl Fasern als auch fertige Textilprodukte (beispielsweise Gewebe, Gewirke, Vliese etc.), die beispielsweise als Stoff oder als bereits verarbeitetes Produkt (z. B. Kleidungsstück) vorliegen können. Die Textilien können aus allen beliebigen, bekannten Materialien bestehen, insbesondere natürlichen und/oder synthetischen Materialien, insbesondere Baumwolle, Leinen, Seide, Hanf, Jute, Wolle, Sisal, Viskose, Polyamid, Polyester etc. sowie Mischungen hieraus.

Im Rahmen dieser Erfindung soll der Begriff "textile Produkte" explizit auch Wundauflagen umfassen, beispielsweise von Wundschnellverbänden (Pflastern), sowie Verbandsmaterialien.

Es ist bekannt, dass Textilien mit Substanzen und Wirkstoffen beladen werden können. So können beispielsweise Textilien mit Cyclodextrinen als Wirkstoffträger ausgerüstet und in diese Cyclodextrine niedermolekulare Wirkstoffe eingelagert werden, von wo sie die anschliessend freigesetzt werden. So können Wirkstoffe von einem textilen Produkt auf die Haut des Trägers migrieren, wo sie eine bestimmte gewünschte Wirkung entfalten können. Beispielsweise können auf diese Art kosmetische und/oder medizinische Wirkstoffe transdermal aufgenommen werden.

Ebenso können Textilien beispielsweise mit antibakteriellen oder fungizidalen Stoffen beladen werden, um zum Beispiel Geruchsbildung vorzubeugen, oder mit UV-absorbierenden Substanzen, um die UV-Absorption des Gewebes zu erhöhen. Ebenfalls denkbar sind Substanzen, welche Insekten fernhalten. Bei mit niedermolekularen Wirkstoffen beladenen Textilien geht bei jedem Waschgang unvermeidlich ein Teil der Wirkstoffe verloren. Es ist bei solchen funktionell modifizierten Textilien deshalb wünschenswert, diese auf einfache Weise erneut mit Wirkstoffen zu beladen zu können.

Ebenfalls bekannt ist die Ausrüstung von Textilien mit sogenannten Mikrokapseln, welche niedermolekulare Wirkstoffe enthalten, sowie die individuelle Beladung von Textilien mit solchen Mikrokapseln. Mikrokapseln besitzen den Nachteil, dass bei mechanischer Einwirkung und Zerstörung der Mikrokapseln die Wirkstoffe schlagartig freigesetzt werden. Solche Ausrüstungen sind deshalb für eine kontrollieret Abgabe über einen längeren Zeitraum wenig geeignet.

### Aufgabe der Erfindung

Aufgabe der Erfindung ist es, eine vorteilhafte Ausrüstungsformulierung zur Verfügung zu stellen, welche die oben erwähnten und andere Nachteile des Standes der Technik nicht aufweist.

Insbesondere ist es eine Aufgabe der Erfindung, eine Ausrüstungsformulierung zur Verfügung zu stellen, mit welcher textile Produkte und/oder Wundauflagen mit einer Ausrüstung versehen werden können, die effizient und gezielt mit kationischen Mikroemulsionen und/oder kationischen Wirkstoffen in Lösung beladbar sind. Vorzugsweise kann die Beladung mehrmals erfolgen. Die Tröpfchen der Mikroemulsion können insbesondere Wirkstoffe und andere aktive Substanzen enthalten.

Die Wirkstoffe der Ausrüstungsschicht sollen mit einer definierten Desorptionsrate freigesetzt werden können.

Noch eine Aufgabe der Erfindung ist es, Polymerverbindungen für eine solche Ausrüstungsformulierung bzw. eine solche Ausrüstung zur Verfügung zu stellen.

Ebenfalls Teil der Offenbarung ist eine Mikroemulsion. mit welcher eine erfindungsgemässe Ausrüstungsschicht mit niedermolekularen Verbindungen, insbesondere Wirkstoffen, beladen werden kann. Vorzugsweise kann eine solche Beladung in grosser Verdünnung der Emulsion stattfinden, beispielsweise während eines Waschvorgangs.

Diese und andere Aufgaben werden gelöst durch eine erfindungsgemässe Polymerverbindung, eine Ausrüstungsformulierung mit einer solchen Polymerverbindung, eine Ausrüstungsschicht mit einer solchen Polymerverbindung, und ein ausgerüstetes textiles Produkt bzw. eine beschichtete Wundauflage, gemäss den unabhängigen Ansprüchen. Weitere bevorzugte Ausführungsformen und Varianten sind in den abhängigen Ansprüchen gegeben.

### Darstellung der Erfindung

Eine erfindungsgemässe Polymerverbindung umfasst ein Acrylsäure-Copolymer aus Acrylsäure-Derivaten und/oder Methacrylsäure-Derivaten, welches beinhaltet: a) mindestens ein Acrylsäure-Derivat und/oder Methacrylsäure-Derivat, substituiert mit einer Sulfonsäuregruppe; b) mindestens ein hydrophil substituiertes Acrylsäure-Derivat und/oder Methacrylsäure-Derivat; c) mindestens ein lipophil substituiertes Acrylsäure-Derivat und/oder Methacrylsäure-Derivat; und d) mindestens ein Acrylsäure-Derivat und/oder Methacrylsäure-Derivat, welches als Vernetzer wirkt.

Ein mögliches Acrylsäure- bzw. Methacrylsäure-Derivat-Monomer mit einer Sulfonsäuregruppe ist beispielsweise 2-Acryloyl-2-methyl-propansulfonsäure. Die Sulfonsäuregruppen der erfindungsgemässen Polymerverbindung stellen in der Polymermatrix negative Ladungsstellen zur Verfügung, analog zu einem lonentauscher-Polymer. Die Ladungsstellen sind dabei aufgrund des sehr tiefen pK-Werts der Sulfonsäuregruppen bei den beim Waschen üblichen pH-Werten deprotoniert. Aufgrund der negativen Ladungsstellen der Polymermatrix resultiert eine negative Oberflächenladung, die natürlich durch Kationen ausgeglichen ist. Ohne sich auf eine bestimmte Erklärung einzuschränken zu wollen, liegt die erfindungsgemässe Wirkung der Polymerverbindung darin, dass mit einer positiven Oberflächenladung beladene dispergierte Partikel einer Mikroemulsion oder auch positiv geladene Wirkstoffe wie beispielsweise Hydrochloride von heterocyclischen Verbindungen oder andere kationische Verbindungen, sich in die Matrix der erfindungsgemässen Polymerverbindung einlagern können. Die Polymermatrix verfügt aufgrund der negativen Ladungsstellen über eine negative Oberflächenladung. Auf diese Weise kann die dispergierte Phase einer entsprechenden Emulsion oder die kationischen Verbindungen effizient in einer erfindungsgemässen Ausrüstungsschicht eingelagert werden.

In der dispergierten Phase einer solchen Emulsion sind niedermolekulare Verbindungen enthalten, die eine gewisse Wirkung entfalten sollen. Die genannten Verbindungen können von der Ausrüstungsschicht in kontrollierter Weise, d.h. kontinuierlich über einen längeren Zeitraum, wieder abgegeben werden, oder an Ort und Stelle verbleiben. Beispielsweise können Wirkstoffe nach der Beladung eines Textils von diesem auf die Haut des Trägers gelangen, dort transdermal aufgenommen werden und ihre spezifische Wirkung voll entfalten. Das Desorptionsverhalten kann dabei durch die Steuerung des Hydrophilie/Lipophilie-Verhältnisses, also durch das Einstellen der amphiphilen Eigenschaften der Donorschicht, massgeschneidert werden. So ist es beispielsweise problemlos möglich, die Desorptionszeit auf 16 h einzustellen, was einer realistischen Tragezeit von Textilien am Körper entspricht, oder wahlweise auf eine beliebige, gewünschte Zeitdauer anzupassen.

Wird das mit der Wirksubstanz beladene Textil direkt am Körper getragen, lösen Wärme, Reibung und Feuchtigkeit den Entladevorgang aus. Eine erfindungsgemässe Ausrüstungsschicht, auch als Donorschicht bezeichnet, besitzt im Weiteren die Eigenschaft, dass die Desorption durch die Salze im Körperschweiss getriggert werden kann. Insbesondere die im Schweiss vorhandenen Natriumionen erhöhen die Wirkstoffabgabe von der Donorschicht an die Haut. Entsprechend beanspruchte Körperstellen, beispielsweise bei sportlicher Betätigung, können dadurch bevorzugt mit Wirkstoffen versorgt werden.

Eine erfindungsgemässe Ausrüstungsschicht kann beliebig oft entladen und wieder beladen werden.

Die Beladung eines mit einer erfindungsgemässen Polymerverbindung ausgerüsteten textilen Produkts kann auch bei einer vergleichsweise starken Verdünnung der Emulsion erfolgen. So kann beispielsweise eine solche Emulsion in einem letzten Spülgang eines Waschprogramms einer Haushaltswaschmaschine dem Spülwasser beigegeben werden, analog zu einem Weichspüler. Eine Beladung kann jedoch auch mittels Handwäsche oder durch Besprühen der Textilien erfolgen, was je nach Anwendungszweck vorteilhafter sein kann. Bei Wundauflagen erfolgt die Beschichtung vorzugsweise in der sterilen Umgebung der Herstellungsanlage. Bei Verbandsmaterialien ist eine analoge Wiederbeladung wie bei Kleidung möglich.

Analog wäre es denkbar, dass die Polymermatrix positive Ladungsstellen aufweist, beispielsweise in Form von quartären Ammonium-Gruppen anstatt der Sulfonsäuregruppen, so dass Emulsionspartikel mit einer negativen Oberflächenladung daran adsorbieren. Eine solche Variante hat jedoch den Nachteil, dass beim Waschen eines entsprechend ausgerüsteten textilen Produkts sich die üblichen anionischen Tenside an die positiven Ladungsstellen anlagern können, womit diese Ladungsstellen abgeschirmt und nicht mehr zugänglich sind.

Damit die Beladungskapazität einer Ausrüstungsschicht optimal ist, sollten möglichst viele Ladungsstellen für die Partikel der Emulsion zugänglich sein. Zu diesem Zweck ist es deshalb vorteilhaft, wenn die Ausrüstungsschicht auf dem Textil eine gewisse Quellung aufweist, da auf diese Weise die zugängliche Oberfläche der Polymermatrix steigt, und damit auch die zugängliche Oberflächenladung.

Um die gewünschte Hydratisierbarkeit und Quellbarkeit der erfindungsgemässen Ausrüstungsschicht zu erreichen, enthält die erfindungsgemässe Polymerverbindung hydrophil substituierte Acrylsäure- bzw. Methacrylsäure-Derivat-Monomere, wie beispielsweise Ethyltriglykol-methacrylat, 2-Hydroxyethyl-methacrylat (HEMA), und/oder mPEG-Methacrylate, insbesondere mPEG-1000-Methacrylat und mPEG-350-Methacrylat. HEMA hat den zusätzlichen Effekt, dass es bei einer Fixierung der Ausrüstungsschicht auf dem Textil auch als Andockstelle für Vernetzer-Monomere wirkt, was zu einer polymerinternen Vernetzung führt. Insgesamt verhalten sich Ausrüstungsschichten mit HEMA eher spröde, wenn sie nicht hydratisiert sind, während Ausrüstungsschichten mit mPEG-Methacrylaten in trockenem Zustand eher elastisch bleiben.

Lipophil substituierte Acrylsäure- bzw. Methacrylsäure-Derivat-Monomere, wie beispielsweise 2-Ethylhexyl-acrylat, stellen eine gewisse Lipophilie der Matrix einer erfindungsgemässen Ausrüstungsschicht sicher. Das Verhältnis der Anteile von hydrophilen und lipophilen Monomeren bestimmt dabei unter anderem die Sorptions- und Desorptionseigenschaften einer erfindungsgemässen Ausrüstungsschicht. Weiter können lipophile Verbindungen innerhalb der adsorbierten Emulsionspartikel in die lipophilen Domänen der Polymermatrix migrieren, was die Beladungskapazität der Ausrüstungsschicht erhöht.

Um eine hinreichende Permanenz der Ausrüstungsschicht auf dem textilen Produkt zu erreichen, muss die Ausrüstungsschicht auf den Fasern fixiert werden. Dazu wird die erfindungsgemässe Polymerverbindung mit den Textilfasern vernetzt. Vernetzer für textile Ausrüstungen sind aus dem Stand der Technik bekannt. Ein mögliches Vernetzer-Monomer für eine erfindungsgemässe Polymerverbindung ist zum Beispiel N-(Butoxymethyl)-acrylamid. Bei einer thermischen und/oder säurekatalysierten Fixierung wird das entsprechende Monomer kovalent mit OH- und NH₂-Gruppen der Fasern verbunden.

Vorteilhaft sind die Vernetzer-Monomere des Acrylsäure-Copolymers ausgewählt aus einer Gruppe bestehend aus N-(Butoxymethyl)-acrylamid, N-(Methylol)-acrylamid, Glycidyl-methacrylat, p-EMKO-TDI-o-HEMA und EMKO-2-(N-(tert-Butyt){[(3-isocyanato-1, 5, 5-trimethylcyclohexyl)-methyl]-amino} carbonylamino)-ethyl-methacrylat.

Eine erfindungsgemässe Polymerverbindung kann weitere Polymerverbindungen enthalten, beispielsweise Polyethersulfone, Polyurethane, Polyesterurethane, Polyetherurethane, Polyamide oder Gemische davon. Eine erfindungsgemässe Polymerverbindung kann ein Blend solcher verschiedener Polymerverbindungen sein, die vorteilhaft vernetzbar sind.

Um eine erfindungsgemässe Ausrüstungsschicht auf einem textilen Produkt bzw. einer Wundauflage zu erzeugen, wird eine Ausrüstungsformulierung auf das textile Produkt bzw. die Wundauflage aufgebracht, beispielsweise in einer wässrigen Flotte. Die Ausrüstungsformulierung enthält eine erfindungsgemässe Polymerverbindung in gelöster Form und/oder in Form einer Mikroemulsion. Nach einem ersten Trocknungsschritt wird die Polymerverbindung thermisch und/oder säurekatalytisch auf dem Faseruntergrund fixiert. Dem Fachmann sind die entsprechenden Verfahren zum Ausrüsten von Textilien mit Polymerbeschichtungen aus dem Stand der Technik bekannt.

Eine erfindungsgemässe Ausrüstungsformulierung kann neben erfindungsgemässen Polymerverbindungen weitere Polymere wie PES, PU, PUE, PA und Vernetzersysteme usw. und/oder Mischungen davon enthalten. Aufgrund ihrer amphiphilen Struktur sind die erfindungsgemässen Polymerverbindungen mit vielen Polymeren mischbar und/oder vernetzbar, bzw. zu Blends verarbeitbar.

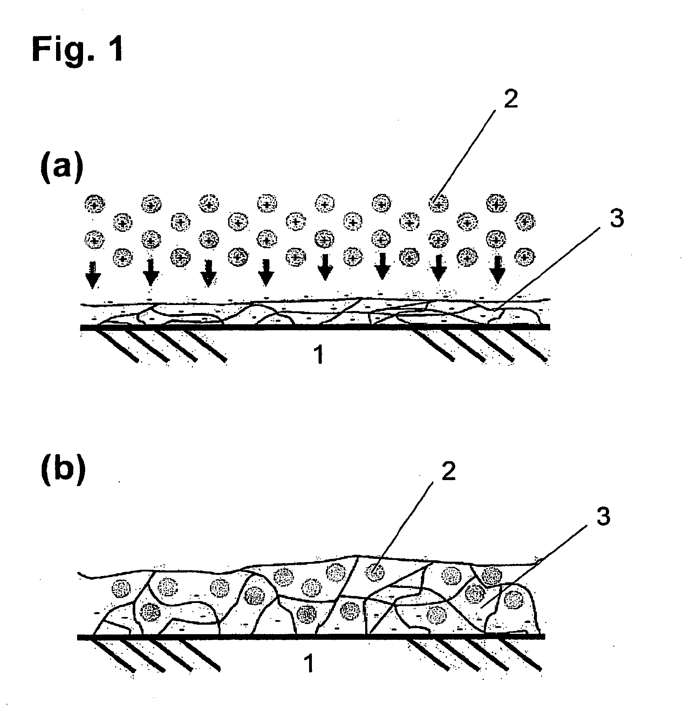

Die Sorption von Emulsionspartikeln in einer erfindungsgemässen Ausrüstungsschicht ist in Figur 1 schematisch dargestellt. Durch Aufnahme von Wasser aus der Umgebung, beispielsweise Luftfeuchtigkeit oder Spülwasser aus dem Beladungsprozess, wird die auf der Faserunterlage 1 fixierte Ausrüstungsschicht 3 hydratisiert und gequellt. Die negativen Ladungsstellen der Polymermatrix werden zugänglich. Wird nun die Ausrüstungsschicht 3 mit einer Emulsion zusammengebracht, deren Emulsionspartikel 2 eine positive Oberflächenladung aufweisen, so können diese Partikel 2 in die Poren und Lücken der Polymermatrix migrieren (Figur 1(a)), wo sie sich in die Polymermatrix einlagern (Figur 1(b)).

Bei einem offenbarungsgemassen Verfahren zur Beladung von textilen Produkten mit niedermolekularen Verbindungen wird a) ein textiles Produkt mit einer Ausrüstungsschicht versehen, deren zugängliche Oberfläche eine negative Ladung aufweist; b) das textile Produkt mit einer Emulsion zusammengebracht, beispielsweise durch Eintauchen des textilen Produkts in die Emulsion oder durch Aufsprühen der Emulsion auf das textile Produkt. Mindestens eine niedermolekulare Verbindung ist in der dispergierten Phase der Emulsion enthalten. Die Grenzfläche der dispergierten Phase weist eine positive Ladung auf. Anstelle einer Emulsion kann auch eine Lösung verwendet werden, wenn die darin gelöste niedermolekulare Verbindung kationisch ist.

Vorzugsweise wird der Schritt b) mehrfach durchgeführt, in beliebigen zeitlichen Abständen. Auf diese Weise lassen sich die ausgerüsteten Materialien immer wieder neu mit den gewünschten niedermolekularen Verbindungen beladen.

Bevorzugt handelt es sich bei der Emulsion mit der niedermolekularen Verbindung um eine offenbarungsgemässe Emulsion wie sie nachfolgend beschrieben wird.

Eine offenbarungsgemasse Emulsion enthält mindestens eine niedermolekulare Verbindung, mit welcher die Textilien beladen werden sollen, in der dispergierten Phase der Emulsion. Die Oberfläche der Partikel der dispergierten Phase weist eine positive Ladung auf. Diese ist natürlich durch negativ geladene Gegenionen ausgeglichen. Vorteilhaft beträgt die Oberflächenladung der Partikel der dispergierten Phase der Emulsion mindestens 15 mC/g Emulsion, besonders vorteilhaft mindestens 90 mC/g Emulsion.

Erreicht wird die positive Oberflächenladung der Emulsionspartikel durch Emulgatoren bzw. grenzflächenaktive Verbindungen, die an ihrem polaren Ende eine positive Ladung aufweisen. Diese positiven Ladungen befinden sich dann bei einer Öl-in-Wasser-Emulsion an der Oberfläche der Partikel. Geeignet als grenzflächenaktive Verbindungen sind beispielsweise Lecithin, insbesondere Phosphatidylcholin-Lecithine, und/oder quartäre Ammoniumverbindungen mit einem oder zwei langkettigen lipophilen Resten, insbesondere Behenyltrimethylammonium, oder Ethyl-N^{alpha}-Lauroyl-L-Arginat/HCl.

Damit die Partikel der dispergierten Phase in die gequellte Polymermatrix der Ausrüstungsschicht eindringen können, sollte der Durchmesser der Partikel eine gewisse Grösse nicht überschreiten. Je kleiner die Partikel sind, desto besser und schneller können sie in die Poren der Polymermatrix eindringen, und sich an die Matrix anlagern. Bevorzugt weisen mindestens 90%-Vol. der Partikel der dispergierten Phase der Emulsion einen hydrodynamischen Durchmesser von weniger als 1000 nm auf, besonders bevorzugt weniger als 700 nm.

Die Emulsion kann eine Öl-in-Wasser-Emulsion sein, wobei mindestens eine niedermolekulare Verbindung sich in der lipophilen dispergierten Phase befindet. Diese Variante ist insbesondere für lipophile niedermolekulare Verbindungen geeignet.

Für hydrophile niedermolekulare Verbindungen sind beispielsweise Wasser-in-Öl-in-Wasser-Emulsionen geeignet wobei sich mindestens eine niedermolekulare Verbindung in der wässrigen dispergierten Phase innerhalb der lipophilen dispergierten Phase befindet. Ebenfalls geeignet sind Emulsionen, die Liposomen enthalten, wobei sich die hydrophilen niedermolekularen Verbindungen in diesem Fall in der wässrigen Phase innerhalb der Liposomen befinden.

Sollen sowohl lipophile als auch hydrophile Verbindungen auf das Textil geladen werden, so können die verschiedenen Emulsionsarten auch kombiniert werden. Alternativ können auch verschiedene Emulsionen sequentiell angewendet werden.

Ebenfalls Teil der Offenbarung ist die Verwendung einer offenbarten Emulsion zur Beladung eines textilen Produkts bzw. einer Wundauflage mit lipophilen und/oder hydrophilen niedermolekularen Verbindungen, wobei bevorzugt das textile Produkt bzw. die Wundauflage eine erfindungsgemässe Ausrüstungsschicht aufweist, und/oder mit einer erfindungsgemässen Ausrüstungsformulierung ausgerüstet worden ist.

Eine erfindungsgemässe Ausrüstungsschicht kann auch mit der in WO 2002/075038 A1 offenbarten 3XDry® Technologie der Anmelder kombiniert werden. So kann beispielsweise die hydrophile erfindungsgemässe Ausrüstungsschicht auf der Aussenseite mit einer hydrophoben Beschichtung versehen werden. Auf diese Weise kann ein erfindungsgemässes Textil nicht nur auf einer Aussenfläche wasserabweisend gemacht werden, während es auf der Innenseite weiterhin hydrophil ist, sondern die hydrophobe Schicht dient gleichzeitig als Wirkstoffbarriere gegen aussen. Solche Barriereschichten sind beispielsweise bei transdermalen Pflastern üblich, wo Wirkstoffe nur in eine definierte Richtung desorbieren sollen.

Eine erfindungsgemässe Ausrüstungsschicht verfügt weiter über die Eigenschaft, kationische Schwermetallionen wie z.B. Cadmium, Blei oder andere toxische Substanzen binden zu können. Dies ist insbeson-dere z.B. in Ländern mit hohen Arsengehalten im Trinkwasser von Bedeutung, da auf diese Weise Trinkwasser geniessbar gemacht werden kann. Die Regeneration des mit der Polymerschicht ausgerüsteten Textils kann durch Salz, Meerwasser, Seife oder Waschpulver erfolgen. Weiter kann eine erfindungsgemässe Ausrüstungsschicht organische Verunreinigungen in Wasser binden, beispielsweise Diesel oder Benzin, da organische Verunreinigungen an die lipophilen Strukturen der erfindungsgemässen, amphiphilen Polymerverbindungen adsorbieren können. Durch diese Anwendungsmöglichkeit kann ein erfindungsgemässes Textil zur Aufbereitung von Trinkwasser verwendet werden.

### Ausführung der Erfindung

Die im Folgenden gegebenen Beispiele dienen der Veranschaulichung, sind jedoch nicht dazu geeignet, die Erfindung auf die hierin offenbarten Merkmale zu beschränken.

### A. Polymere

### Polymerverbindung P-002

Bei der Polymerverbindung P-002 handelt es sich um ein Acrylsäure-Copolymer, nämlich Poly(Acrylsäure-stat-2-Ethylhexyl-acrylat-stat-N-(Butoxymethyl)-acrylamid). Dieses beinhaltet die Monomere Acrylsäure, deren Carbonsäuregruppe der Zurverfügungstellung von negativen Ladungsstellen in der Polymermatrix dient; 2-Ethylhexyl-acrylat, als lipophile Gruppe, und N-(Butoxymethyl)-acrylamid. Letzteres dient der Vernetzung des Polymers mit OH- und NH₂-Gruppen der Textilfasern. Die Synthese erfolgt mittels radikalischer Emulsionspolymerisation.

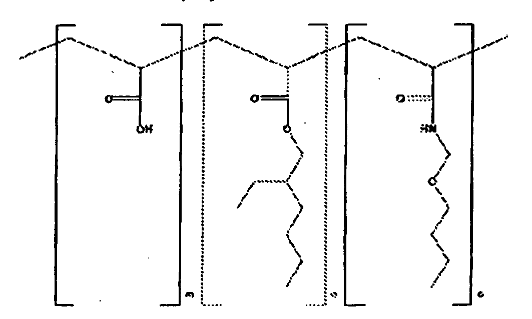

### Eduktlösung:

| *Einwaage* | *Produktbezeichnung* |
|---|---|
| 30.0 | Acrylsäure, 99.5%, stabilisiert, Acros Organics, M = 72.06 g/mol, d = 1.050 g/ml, bp = 1 39 °C |
| 115.5 g | 2-Ethylhexyl-acrylat, 99%, stabilisiert, Acros Organics, d = 0.880g/ml, bp = 215 °C |
| 4.5 g | Cylink NBMA Monomer: N-(Butoxymethyl)-acrylamid, 81%, Cytec Industries, d = 0.96 g/ml, bp = 125-128 °C/0.03 mmHg |
| 4.5 g | Disponil AFX 1080, Cognis Deutschland GmbH & Co KG |
| 0.75 g | Natriumdodecylsulfat (SDS), p.A., Serva Feinbiochemika |
| 25.0 g | Dipropylenglykol, d = 1.023 g/ml, bp = 90-95 °C/1 mmHg |
| 275.0 g | Wasser deionisiert |

### Initiatorlösung (entspricht 1%-Gew. V-501 bezogen auf Monomere und Vernetzer):

| *Einwaage* | *Produktbezeichnung* |
|---|---|
| 1.5 g | V-501: 4, 4'-Azobis-(4-cyanovaleriansäure), ≥98.0%, Fluka, M = 280.28 g/mol |
| 1.0 g | Natriumhydroxid 50%-ig |
| 50.0 g | Wasser deionisiert |

**Apparatur:** 1 I-Vierhalssrundkolben mit Rührwerk, Rückflusskühler, Septum und Temperaturfühler. Ein Abgang mit Hahn auf dem Rückflusskühler dient dem Evakuieren und Belüften mit Stickstoff.

**Vorhomogenisation:** Die Eduktlösung wird fünf Mal bei 600 bar durch den Hochdruck-Homogenisierer gefördert, wodurch eine bläulich schimmernde Emulsion entsteht 1 g homogenisierte Eduktlösung wird mit 39 g Wasser verdünnt (1:40). Viskosität: 1.01 mPa · s. Die Partikelgrössenverteilung wurde mittels Photonenkorrelationsspektroskopie (PCS) bestimmt (Malvern Instruments Ltd., Malvern, Worcestershire, WR14 1XZ, UK; Modell: Zetasizer Nano-S ZEN 1600): Peak 1: d(H) = 1140 nm - 98.1%-Vol.; Peak 2: d(H) = 5290 nm - 1.9%-Vol.

**Synthese:** 455 g homogenisierte Eduktlösung wird in der Apparatur vorgelegt und auf 90 °C erhitzt. Anschliessend wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 25 g Initiatorlösung durch das Septum zudosiert. Die Reaktion springt sofort an. Aufgrund der starken Exothermie (5 °C/min) wird sofort mit einem Wasserbad gegengekühlt und die Reaktionstemperatur bei 90 °C konstant gehalten. (keine Nachexothermie feststellbar). Nach Abklingen der Startexothermie werden nach 40 min wiederum mittels Spritze die restlichen 27 g Initiatorlösung zugegeben (keine Nachexothermie feststellbar).

**In-Prozess-Kontrolle:** Nach einer Reaktionszeit von insgesamt 4 h werden der Apparatur 2 g Reaktionslösung entnommen, auf eine tarierte Aluschale gegeben und während 30 min bei 180 °C im Trockenofen getrocknet: Einwaage = 2.023 g dünnflüssige, weisse Emulsionslösung; Auswaage = 0.594 g milchig-weisses Polymer; Trockensubstanz: 29.36% (TS_{100% theoretischer Umsatz}: 30.87%) = 95.1% Umsatz. Quellverhalten: Etwas getrocknetes Polymer wird in einem Reagenzglas mit THF versetzt, optisch ist ein leichtes Quellen feststellbar. pH = 3.3.

**Reaktionslösung:** Die blau-rosa schimmernde, niedrigviskose Reaktionslösung wird auf Raumtemperatur abgekühlt. 1 g Reaktionslösung wird mit 39 g Wasser verdünnt (1:40). Viskosität: 1.05 mPa · s. Partikelgrössenverteilung (PCS-Messung): Peak 1: d(H) = 503 nm - 92.6%-Vol.; Peak 2: d(H) = 5220 nm - 7.4%-Vol. Der 1:40 verdünnten Reaktionslösung wird 1 ml 25%-ige Ammoniak-Lösung zugegeben, um die Zugänglichkeit der Carboxylgruppen bzw. die Quellbarkeit des Polymers zu eruieren. Viskosität: 2.36 mPa · s. Partikelgrössenverteilung (PCS-Messung): Peak 1: d(H) = 587 nm - 93.3%-Vol.; Peak 2: d(H) = 5340 nm - 6.70%-Vol. Emulsionstropfenvolumen (berechnet): Peak 1: V(H) = 66.6E+6 nm³ ohne NH₃; Peak 1: V(H) = 105.9E+6 nm³ mit NH₃; Quellung Emulsionstropfen: Peak 1: 59% Volumenzunahme.

### Polymerverbindung P-004

Die Polymerverbindung P-004 Poly(2-Acryloylamino-2-methylpropan-natriumsulfonat-stat-2-Ethylhexyl-acrylat-stat-N-(Butoxymethyl)-acrylamid) beinhaltet die Monomere 2-Acryloylamino-2-methylpropan-natriumsulfonat, 2-Ethylhexyl-acrylat, und N-(Butoxymethyl)-acrylamid. Die Sulfonatgruppen stellen die negativen Ladungsstellen in der Polymermatrix zur Verfügung.

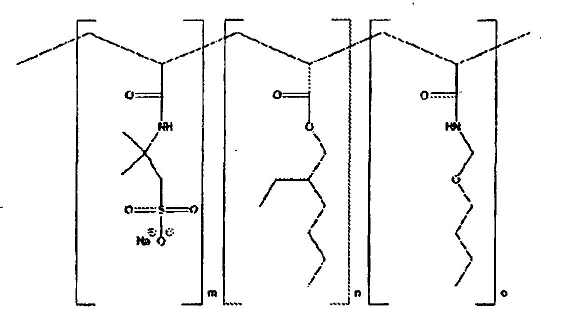

### Eduktlösung:

| *Einwaage* | *Produktbezeichnung* |
|---|---|
| 30.0 g | 2-Acryloylamino-2-methylpropan-sulfonsäure, >98.0%, Fluka |
| 115.5 g | 2-Ethylhexyl-acrylat, 99%, stabilisiert, Acros Organics, d = 0.880g/ml, bp = 215 °C |
| 4.5 g | Cylink NBMA Monomer: N-(Butoxymethyl)-acrylamid, 81%, Cytec Industries |
| 4.5 g | Disponil AFX 1080, Cognis Deutschland GmbH & Co KG |
| 0.75 g | Natriumdodecylsulfat (SDS), p.A., Serva Feinbiochemika |
| 25.0 g | Dipropylenglykol, d = 1.023 g/ml, bp = 90-95 °C/l mmHg |
| 12.7 g | Natriumhydroxid 50%-ig (Wassergehalt berücksichtigt) |
| 262.3 g | Wasser deionisiert |

**Vorhomogenisation:** Die Eduktlösung (pH = 6.5) wird fünf Mal bei 600 bar durch den Hochdruck-Homogenisierer gefördert, wodurch eine bläulich schimmernde Emulsion entsteht. 1 g homogenisierte Eduktlösung wird mit 39 g Wasser verdünnt (1:40). Viskosität: 0.95 mPa · s; Partikelgrössenverteilung (PCS-Messung): Peak 1: d(H) = 1370 nm - 93.9%-Vol.; Peak 2: d(H) = 4780 nm - 6.1%-Vol.

**Synthese:** Initiatorlösung wie bei P-002, 1%-Gew. V-501 bezogen auf Monomere und Vernetzer. Apparatur wie bei P-002. 455 g homogenisierte Eduktlösung wird in der Apparatur vorgelegt und auf 90 °C erhitzt. Bei Erreichen von etwa 70 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 25 g Initiatorlösung durch ein Septum zudosiert. Die Reaktion springt sofort an. Aufgrund der starken Exothermie (5 °C/min) wird sofort gegengekühlt und die Reaktionstemperatur bei 90 °C konstant gehalten. Nach Abklingen der Startexothermie werden nach 30 min wiederum mittels Spritze die restlichen 27 g Initiatorlösung zugegeben (keine Nachexothermie feststellbar). Die Reaktionslösung schäumt während etwa 30 min auf, danach verschwindet der Schaum jedoch wieder.

**In-Prozess-Kontrolle:** Nach einer Reaktionszeit von insgesamt 4 h werden der Apparatur 2 g Reaktionslösung entnommen, auf eine tarierte Aluschale gegeben und während 30 min bei 180 °C im Trockenofen getrocknet: Einwaage = 2.024 g dünnflüssige, weisse Emulsionslösung. Auswaage = 0.609 g milchig-weisses, leicht klebriges Polymer. Trockensubstanz: 30.09% (TS_{100% theoretischer Umsatz}: 31.50%) = 95.5% Umsatz. Quellverhalten: Etwas getrocknetes Polymer wird in einem Reagenzglas mit THF versetzt, optisch ist eine Quellbarkeit feststellbar. pH = 8.5.

**Reaktionslösung:** Die bläulich schimmernde, niedrigviskose Reaktionslösung wird auf Raumtemperatur abgekühlt. 1 g Reaktionslösung wird mit 39 g Wasser verdünnt (1:40). Viskosität 1.33 mPa · s. Partikelgrössenverteilung (PCS-Messung): Peak 1: d(H) = 329 nm - 100%-Vol.; Peak 2: Kein Signal vorhanden.

### Polymerverbindung P-005

Die Polymerverbindung P-005 Poly(2-Acryloylamino-2-methylpropan-natriumsulfonat-stat-2-Ethylhexyl-acrylat-stat-mPEG-1000-Methacrylat-stat-N-(Butoxymethyl)-acrylamid) beinhaltet neben 2-Acryloylamino-2-methylpropan-natriumsulfonat, 2-Ethylhexyl-acrylat, und N-(Butoxymethyl)-acrylamid als weiteres Monomer mPEG1000-Methacrylat. Das mPEG1000-Methacrylat-Monomer dient der Hydrophilisierung der Polymerschicht, die so Wasser aufnehmen kann, damit die negativen Ladungsstellen in der Matrix besser zugänglich sind.

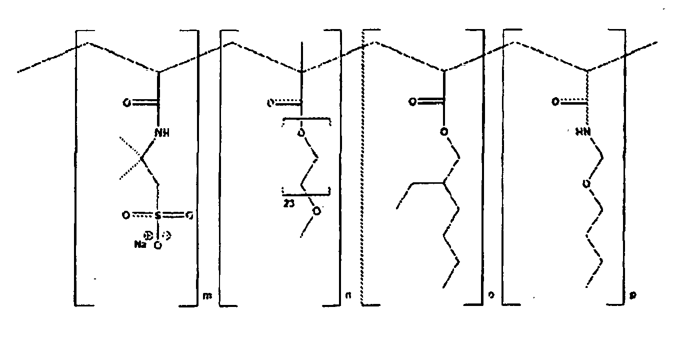

### Eduktlösung:

| *Einwaage* | *Produktbezeichnung* |
|---|---|
| 30.0 g | 2-Acryloylamino-2-methylpropan-sulfonsäure, >98.0%, Fluka |
| 120.0 g | Plex 6969-O: mPEG-1000-Methacrylat (enthält 50% Wasser und 1-5% Methacrylsäure), Evonik Industries, M = 1000 g/mol, d = 1.08 g/ml |
| 40.5 g | 2-Ethylhexyl-acrylat, 99%, stabilisiert, Acros Organics, d = 0.880g/ml, bp = 215 °C |
| 4.5 g | Cylink NBMA Monomer N-(Butoxymethyl)-acrylamid, 81%, Cytec Industries |
| 4.5 g | Disponil AFX 1080, Cognis Deutschland GmbH & Co KG |
| 0.75 g | Natriumdodecylsulfat (SDS), p.A., Serva Feinbiochemika |
| 25.0 g | Dipropylenqlykol, d = 1.023 g/ml, bp = 90-95 °C/1 mmHg |
| 21.0 g | atriumhydroxid 50%-ig (Wasserqehalt berücksichtigt) |
| 194.0 g | Wasser deionisiert |

**Synthese:** Initiatorlösung wie bei P-002, 1%-Gew. V-501 bezogen auf Monomere und Vernetzer. Apparatur wie bei P-002. Vorhomogenisation wie bei P-002. 455 g homogenisierte Eduktlösung wird in der Apparatur vorgelegt und auf 90 °C erhitzt. Bei Erreichen von etwa 70 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 25 g Initiatorlösung durch das Septum zudosiert Es ist nur eine leichte Exothermie feststellbar. Die Reaktionstemperatur wird bei 90 °C konstant gehalten. Nach 30 min werden wiederum mittels Spritze die restlichen 27 g Initiatorlösung zugegeben (keine Nachexothermie feststellbar). Es entsteht eine bläulich schimmernde Emulsion. Die Reaktionslösung, dessen Viskosität zunimmt, schäumt während etwa 30 min auf, danach verschwindet der Schaum jedoch wieder.

**In-Prozess-Kontrolle:** Nach einer Reaktionszeit von insgesamt 4 h werden der Apparatur 2 g Reaktionslösung entnommen, auf eine tarierte Aluschale gegeben und während 30 min bei 180 °C im Trockenofen getrocknet: Einwaage = 2.185 g viskose, weisse Emulsionslösung. Auswaage = 0.746 g bräunliches, leicht klebriges Polymer. Trockensubstanz: 34.14% (TS_{100% theoretischer Umsatz}: 31.81%) = 107.3% (aufgrund von Lösungsmittelverlust während der Reaktion handelt es sich hierbei nicht um Umsatz). Quellverhalten: Etwas getrocknetes Polymer wird mit Wasser übergossen, wodurch das Polymer innert Minuten um ein Vielfaches anquellt und sich ablöst. Die Permanenz scheint ungenügend. pH = 6.5.

### Polymerverbindung P-008

Die Polymerverbindung P-008 Poly(2-Acryloylamino-2-methylpropan-natriumsulfonat-stat-2-Ethylhexyl-acrylat-stat-2-Hydroxyethyl-methacrylat-stat-N-(Butoxymethyl)-acrylamid) beinhaltet die Monomere 2-Acryloylamino-2-methylpropan-natriumsulfonat, 2-Hydroxyethyl-methacrylat (HEMA), 2-Ethylhexyl-acrylat, und N-(Butoxymethyl)-acrylamid. HEMA dient der Hydrophilisierung der Polymerschicht, die so Wasser aufnehmen kann, damit die negativen Ladungsstellen in der Matrix besser zugänglich sind.

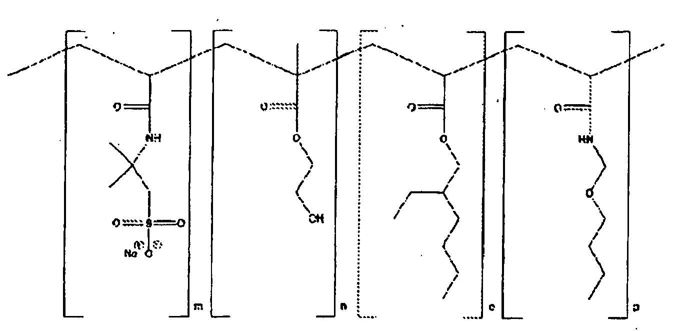

Um eine vollständige Aufnahme des Lösungsmittels durch das sehr quellbare Polymer zu vermeiden, wird die Eduktlösung mit 2-Propanol und Wasser 1:4 verdünnt. Die Initiatorlösung wird unverdünnt eingesetzt, um die Reaktionsgeschwindigkeit ohne grosse Einbussen aufrecht zu erhalten.

### Eduktlösung:

| *Einwaage* | *Produktbezeichnung* |
|---|---|
| 5.0 g | 2-Acryloyl-2-methylpropan-sulfonsäure, >98.0%, Fluka |
| 6.7 g | 2-Hydroxyethyl-methacrylat (HEMA), 95%, Fluka, d = 1.07 g/ml, bp = 205-208 °C |
| 4.5 g | 2-Ethylhexyl-acrylat, 99%, stabilisiert, Acros Organics, d = 0.880g/ml, bp = 215 °C |
| 0.5 g | Cylink NBMA Monomer: N-(Butoxymethyl)-acrylamid, 81%, Cytec Industries |
| 10.0 g | 2-Propanol EP4, Schweizerhall, d = 0.785 g/ml, bp = 82 °C |
| 20.0 g | Dipropylenglykol, d = 1.023 g/ml, bp = 90-95 °C/1 mmHg |
| 2.0 g | Natriumhydroxid 50%-ig (Wassergehalt berücksichtigt) |
| 12.0 g | Wasser deionisiert |

### Initiatorlösung:

| *Einwaage* | *Produktbezeichnung* |
|---|---|
| 1.7 g | V-501: 4, 4'-Azobis-(4-cyanovaleriansäure), >98.0%. Fluka, M = 280.28 g/mol |
| 0.8 g | Natriumhydroxid 50%-ig |
| 67.5 g | Wasser deionisiert |

Eingesetzt wird 1/10 der Initiatorlösung, was 1%-Gew. V-501 bezogen auf Monomere und Vernetzer entspricht.

**Apparatur:** Ein 50 ml-Schlenkrohr mit Magnetrührer und Septum wird auf eine Magnetrührheizplatte mit einem Aluminium-Heizblock montiert und mit einem Temperaturfühler ausgerüstet. Der Abgang mit Hahn am Schlenkrohr dient dem Evakuieren und Belüften mit Stickstoff.

**Synthese:** 10 g Eduktlösung (pH = 5.5) wird im Schlenkrohr vorgelegt und durch Zugabe von 10 g 2-Propanol und 20 g Wasser 1:4 verdünnt. Dann wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird das Schlenkrohr mit Stickstoff hinterleitet. Nun wird mittels Heizpilz auf 90 °C erhitzt. Bei Erreichen von etwa 70 °C werden unter gutem Rühren mittels einer Spritze 3.5 g Initiatorlösung durch das Septum zudosiert Es ist nur eine leichte Exothermie feststellbar. Die Reaktionstemperatur wird bei 82 °C (Siedepunkt 2-Propanol) konstant gehalten. Es entsteht eine klare, weissliche Lösung. Nach 30 min werden wiederum mittels Spritze die restlichen 3.5 g Initiatorlösung zugegeben (keine Nachexothermie feststellbar).

**In-Prozess-Kontrolle:** Nach einer Reaktionszeit von insgesamt 4 h werden der Apparatur 8 g Reaktionslösung entnommen, auf eine tarierte Aluschale gegeben und während 30 min bei 180 °C im Trockenofen getrocknet. Einwaage = 8.033 g klare, bläulich-weisse Lösung. Auswaage = 0.584 g klares, goldbraunes, nicht klebriges, brüchiges Polymer. Trockensubstanz: 7.27% (TS_{100% theoretischer Umsatz}: 6.40%) = 113.6% (aufgrund von Lösungsmittelverlust während der Reaktion handelt es sich hierbei nicht um Umsatz). Quellverhalten: Etwas getrocknetes Polymer wird mit Wasser übergossen, wodurch das Polymer innert Minuten etwas anquellt. Das Polymer scheint visuell nur leicht vernetzt zu sein. pH = 6.7.

### Polymerverbindung P-009

Die Polymerverbindung P-009 wird analog zu P-008 hergestellt. Die Eduktlösung wird nur 1:2 verdünnt, jedoch mit Hexylenglykol (bp = 197 °C) anstelle von 2-Propanol, um die Polymerisationstemperatur auf 90 °C zu steigern. Die Initiatorlösung wird wiederum unverdünnt eingesetzt, um die Reaktionsgeschwindigkeit ohne grosse Einbussen aufrecht zu erhalten.

**Synthese:** Eduktlösung, Initiatorlösung, Apparatur wie in P-008. 19 g Eduktlösung (pH = 5.5) wird im Schlenkrohr vorgelegt und durch Zugabe von 19 g Hexylenglykol 1:2 verdünnt. Dann wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird das Schlenkrohr mit Stickstoff hinterleitet. Nun wird mittels Heizpilz auf 90 °C erhitzt. Bei Erreichen von etwa 70 °C werden unter gutem Rühren mittels einer Spritze 3.5 g Initiatorlösung durch das Septum zudosiert. Es ist nur eine leichte Exothermie feststellbar. Die Reaktionstemperatur wird bei 90 °C konstant gehalten. Nach 30 min werden wiederum mittels Spritze die restlichen 3.5 g Initiatorlösung zugegeben (keine Nachexothermie feststellbar). Es entsteht eine klare, viskose Lösung.

**In-Prozess-Kontrolle:** Nach einer Reaktionszeit von insgesamt 4 h werden der Apparatur 4 g Reaktionslösung entnommen, auf eine tarierte Aluschale gegeben und während 30 min bei 180 °C im Trockenofen getrocknet. Da aufgrund des schwerflüchtigen Hexylenglykols (bp = 197 °C) das Polymer noch nicht trocken ist, wird es mit etwas Wasser angequollen und nochmals während 30 min bei 180 °C im Trockenofen getrocknet: Einwaage = 4.093 g klare, bläulich-weisse Lösung. Auswaage = 0.537 g klares, goldbraunes, nicht klebriges, brüchiges Polymer. Trockensubstanz: 13.12% (TS_{100% theoretischer Umsatz}: 12.36%) = 106.1% (aufgrund von Lösungsmittelverlust während der Reaktion handelt es sich hierbei nicht um Umsatz). Quellverhalten: Etwas getrocknetes Polymer wird mit Wasser übergossen, wodurch das Polymer unmittelbar um ein Vielfaches anquellt und sich ablöst. Die Permanenz scheint ungenügend zu sein. pH = 7.2.

### Polymerverbindung P-010

Die Polymerverbindung P-010 - Poly(2-Acryloylamino-2-methylpropan-natriumsulfonat-stat-2-Ethylhexyl-acrylat-stat-mPEG-350-Methacrylatstat-N-(Butoxymethyl)-acrylamid) - wird analog zu P-005 hergestellt, jedoch mit mPEG-350-Methacrylat anstelle von mPEG-1000-Methacrylat, um den Einfluss eines kürzerkettigen mPEG-Monomers auf die Permanenz zu untersuchen. Zusätzlich zu den Emulgatoren wird noch zusätzlich 2-Propanol zugesetzt, um eine Phasentrennung der Eduktlösung zu vermeiden.

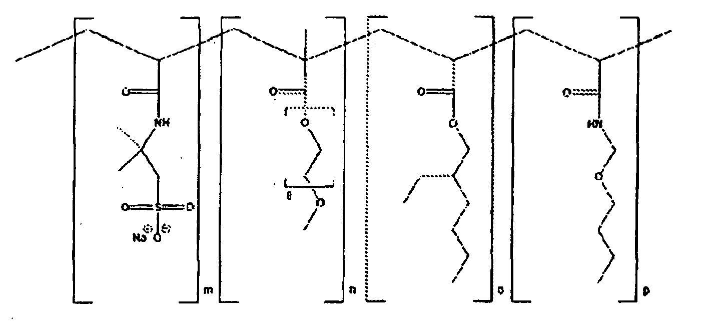

### Eduktlösung:

| *Einwaage* | *Produktbezeichnung* |
|---|---|
| 2.48 g | 2-Acryloylamino-2-methylpropan-sulfonsäure, >98.0%, Fluka |
| 3.30 g | mPEG-350-Methacrylat, >95%, Evonik Industries, M = 418 g/mol, d = 1.08 g/ml |
| 2.23 g | 2-Ethylhexyl-acrylat, 99%, stabilisiert, Acros Organics |
| 0.25 g | Cylink NBMA Monomer: N-(Butoxymethyl)-acrylamid, 81%, Cytec Industries |
| 0.25 g | isponil AFX 1080, Coqnis Deutschland GmbH & Co KG |
| 0.04 g | Natriumdodecylsulfat (SDS), p.A., Serva Feinbiochemika |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 1.0 g | Natriumhydroxid 50%-ig (Wassergehalt berücksichtigt) |
| 6.0 g | Wasser deionisiert |

**Vorhomogenisation:** Die Eduktlösung wird während zwei Minuten im Ultraschallbad homogenisiert.

**Synthese:** Initiatorlösung, Apparatur wie in P-008. 30 g homogenisierte Eduktlösung wird in der Apparatur vorgelegt und auf 80 °C erhitzt. Bei Erreichen von etwa 70 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 1.8 g Initiatorlösung durch das Septum zudosiert. Es ist nur eine leichte Exothermie feststellbar. Die Reaktionstemperatur wird bei 80 °C konstant gehalten. Nach 30 min werden wiederum mittels Spritze die restlichen ca. 1.7 g Initiatorlösung zugegeben (keine Nachexothermie feststellbar). Es entsteht eine opake, niedrigviskose Flüssigkeit.

**In-Prozess-Kontrolle:** Nach einer Reaktionszeit von insgesamt 4 h werden der Apparatur 2 g Reaktionslösung entnommen, auf eine tarierte Aluschale gegeben und während 30 min bei 180 °C im Trockenofen getrocknet: Einwaage = 2.002 g viskose, weisse Emulsionslösung. Auswaage = 0.544 g milchigweisses, nicht klebriges Polymer. Trockensubstanz: 27.17% (TS_{100% theoretischer Umsatz}: 26.1 1%) = 104.1% (aufgrund von Lösungsmittelverlust während der Reaktion handelt es sich hierbei nicht um Umsatz). Quellverhalten: Etwas getrocknetes Polymer wird mit Wasser übergossen, wodurch das Polymer innert Minuten um ein Vielfaches anquellt und sich ablöst Die Permanenz scheint ungenügend zu sein. pH = 8.1.

**Vernetzungsversuch mit Katalysator-Stammlösung:** 2 g Reaktionslösung werden auf eine tarierte Aluschale gegeben und mit 7 g Wasser verdünnt. Dann werden 1 g Katalysator-Stammlösung (50 g/kg Magnesiumchlorid x 6 H₂O + 20 g/kg L-(+)-Weinsäure) zugegeben und während 30 min bei 180 °C im Trockenofen getrocknet. Es resultiert ein weisses, leicht brüchiges Polymer. Quellverhalten: Etwas getrocknetes Polymer wird mit Wasser übergossen, wodurch das Polymer innert Minuten anquellt. Das Polymer ist nur mässig vernetzt.

**Zusammenfassung:** Die radikalische Polymerisation mit mPEG-350-Methacrylat anstelle mPEG-1000-Methacrylat (P-005) verlief erfolgreich. Es konnte ein innert kürzester Zeit sehr gut quellbares Polymer synthetisiert werden. Die Permanenzeigenschaften des Polymers scheinen gegenüber P-005 jedoch nicht besser zu sein.

### Polymerverbindung P-011

Die Polymerverbindung P-011 wird analog zu P-010 hergestellt, jedoch mit 12% N-(Butoxymethyl)-acrylamid als Vernetzer anstatt nur 3% wie bisher, um die Permanenz zu erhöhen.

### Eduktlösung:

| *Einwaage* | *Produktbezeichnung* |
|---|---|
| 2.48 g | 2-Acryloylamino-2-methylpropan-sulfonsäure, >98.0%, Fluka |
| 3.30 g | mPEG-350-Methacrylat, >95%, Evonik Industries |
| 1.49 g | 2-Ethylhexyl-acrylat, 99%, stabilisiert, Acros Organics |
| 0.99 g | Cylink NBMA Monomer: N-(Butoxymethyl)-acrylamid, 81%, Cytec Industries |
| 0.25 g | Disponil AFX 1080, Cognis Deutschland GmbH & Co KG |
| 0.04 g | Natriumdodewlsulfat (SDS), p.A., Serva Feinbiochemika |
| 5.0 g | 2-Propanol, Schweizerhall, M = 60.10 g/mol, d = 0.785 g/ml, bp = 82 ° C |
| 10.0 g | ipropylenqlykol, d = 1.023 g/ml, bp = 90-95 °C/1 mmHg |
| 1.0 g | Natriumhydroxid 50%-ig (Wasserqehalt berücksichtigt) |
| 6.0 g | Wasser deionisiert |

Initiatorlösung, Apparatur wie in P-008. Vorhomogenisation, Synthese, In-Prozess-Kontrolle wie in P-010.

**Vernetzungsversuch mit Katalysator-Stammlösung:** 2 g Reaktionslösung werden auf eine tarierte Aluschale gegeben und mit 7 g Wasser verdünnt. Dann werden 1 g Katalysator-Stammlösung (50 g/kg Magnesiumchlorid x 6 H₂O + 20 g/kg L-(+)-Weinsäure) zugegeben und während 30 min bei 180 °C im Trockenofen getrocknet. Es resultiert ein weisses, leicht brüchiges Polymer. Quellverhalten: Etwas getrocknetes Polymer wird mit Wasser übergossen, wodurch das Polymer innert Minuten anquellt. Das Polymer scheint visuell recht gut vernetzt zu sein.

**Zusammenfassung:** Die radikalische Polymerisation mit 12% N-(Butoxymethyl)-acrylamid als Vernetzer anstatt nur 3% (P-010) verlief erfolgreich. Ein Vernetzungsversuch mit Katalysator-Stammlösung zeigte, dass ein innert Minuten quellbares, visuell recht gut vernetztes Polymer synthetisiert werden konnte.

### Polymerverbindung P-012

Bei P-012 handelt es sich um Poly(2-Acrylamido-2-methylpropan-natriumsulfonat-stat-2-Ethylhexyl-acrylat-stat-2-Hydroxyethyl-methacrylat-statmPEG-350-Methacrylat-stat-N-(Butoxymethyl)-acrylamid). Die Zusammensetzung ist analog zu P-010, jedoch mit nur 10% mPEG-350-Methacrylat, dafür aber mit 30% 2-Hydroxyethyl-methacrylat (HEMA). Da eine Lösungspolymerisation durchgeführt wird, wird ohne Emulgatoren gearbeitet.

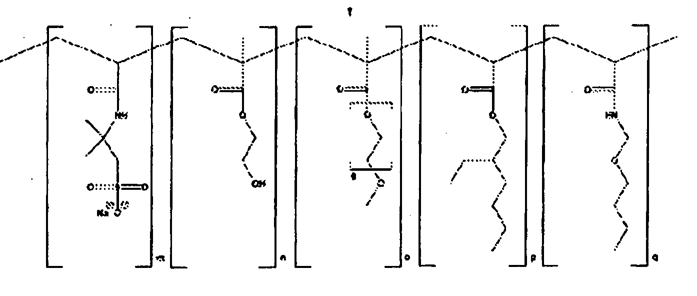

### Eduktlösung:

| *Einwaage* | *Produktbezeichnung* |
|---|---|
| 2.48 g | 2-Acryloylamino-2-methylpropan-sulfonsäure, >98.0%, Fluka |
| 0.83 g | mPEG-350-Methacrylat, >95%, Evonik Industries |
| 2.48 g | 2-Hydroxyethyl-methacrylat (HEMA), 97%, stabilisiert, Acros Organics, M = 130.15 g/mol, d = 1.07 g/ml, bp = 205-208 °C |
| 2.23 g | 2-Ethylhexyl-acrylat, 99%, stabilisiert, Acros Organics |
| 0.25 g | Cylink NBMA Monomer: N-(Butoxymethyl)-acrylamid, 81%, Cytec Industries |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 1.0 g | Natriumhydroxid 50%-ig (Wassergehalt berücksichtigt) |
| 6.0 g | Wasser deionisiert |

Initiatorlösung, Apparatur wie in P-008. Vorhomogenisation, Synthese, In-Prozess-Kontrolle wie in P-010. Quellverhalten: Etwas getrocknetes Polymer wird mit Wasser übergossen, wodurch das Polymer um ein Vielfaches anquellt und sich klumpig ablöst Die Permanenz scheint besser zu sein.

**Vernetzungsversuch mit Katalysator-Stammlösung:** Wie P-011. Es resultiert ein weisses, leicht brüchiges Polymer. Quellverhalten: Etwas getrocknetes Polymer wird mit Wasser übergossen, wodurch das Polymer innert Minuten anquellt. Das Polymer hat keine gute Permanenz, da es aufgrund des hohen HEMA-Anteils sehr brüchig ist.

### Polymerverbindung P-013

Bei P-013 handelt es sich analog zu P-012 um Poly(2-Acrylamido-2-methylpropan-natriumsulfonatstat-2-Ethylhexyl-acrylat-stat-2-Hydroxyethyl-methacrylat-statmPEG-350-Methacrylat-stat-N-(Butoxymethyl)-acrylamid). Zusätzlich wird der Vernetzer auf 12% erhöht. Wiederum wird die Quellbarkeit und die Vernetzung untersucht. Gegenüber P-012 werden die Emulgatoren wieder eingesetzt.

### Eduktlösung:

| *Einwaage* | *Produktbezeichnung* |
|---|---|
| 2.48 g | 2-Acrvloylamino-2-methylpropan-sulfonsäure, >98.0%, Fluka |
| 0.83 g | mPEG-350-Methacrylat, >95%, Evonik Industries, M = 418 g/mol, d = 1.08 g/ml |
| 2.48 g | 2-Hydroxyethyl-methacrylat (HEMA), 97%, stabilisiert, Acros Organics |
| 1.49 g | 2-Ethylhexyl-acrylat, 99%, stabilisiert, Acros Organics |
| 0.99 g | Cylink NBMA Monomer: N-(Butoxymethyl)-acrylamid, 81%, Cytec Industries |
| 0.25 g | Disponil AFX 1080, Coqnis Deutschland GmbH & Co KG |
| 0.04 g | Natriumdodecylsulfat (SDS), p.A., Serva Feinbiochemika |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 9 0-9 5 °C/1 mmHg |
| 1.0 g | Natriumhydroxid 50%-ig (Wassergehalt berücksichtigt) |
| 6.0 g | Wasser deionisiert |

Initiatorlösung, Apparatur wie in P-008. Vorhomogenisation, Synthese, In-Prozess-Kontrolle wie in P-010. Im Verlauf der Polymerisation wird die Reaktionslösung immer viskoser. Der Rührer bleibt stehen und es bildet sich ein sehr klebriges Gel. Die Reaktion wird abgebrochen.

**Zusammenfassung:** Die Erhöhung des Vernetzergehalts von 3% auf 12% gegenüber P-012 steigerte die Viskosität der Reaktionslösung derart, dass sich ein sehr klebriges, nicht rührbares Gel bildete. Es kann versucht werden, ohne Emulgatoren zu arbeiten, also eine Lösungspolymerisation durchzuführen.

### Polymerverbindung P-014

P-014 entspricht P-013, jedoch ohne Emulgatoren. Eduktlösung: Identisch zu P-013, ohne Emulgatoren (Disponil AFX 1080, Natriumdodecylsulfat). Initiatorlösung, Apparatur wie in P-008. Vorhomogenisation, Synthese, In-Prozess-Kontrolle wie in P-010. Im Verlauf der Polymerisation wird die Reaktionslösung immer viskoser. Der Rührer bleibt stehen und es bildet sich ein sehr klebriges Gel. Die Reaktion wird abgebrochen.

**Zusammenfassung:** Trotz Verzicht auf die Emulgatoren gegenüber P-013 musste die Polymerisation wiederum abgebrochen werden, da ein sehr klebriges, nicht rührbares Gel entstand. Die Polymerisation muss folglich verdünnt wiederholt werden.

### Polymerverbindung P-015

P-015 entspricht P-014. Um die Gelbildung des Polymers während der Reaktion zu vermeiden, wird die Eduktlösung mit Wasser 1:2 verdünnt Die Initiatorlösung wird unverdünnt eingesetzt, um die Reaktionsgeschwindigkeit ohne grosse Einbussen aufrecht zu erhalten.

**Synthese:** Eduktlösung wie P-013, ohne Emulgatoren (Disponil AFX 1080, Natriumdodecylsulfat). Initiatorlösung, Apparatur wie in P-008. Vorhomogenisation, Synthese, In-Prozess-Kontrolle wie in P-010. 15 g homogenisierte Eduktlösung wird im Schlenkrohr vorgelegt und durch Zugabe von 15 g Wasser 1:2 verdünnt. Mit den erhaltenen 30 g Lösung wird analog zu P-010 weiterverfahren. Quellverhalten: Etwas getrocknetes Polymer wird mit Wasser übergossen, wodurch das Polymer langsam anquellt und sich ablöst. Die Permanenz ist schlecht, das Polymer fällt brüchig auseinander.

**Vernetzungsversuch mit Katalysator-Stammlösung:** Wie P-011. Es resultiert ein weisses, nicht klebriges Polymer. Quellverhalten: Etwas getrocknetes Polymer wird mit Wasser übergossen, wodurch das Polymer anquellt und sich ablöst. Die Permanenz ist schlecht, das Polymer fällt körnig (brüchig) auseinander.

**Zusammenfassung:** Die Gelbildung des Polymers während der Reaktion konnte gegenüber P-013 und P-014 durch die Verdünnung (1:2) der Eduktlösung mit Wasser problemlos vermieden werden.

### Polymerverbindung P-016

P-016 beruht auf P-015. Zur Optimierung der Synthese wird direkt das AMPS-Natriumsalz anstelle des Sulfonsäure-Monomers eingesetzt. Dadurch kann auf die notwendige Neutralisation der Eduktlösung mit Natriumhydroxid verzichtet werden.

### Eduktlösung:

| *Einwaage* | *Produktbezeichnung* |
|---|---|
| 2.48 g | AMPS-Na 2403 Monomer 2-Acrylamido-2-methylpropan-natriumsulfonat (enthält ca. 50% Wasser), Lubrizol, M = 229.23 g/mol, d = 1.21 g/ml |
| 0.83 g | PEG-350-Methacrylat, >95%, Evonik Industries |
| 2.48 g | 2-Hydroxyethyl-methacrylat (HEMA), 97%, stabilisiert, Acros Organics |
| 1.49 g | 2-Ethylhexyl-acrylat, 99%, stabilisiert, Acros Organics |
| 0.99 g | Cylink NBMA Monomer: N-(Butoxymethyl)-acrylamid, 81%, Cytec Industries |
| 5.0 g | 2-Propanol, Schweizerhall, M = 60.10 g/mol, d = 0.785 g/ml, bp = 82 °C |
| 10.0 g | Dipropylengykol, d = 1 .023 g/ml, bp = 90-95 °C/1 mmHg |
| 4.5 g | Wasser deionisiert |

Initiatorlösung, Apparatur wie in P-008. Vorhomogenisation, Synthese, In-Prozess-Kontrolle wie in P-010. Quellverhalten: Etwas getrocknetes Polymer wird mit Wasser übergossen, wodurch das Polymer langsam anquellt und sich ablöst. Die Permanenz ist schlecht, das Polymer fällt brüchig auseinander.

**Vernetzungsversuch mit Katalysator-Stammlösung:** Wie in P-011. Es resultiert ein weisses, nicht klebriges Polymer. Quellverhalten: Etwas getrocknetes Polymer wird mit Wasser übergossen, wodurch das Polymer anquellt und sich ablöst. Die Permanenz ist schlecht, das Polymer fällt körnig (brüchig) auseinander.

**Zusammenfassung:** Der Umsatz und die Eigenschaften von P-016 entsprechen demjenigen von P-015. Dadurch kann auf das stark saure AMPS (2-Acrylamido-2-methylpropan-sulfonsäure) verzichtet werden, welches vor der Polymerisation bisher stets mit Natriumhydroxid neutralisiert werden musste. Der Ersatz durch das AMPS-Natriumsalz reduziert nicht nur deutlich den Arbeitsaufwand, sondern es kann auch dem Gesundheitsschutz des Mitarbeiters Rechnung getragen werden, indem auf das stark ätzende und reizende AMPS verzichtet werden kann.

### Polymerverbindung P-017

Bei P-017 handelt es sich um eine Wiederholung von P-016, jedoch mit V-50 als Initiator und bei einer Reaktionstemperatur von 70 °C.

### Initiatorlösung:

| *Einwaage* | *Produktbezeichnung* |
|---|---|
| 0.83 g | V-50, 2, 2'-Azobis-(2-amidinopropan)-dihydrochlorid, Wako, M = 271.19 g/mol, t_{1/2} = 35 min bei 80 °C |
| 0.8 g | Natriumhydroxid 50%-ig |
| 34.2 g | Wasser deionisiert |

Eingesetzt wird 1/10 der Initiatorlösung, was 1%-Gew. V-50 bezogen auf Monomere und Vernetzer entspricht.

Eduktlösung wie P-016. Apparatur wie in P-008. Vorhomogenisation, Synthese, In-Prozess-Kontrolle wie in P-010. Quellverhalten: Etwas getrocknetes Polymer wird mit Wasser übergossen, wodurch das Polymer langsam anquellt und sich ablöst. Die Permanenz ist schlecht, das Polymer fällt brüchig auseinander.

**Vernetzungsversuch mit Katalysator-Stammlösung:** Wie P-011. Es resultiert ein weisses, nicht klebriges Polymer. Quellverhalten: Etwas getrocknetes Polymer wird mit Wasser übergossen, wodurch das Polymer anquellt und sich ablöst. Die Permanenz ist schlecht, das Polymer fällt körnig (brüchig) auseinander. **Zusammenfassung:** Der Umsatz und die Eigenschaften von P-017 entsprechen denjenigen von P-015 und P-016. Das heisst der Ersatz des Initiators V-501 (4, 4'-Azobis-(4-cyano-valeriansäure) durch V-50 (2, 2'-Azobis-(2-amidinopropan)-dihydrochlorid) optimiert die Polymerisationsbedingungen weiter, indem auf die bisherige Neutralisation von V-501 mit Natriumhydroxid zur Erhöhung der Löslichkeit in Wasser verzichtet werden kann. V-50 ist nicht nur gut wasserlöslich, sondern hat auch eine tiefere Halbwertszeit bei entsprechender Temperatur. Dadurch kann die Polymerisationstemperatur bei gleichem Umsatz von anfänglich 90 °C bzw. 80 °C auf ca. 70 °C reduziert werden. Der kationische Charakter von V-50 hat offenbar keinen negativen Einfluss auf die Polymerisation.

### P-018 bis P-026: Screening mit verschiedenen mPEG-350-Methacrylat- und HEMA-Anteilen, sowie mit 0% Vernetzer

Verschiedene Varianten von Poly(2-Acrylamido-2-methylpropan-natriumsulfonat-stat-2-Ethylhexyl-acrylat-stat-2-Hydroxyethyl-methacrylat-stat-mPEG-350-Methacrylat-stat-N-(Butoxymethyl)-acrylamid)-Polymerverbindungen mit 0% Vernetzer wurden getestet.

Damit die anionische Ladung der Polymere während dem Beladungsprozess für die kationische Emulsion zugänglich ist, muss die Donorschicht in wässriger Umgebung gut quellbar sein. Vor allem bei Waschmaschinenapplikation ist insbesondere ein gutes Kurzzeit-Quellungsvermögen wichtig. Dieses wird ebenso wie die Permanenz vom Vernetzungsgrad beeinflusst. Mittels Screeningversuchen wurde die Zusammensetzung der erfindungsgemässen Polymere optimiert. Basierend auf P-017 haben die Polymere folgende Grund-Zusammensetzung: 30%-Gew. AMPS-Natriumsalz, 30%-Gew. 2-Ethylhexyl-acrylat, variabel 0-40%-Gew. mPEG-350-Methacrylat sowie 40-0%-Gew. 2-Hydroxyethyl-methacrylat (HEMA). Die Polymerisationen werden mit V-50 als Initiator bei einer Reaktionstemperatur von 70 °C durchgefü h rt.

### Eduktlösung:

| *Einwaage* | *Produktbezeichnung* |
|---|---|
| 24.8 g | AMPS-Na 2403 Monomer: 2-Acrylamido-2-methylpropan-natriumsulfonat |
| 0-16.5 g | mPEG-350-Methacrylat, >95%, Evonik Industries |
| 16.5-0 g | 2-Hydroxyethyl-methacrylat (HEMA), 97%, stabilisiert, Acros Organics |
| 12.4 g | 2-Ethylhexyl-acrylat, 99%, stabilisiert, Acros Organics |
| 0 g | Cylink NBMA Monomer: N-(Butoxymethyl)-acrylamid, 81%, Cytec Industries |
| 25.0 g | 2-Propanol, Schweizerhall, M = 60.10 g/mol, d = 0.785 g/ml, bp = 82 °C |
| 50.0 g | Dipropylenqlykol, d = 1.023 g/ml, bp = 90-95 °C/1 mmHg |
| 22.5 g | Wasser deionisiert |

| *Polymerverbindung* | *mPEG-350-Methacrylat* | *HEMA* | *Quellungsgrad (Nassgewicht über Trockengewicht)* |
|---|---|---|---|
| P-018 | 0%-Gew.; 0 g | 0%-Gew.; 1.65 g | 8% |
| P-019 | 5%-Gew.; 0.21 g | 35%-Gew.; 1.44 g | 2% |
| P-020 | 10%-Gew.; 0.41 g | 30%-Gew.; 1.24 g | 50% |
| P-021 | 15%-Gew.; 0.62 g | 25%-Gew.; 1.03 g | 772% |
| P-022 | 20%-Gew.; 0.83 g | 20%-Gew.; 0.83 g | 864% |
| P-023 | 25%-Gew.; 1.03 g | 15%-Gew.; 0.62 g | 811% |
| P-024 | 30%-Gew.; 1.24 g | 10%-Gew.; 0.41 g | 614% |
| P-025 | 35%-Gew.; 1.44 g | 5%-Gew.; 0.21 g | 332% |
| P-026 | 40%-Gew.; 1.65 g | 0%-Gew.; 0 g | 176% |

Apparatur wie in P-008. Vorhomogenisation wie in P-010. Initiatorlösung, Synthese, In-Prozess-Kontrolle wie in P-017. Die Trockensubstanz liegt bei allen Polymeren zwischen 12.3% und 16.5%. Die Polymere verändern sich von milchig-weiss (P-018) zu gold-bräunlich (P-026). Die Brüchigkeit der Polymere verändert sich von P-018 zu P-026 nicht. Alle Polymere sind nicht spröde. Die Klebrigkeit der Polymere verändert sich von leicht klebrig (P-018) zu klebrig (P-026) zu. Betreffend der Quellbarkeit der Polymere kann visuell keine eindeutige Tendenz festgestellt werden. Je höher der HEMA-Anteil (P-018), desto mehr quellen die Polymere auf der ganzen Fläche an, wohingegen bei den Polymeren mit höheren mPEG-350-Methacrylat-Anteilen (P-026) das Quellen von der Seite der Polymerfläche beginnt. Die Permanenz ist bei allen Polymeren sehr schlecht (0% Vernetzer). Einige Polymere scheinen aber etwas besser zusammen zu halten. Dies könnte an einer mPEG-350-DiMethacrylat-Verunreinigung liegen, welche als Nebenprodukt stets in den mPEG-350-Methacrylat-Monomeren vorhanden ist.

**Quellverhalten:** Die getrockneten Polymere in den Aluschalen (In-Prozess-Kontrolle) werden während 24 h mit nicht deionisierten Wasser überdeckt bei RT gelagert. Anschliessend werden die gequollenen Polymere über eine tarierte Filterspritze (Plastikspritze mit einem Kunststoffnetzchen, welche als Filter benutzt wird) während 2 min bei 1000 rpm abzentrifugiert, wodurch das überstehende Wasser abgetrennt wird. Durch Rückwiegen der Filterspritze wird das Nassgewicht der Polymere bestimmt.

**Zusammenfassung:** Die radikalischen Polymerisationen verliefen erfolgreich. Aufgrund der Lösungsmittelverluste während der Reaktion lagen die Trockensubstanzen (In-Prozess-Kontrolle) jeweils über den theoretisch möglichen Umsätzen. Daher wurden die Reaktionslösungen anschliessend jeweils mit Wasser auf eine Polymer-Konzentration von 12.0% verdünnt. Somit konnten innert kürzester Zeit sehr gut quellbare Polymere synthetisiert werden. Die Permanenzeigenschaften der Screeningreihe P-018 bis P-026 sind logischerweise sehr schlecht, da die Polymere 0% NBMA-Vernetzer enthalten. Damit ist die Bestimmung des Quellungsgrades mittels Abzentrifugieren bei der P-018-Screeningreihe nicht besonders aussagekräftig, da die Polymere mit 0% NBMA im Trockenofen (In-Prozess-Kontrolle) nicht oder nur undefiniert vernetzen konnten und daher die Filterspritze mehr oder weniger gut passieren konnten. Dennoch ergab die Bestimmung der Quellungsgrade bei einigen Polymeren eine Quellbarkeit von etwa 800% und mehr.

### P-027 bis P-035: Screening mit verschiedenen mPEG-350-Methacrylat- und HEMA-Anteilen, sowie mit 5% Vernetzer

Verschiedene Varianten von Poly(2-Acrylamido-2-methylpropan-natriumsulfonat-stat-2-Ethylhexyl-acrylat-stat-2-Hydroxyethyl-methacrylat-stat mPEG-350-Methacrylat-stat-N-(Butoxymethyl)-acrylamid)-Polymerverbindungen mit 5% Vernetzer wurden getestet.

Basierend auf P-017 haben die Polymere folgende Grund-Zusammensetzung: 30%-Gew. AMPS-Natriumsalz, 5%-Gew. NBMA-Vernetzer, 25%-Gew. 2-Ethylhexyl-acrylat, variabel 0 bis 40%-Gew. mPEG-350-Methacrylat sowie 40 bis 0%-Gew. 2-Hydroxyethyl-methacrylat (HEMA). Die Polymerisationen werden mit V-50 als Initiator bei einer Reaktionstemperatur von 70 °C durchgeführt.

### Eduktlösung:

| *Einwaage* | *Produktbezeichnung* |
|---|---|
| 24.8 g | AMPS-Na 2403 Monomer: 2-Acrylamido-2-methylpropan-natriumsulfonat |
| 0-16.5 g | mPEG-350-Methacrylat, >95%, Evonik Industries |
| 16.5-0 g | 2-Hydroxyethyl-methacrylat (HEMA), 97%, stabilisiert, Acros Organics |
| 10.3 g | 2-Ethylhexyl-acrylat, 99%, stabilisiert, Acros Organics |
| 2.1 g | Cylink NBMA Monomer: N-(Butoxymethyl)-acrylamid, 81%,Cytec Industries |
| 25.0 g | 2-Propanol, Schweizerhall, M = 60.10 g/mol, d = 0.785 g/ml, bp = 82 °C |
| 50.0 g | Dipropylenglykol, d = 1.023 g/ml, bp = 90-95 °C/1 mmHg |
| 22.5 g | Wasser deionisiert |

| *Polymer* | *mPEG-350-Methacrylat* | *HEMA* | *Quellungsgrad (Nassgewicht über Trockengewicht)* |
|---|---|---|---|
| P-027 | 0%-Gew.; 0 g | 40%-Gew.; 1.65 g | 06% |
| P-028 | 5%-Gew.; 0.21 g | 35%-Gew.; 1.44 g | 400% |
| P-029 | 1 0%-Gew.; 0.41 g | 30%-Gew.; 1.24 g | 381% |
| P-030 | 1 5%-Gew.; 0.62 g | 25%-Gew.; 1.03 g | 375% |
| P-031 | 20%-Gew.; 0.83 g | 20%-Gew.; 0.83 g | 361% |
| P-032 | 2,5%-Gew.; 1.03 g | 15%-Gew.; 0.62 g | 336% |
| P-033 | 30%-Gew.; 1.24 g | 0%-Gew.; 0.41 g | 343% |
| P-034 | 35%-Gew.; 1.44 g | %-Gew.; 0.21 g | 345% |
| P-035 | 40%-Gew.; 1.65 g | 0'%-Gew.; 0 g | 363% |

Apparatur, Versuchsablauf, Synthese, Quellversuche etc. sind analog zu P-018 bis P-026. Die Trockensubstanz liegt bei allen Polymeren zwischen 12.5% und 16.5%. Die Polymere verändern sich von leicht gold-bräunlich (P-027) zu gold-bräunlich (P-035). Die Brüchigkeit der Polymere verändert von sehr leicht spröde (P-027) zu nicht spröde (P-035). Die Klebrigkeit der Polymere verändert sich von sehr leicht klebrig (P027) zu leicht klebrig (P-035). Betreffend die Quellbarkeit der Polymere kann visuell keine eindeutige Tendenz festgestellt werden. Je höher der HEMA-Anteil (P-027), desto mehr quellen die Polymere auf der ganzen Fläche an, wohingegen bei den Polymeren mit höheren mPEG-350-Methacrylat-Anteilen (P-035) das Quellen von der Seite der Polymerfläche beginnt.

**Zusammenfassung:** Die radikalischen Polymerisationen verliefen erfolgreich. Die Quellungsgrade nach 24 h bei Raumtemperatur der Screeningreihe P-027 bis P-035 mit 5% NBMA-Vernetzer liegen zwischen 336% und 406%.

### P-036 bis P-044: Screening mit verschiedenen mPEG-350-Methacrylat- und HEMA-Anteilen, sowie mit 10% Vernetzer

Verschiedene Varianten von Poly(2-Acrylamido-2-methylpropan-natriumsulfonat-stat-2-Ethylhexyl-acrylat-stat-2-Hydroxyethyl-methacrylat-stat-mPEG-350-Methacrylat-stat-N-(Butoxymethyl)-acrylamid)-Polymerverbindungen mit 10% Vernetzer wurden getestet.

Basierend auf P-017 haben die Polymere folgende Grund-Zusammensetzung: 30%-Gew. AMPS-Natriumsalz, 10%-Gew. NBMA-Vernetzer, 20%-Gew. 2-Ethylhexyl-acrylat, variabel 0 bis 40%-Gew. mPEG-350-Methacrylat sowie 40 bis 0%-Gew. 2-Hydroxyethyl-methacrylat (HEMA). Die Polymerisationen werden mit V-50 als Initiator bei einer Reaktionstemperatur von 70 °C durchgeführt

### Eduktlösung:

| *Einwaage* | *Produktbezeichnung* |
|---|---|
| 24.8 g | AMPS-Na 2403 Monomer: 2-Acrylamido-2-methylpropan-natriumsulfonat |
| 0-16.5 g | mPEG-350-Methacrylat, >95%, Evonik Industries |
| 16.5-0 g | 2-Hydroxyethyl-methacrylat (HEMA), 97%, stabilisiert, Acros Organics |
| 8.3 g | 2-Ethylhexyl-acrylat, 99%, stabilisiert, Acros Organics |
| 4.1 g | Cylink NBMA Monomer: N-(Butoxymethyl)-acrylamid, 81%, Cytec Industries |
| 25.0 g | 2-Propanol, Schweizerhall, M = 60.10 g/mol, d = 0.785 g/ml, bp = 82 °C |
| 50.0 g | Dipropylenglykol, d = 1.023 g/ml, bp = 90-95 °C/1 mmHg |
| 22.5 g | Wasser deionisiert |

| *Polymer* | *mPEG-350-Methacrylat* | *HEMA* | *Quellungsgrad (Nassgewicht über Trockengewicht)* |
|---|---|---|---|
| P-036 | 0%-Gew.; 0 g | 40%-Gew.; 1.65 g | 261% |
| P-037 | 5%-Gew.; 0.21 g | 35%-Gew.; 1.44 g | 253% |
| P-038 | 10%-Gew.; 0.41 g | 30%-Gew.; 1.24 g | 242% |
| P-039 | 1 5%-Gew.; 0.62 g | 25%-Gew.; 1.03 g | 24% |
| P-040 | 20%-Gew.; 0.83 g | 0%-Gew.; 0.83 g | 38% |
| P-041 | 25%-Gew.; 1.03 g | 5%-Gew.; 0.62 g | 237% |
| P-042 | 30%-Gew.; 1.24 g | 10%-Gew.; 0.41 g | 242% |
| P-043 | 35%-Gew.; 1.44 g | 5%-Gew.; 0.21 g | 265% |
| P-044 | 40%-Gew.; 1.65 g | 0%-Gew.; 0 g | 278% |

Apparatur, Versuchsablauf, Synthese, Quellversuche etc. sind analog zu P-018 bis P-026. Die Trockensubstanz liegt bei allen Polymeren zwischen 12.2% und 14.3%. Die Polymere verändern sich von leicht gold-bräunlich (P-036) zu gold-bräunlich (P-044). Die Brüchigkeit der Polymere verändert von leicht spröde (P-036) zu nicht spröde (P-044). Die Klebrigkeit der Polymere verändert sich von P-036 zu P-044 nicht. Alle Polymere sind nicht klebrig. Betreffend der Quellbarkeit der Polymere kann visuell keine eindeutige Tendenz festgestellt werden. Je höher der HEMA-Anteil (P-036), desto mehr quellen die Polymere auf der ganzen Fläche an, wohingegen bei den Polymeren mit höheren mPEG-350-Methacrylat-Anteilen (P-044) das Quellen von der Seite der Polymerfläche beginnt.

**Zusammenfassung:** Die radikalischen Polymerisationen verliefen erfolgreich. Die Quellungsgrade nach 24 h bei Raumtemperatur der Screeningreihe P-036 bis P-044 mit 10% NBMA-Vernetzer liegen zwischen 224% und 278%.

### P-045 bis P-053: Screening mit verschiedenen mPEG-350-Methacrylat- und HEMA-Anteilen, sowie mit 15% Vernetzer

Verschiedene Varianten von Poly(2-Acrylamido-2-methylpropan-natriumsulfonat-stat-2-Ethylhexyl-acrylat-stat-2-Hydroxyethyl-methacrylat-stat-mPEG-350-Methacrylat-stat-N-(Butoxymethyl)-acrylamid)-Polymerverbindungen mit 15% Vernetzer wurden getestet.

Basierend auf P-017 haben die Polymere folgende Grund-Zusammensetzung: 30%-Gew. AMPS-Natriumsalz, 15%-Gew. NBMA-Vernetzer, 15%-Gew. 2-Ethylhexyl-acrylat, variabel 0 bis 40%-Gew. mPEG-350-Methacrylat sowie 40 bis 0%-Gew. 2-Hydroxyethyl-methacrylat (HEMA). Die Polymerisationen werden mit V-50 als Initiator bei einer Reaktionstemperatur von 70 °C durchgeführt.

### Eduktlösung:

| *Einwaage* | *Produktbezeichnung* |
|---|---|
| 24.8 g | AMPS-Na 2403 Monomer: 2-Acrylamido-2-methylpropan-natriumsulfonat |
| 0-16.5 q | mPEG-350-Methacrylat, >95%, Evonik Industries |
| 16.5-0 g | 2-Hydroxyethyl-methacrylat (HEMA), 97%, stabilisiert, Acros Organics |
| 6.2 g | 2-Ethylhexyl-acrylat, 99%, stabilisiert, Acros Organics |
| 6.2 q | Cylink NBMA Monomer: N-(Butoxymethyl)-acrylamid, 81%, Cytec Industries |
| 25.0 g | 2-Propanol, Schweizerhall, M = 60.10 g/mol, d = 0.785 g/ml, bp = 82 °C |
| 50.0 g | Dipropylenglykol, d = 1.023 q/ml, bp = 90-95 °C/1 mmHg |
| 22.5 g | Wasser deionisiert |

| *Polymer* | *mPEG-350-Methacrylat* | *HEMA* | *Quellungsgrad (Nassgewicht über Trockengewicht)* |
|---|---|---|---|
| P-045 | 0%-Gew.; 0 g | 40%-Gew.; 1.65 g | 219% |
| P-046 | 5%-Gew.; 0.21 g | 35%-Gew.; 1.44 g | 216% |
| P-047 | 10%-Gew.; 0.41 g | 30%-Gew.; 1.24 g | 205% |
| P-048 | 15%-Gew.; 0.62 g | 25%-Gew.; 1.03 g | 201% |
| P-049 | 20%-Gew.; 0.83 g | 20%-Gew.; 0.83 g | 206% |
| P-050 | 25%-Gew.; 1.03 g | 15%-Gew.; 0.62 g | 224% |
| P-051 | 30%-Gew.; 1.24 g | 10%-Gew.; 0.41 g | 226% |
| P-052 | 35%-Gew.; 1.44 g | 5%-Gew.; 0.21 g | 239% |
| P-053 | 40%-Gew.; 1.65 g | 0'%-Gew.; 0 g | 269% |

Apparatur, Versuchsablauf, Synthese, Quellversuche etc. sind analog zu P-018 bis P-026. Die Trockensubstanz liegt bei allen Polymeren zwischen 12.6% und 17.0%. Die Polymere verändern sich von leicht gold-bräunlich (P-045) zu gold-bräunlich (P-053). Die Brüchigkeit der Polymere verändert von spröde (P-045) zu leicht spröde (P-053). Die Klebrigkeit der Polymere verändert sich von P-045 zu P-053 nicht. Alle Polymere sind nicht klebrig. Betreffend der Quellbarkeit der Polymere kann visuell keine eindeutige Tendenz festgestellt werden. Je höher der HEMA-Anteil (P-045), desto mehr quellen die Polymere auf der ganzen Fläche an, wohingegen bei den Polymeren mit höheren mPEG-350-Methacrylat-Anteilen (P-053) das Quellen von der Seite der Polymerfläche beginnt.

**Zusammenfassung:** Die radikalischen Polymerisationen verliefen erfolgreich. Die Quellungsgrade nach 24 h bei Raumtemperatur der Screeningreihe P-045-053 mit 15% NBMA-Vernetzer liegen zwischen 201% und 269%.

### Polymerverbindung P-054

Die Durchführung der Screeningversuche P-018 bis P-053 mit variablen Gehalten von mPEG-350-Methacrylat und 2-Hydroxyethyl-methacrylat (HEMA) und unterschiedlichen NBMA-Vernetzergehalten ergab bei allen Polymeren gute Quellbarkeiten. Die Waschpermanenzen der mit den Polymeren imprägnierten PA-Gewebe sind eher ungenügend, jedoch über das gesamte Screening mit rund 80% Permanenzverlust konstant. Dies unabhängig von den NBMA-Vernetzergehalten von 5%, 10% und 15%, mit Ausnahme von 0% NBMA-Vernetzer (sehr schlechte Permanenz).

Überraschenderweise stellte sich aber heraus, dass die Polymere mit 30% und 35% mPEG-350-Methacrylat bzw. 10% und 5% 2-Hydroxyethyl-methacrylat (HEMA) bis zu einer Vernetzerkonzentration von 10% die beste Zugänglichkeit der anionischen Ladung aufweisen. Dies liegt daran, dass bei höheren HEMA-Gehalten die Netzwerkdichte zunimmt, da die Vernetzermoleküle mit HEMA vernetzen können.

Basierend auf der Zusammensetzung von P-042 wurde die Polymerisation mit nur 1% NBMA-Vernetzer wiederholt. Hierbei soll untersucht werden, ob insbesondere die Waschpermanenz wider erwarten besser ist als dies bei den Screeningversuchen mit 5%, 10% und 15% NBMA-Vernetzer der Fall ist. Vorversuche zeigten, dass bei der Verdünnung (1:2) der Eduktlösung mit Wasser eine Phasentrennung auftritt, die sich auch nicht durch den Einsatz von 3.0% Disponil AFX 1080 und 0.5% Natriumdodecylsulfat (SDS) beseitigen lässt. Daher wird die Polymerisation unverdünnt durchgeführt.

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | mPEG-350-Methacrylat, >95%, Evonik Industries |
| 0.83 g | 2-Hydroxyethyl-methacrylat (HEMA), 97%, stabilisiert, Acros Organics |
| 2.4 g | 2-Ethylhexyl-acrylat, 99%, stabilisiert, Acros Organics |
| 0.08 g | Cylink NBMA Monomer: N-(Butoxymethyl)-acrylamid, 81%, Cytec Industries |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 4.5 g | Wasser deionisiert |

Apparatur, Versuchsablauf, Synthese, Quellversuche etc. sind analog zu P-018 bis P-026. Initiatorlösung wie in P-017.

**Zusammenfassung:** Die radikalische Polymerisation mit nur 1 % NBMA-Vernetzer verlief erfolgreich. Der Umsatz liegt nach einer Reaktionszeit von 4 h bei 99.0%. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden. Das Polymer zerfällt dabei zwar in grobe Stücke, dennoch scheint die Permanenz visuell besser zu sein.

### Polymerverbindung P-055

Basierend auf der Zusammensetzung von P-042 wurde versuchsweise NMA-Vernetzermonomer (N-(Methylol)-acrylamid) anstelle von N-(Butoxymethyl)-acrylamid (NBMA) als Vernetzer verwendet, um die Elastizität der Donorschicht beim Quellen zu verbessern, wodurch sich die Schichtpermanenz aufgrund abnehmender Spannungsbrüche erhöhen sollte. Die Polymerisation wird in einem Schlenkrohr mit V-50 als Initiator bei einer Reaktionstemperatur von 70 °C durchgeführt. Um die tatsächlichen Permanenzen beurteilen zu können, wird NMA äquimolar bezogen auf NBMA (P-042) eingesetzt. Vorversuche zeigten, dass bei der Verdünnung (1:2) der Eduktlösung mit Wasser eine Phasentrennung auftritt, die sich auch nicht durch den Einsatz von 3.0% Disponil AFX 1080 und 0.5% Natriumdodecylsulfat (SDS) beseitigen lässt. Daher wird die Polymerisation unverdünnt durchgeführt.

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | PEG-350-Methacrylat, >95%, Evonik Industries |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 0.91 g, 4.33 mmol | Cylink NMA Monomer: N-(Methylol)-acrylamid, 48% in Wasser, Cytec Industries, M = 101.10 g/mol, d = 1.074 g/ml, bp = 100 °C (Wasser) |
| 5.0 q | 2-Propanol, Schweizerhall, M = 60.10 g/mol, d = 0.785 g/ml, bp = 82 °C |
| 10.0 g | Dipropylenglykol, d = 1.023 q/ml, bp = 90-95 °C/1 mmHg |
| 4.42 g | Wasser deionisiert |

Apparatur, Versuchsablauf, Synthese, Quellversuche etc. analog zu P-054. Initiatorlösung wie in P-017.

**Zusammenfassung:** Die radikalische Polymerisation mit dem NMA-Vernetzermonomer (N-(Methylol)-acrylamid) anstelle von N-(Butoxymethyl)-acrylamid (NBMA) verlief erfolgreich. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-056

Basierend auf der Zusammensetzung von P-042 wurde versuchsweise GMA-Vernetzermonomer (Glycidyl-methacrylat) anstelle von N-(Butoxymethyl)-acrylamid (NBMA) als Vernetzer verwendet, um die Elastizität der Donorschicht beim Quellen zu verbessern, wodurch sich die Schichtpermanenz aufgrund abnehmender Spannungsbrüche erhöhen sollte. Um die tatsächlichen Permanenzen beurteilen zu können, wird das Vernetzermonomer äquimolar bezogen auf NBMA (P-042) eingesetzt.

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | mPEG-350-Methacrylat, >95%, Evonik Industries |
| 0.83 g | 2-Hydroxyethyl-methacrylat (HEMA), 97%, stabilisiert, Acros Organics |
| 0.62 g | 2-Ethylhexyl-acrylat, 99%, stabilisiert, Acros Organics, d = 0.880g/ml, bp = 215 °C |
| 0.91 g, 4.33 mmol | Glycidyl-methacrylat (GMA), ≥97%, Fluka, M = 142.15 g/mol, d = 1.075 g/ml, bp = 192-197 °C |
| 5.0 g | 2-Propanol, Schweizerhall, M = 60.10 g/mol, d = 0.785 g/mol, bp = 82 °C |
| 10.0 g | Dipropylenqlykol, d = 1.023 q/ml, b p = 9 0-9 5. °C/ 1 mmHg |
| 4.71 g | Wasser deionisiert |

Apparatur, Versuchsablauf, Synthese, Quellversuche etc. analog zu P-054. Initiatorlösung wie in P-017.

**Zusammenfassung:** Die radikalische Polymerisation mit GMA verlief erfolgreich. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-057

Basierend auf der Zusammensetzung von P-042 wurde versuchsweise p-EMKO-TDI-o-HEMA-Vernetzermonomer anstelle von N-(Butoxymethyl)-acrylamid (NBMA) verwendet. p-EMKO-TDI-o-HEMA ist längerkettiger als die Vernetzer NBMA, NMA und GMA. Durch den daraus resultierenden grösseren Abstand der Polymerketten nach der Vernetzung erhöht sich die Elastizität der Donorschicht beim Quellen. Dadurch sollten die Spannungsbrüche im Polymer beim Quellen abnehmen und sich folglich die Schichtpermanenz erhöhen. Um die tatsächlichen Permanenzen beurteilen zu können, wird das Vernetzermonomer äquimolar bezogen auf NBMA (P-042) eingesetzt Vorversuche ergaben, dass bei der unverdünnten Polymerisation Gelbildung eintritt. Um dies zu vermeiden und dennoch eine einphasige Eduktlösung zu erhalten, wird diese nicht mit Wasser sondern mit 2-Propanol 1:2 verdünnt. Die Initiatorlösung wird unverdünnt eingesetzt, um die Reaktionsgeschwindigkeit ohne grosse Einbussen aufrecht zu erhalten.

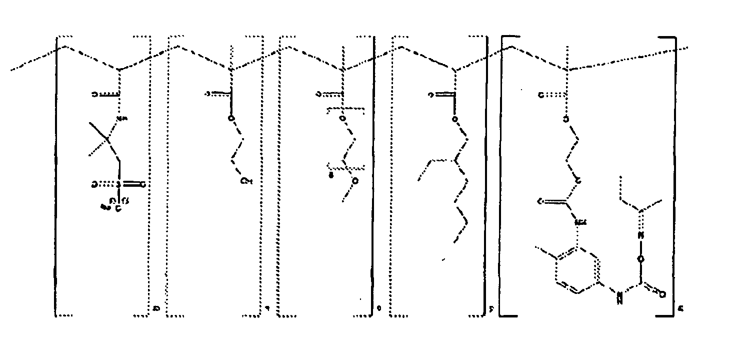

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | PEG-350-Methacrylat, >95%, Evonik Industries |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 1.69 g, 4.33 mmol | p-EMKO-TDI-o-HEMA, rein gemäss FT-IR, M = 391.42 g/mol |
| 5.0 g | 2-Propanol, Schweizerhall, M = 60.10 g/mol, d = 0.785 g/ml, bp = 82 °C |
| 10.0 g | Dipropylenqlykol, d = 1.023 g/ml, bp = 90-95 °C/1 mmHg |
| 3.64 g | Wasser deionisiert |

Apparatur, Versuchsablauf, Synthese, Quellversuche etc. analog zu P-054. Initiatorlösung wie in P-017.

**Synthese des Vernetzermonomers:** Die unterschiedliche Reaktivität der beiden Isocyanatgruppen von 2, 4-Toluylendiisocyanat eröffnet einen Weg zu einem Vernetzermonomer, indem man in einem ersten Reaktionsschritt die Isocyanatgruppe in p-Stellung selektiv mit einer EMKO-Schutzgruppe blockiert und anschliessend in einem zweiten Reaktionsschritt die Umsetzung der verbliebenen Isocyanatgruppe mit der radikalisch polymerisierbaren Einheit 2-Hydroxyethyl-methacrylat durchführt. Es muss unbedingt mit trockenem Heptan als Lösungsmittel gearbeitet werden, damit das p-EMKO-TDI-Addukt der 1. Stufe nicht ausölt, sondern als Kristallnadeln ausfällt. Dadurch wird das Monoaddukt der Reaktion entzogen, damit die zweite NCO-Gruppe von 2, 4-TDI nicht ebenfalls mit EMKO abreagieren kann (Selektivität gemäss NMR >93%). Die Verwendung von DABCO als Katalysator ist notwendig, um die Reaktivität der OH-Gruppen von HEMA zu erhöhen (OH-Gruppen sind gegenüber Aminen etwa 4000 mal weniger nukleophil). Literaturquelle: Duschek, G. K., Teilfluorierte und reaktive Polymere für die ölabweisende Oberflächenmodifikation von Baumwolle und Cellulose, Dissertation Universität Ulm, 1997, 64-69/178.

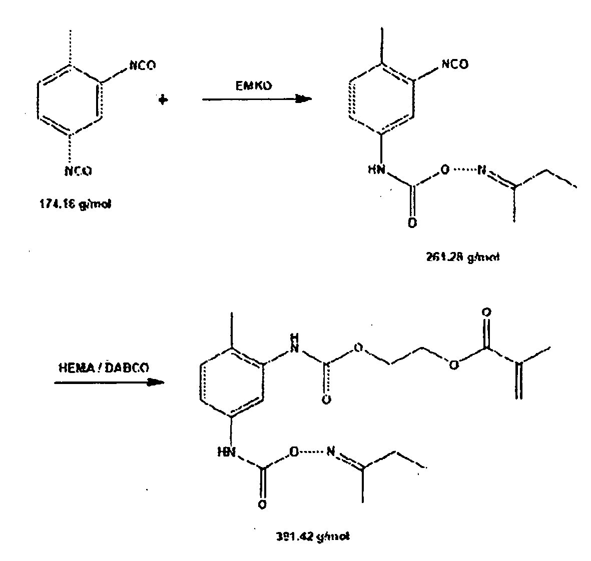

**Zusammenfassung:** Die radikalische Polymerisation mit p-EMKOTDI-o-HEMA-Vernetzermonomer verlief erfolgreich. Es konnte ein innert kürzester Zeit sehr gut quellbares Polymer synthetisiert werden.

### Polymerverbindung P-058

Basierend auf der Zusammensetzung von P-042 wurde versuchsweise (EMKO-2-(N-(tert.-Butyl){[(3-isocyanato-1, 5, 5-trimethylcyclohexyl)methyl]amino}-carbonylamino)ethyl-methacrylat) als Vernetzermonomer anstelle von NBMA verwendet. EMKO-2-(N-(tert.-Butyl){[(3-isocyanato-1, 5, 5-trimethylcyclohexyl)methyl]amino}-carborlylamino)ethyl-methacrylat ist längerkettiger als die Vernetzer NBMA, NMA und GMA. Durch den daraus resultierenden grösseren Abstand der Polymerketten nach der Vernetzung erhöht sich die Elastizität der Donorschicht beim Quellen. Dadurch sollten die Spannungsbrüche im Polymer beim Quellen abnehmen und sich folglich die Schichtpermanenz erhöhen. Um die tatsächlichen Permanenzen beurteilen zu können, wird das Vernetzermonomer äquimolar bezogen auf NBMA (P-042) eingesetzt. Vorversuche ergaben, dass bei der unverdünnten Polymerisation Gelbildung eintritt. Um dies zu vermeiden und dennoch eine einphasige Eduktlösung zu erhalten, wird diese nicht mit Wasser sondern mit 2-Propanol 1:2 verdünnt. Die Initiatorlösung wird unverdünnt eingesetzt, um die Reaktionsgeschwindigkeit ohne grosse Einbussen aufrecht zu erhalten.

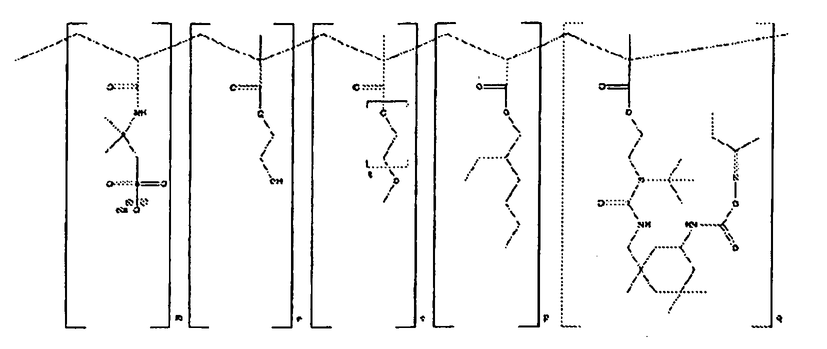

### Eduktlösung:

| *Einwaage* | *Produktbezeichnung* |
|---|---|
| 4.96 g | MPS-Na 2403 Monomer: 2-Acrylamido-2-methylpropan-natriumsulfonat |
| 2.48 g | PEG-350-Methacrylat, >95%, Evonik Industries |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 2.14 g, 4.33 mmol | EMKO-Alkenylisocyanat: EMKO-2-(N-(tert.-Butyl){[(3-isocyanato-1, 5, 5-trimethylcyclohexyl)-methyl]amino}-carbonylamino)ethylMethacrylat, rein gemäss FT-IR, M = 494.67 g/mol |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 3.19 g | Wasser deionisiert |

### Apparatur, Versuchsablauf, Synthese, Quellversuche etc. analog zu P-054. Initiatorlösung wie in P-017.

**Synthese des Vernetzermonomers:** Die unterschiedliche Reaktivität der beiden Isocyanatgruppen von Isophorondiisocyanat wird im ersten Reaktionsschritt genutzt und mit N-(tert.-Butylamino)-ethylmethacrylat umgesetzt. Die Reaktionstemperatur wird dabei bei maximal 30 °C gehalten, um die Selektivität aufrecht zu erhalten (bei höheren Temperaturen steigt die Reaktivität der andern NCO-Gruppe an). In einem zweiten Reaktionsschritt wird die verbliebene Isocyanatgruppe mit Ethylmethylketoxim (EMKO) blockiert, um beim Vernetzen im Spannrahmen eine vorzeitige Reaktion mit der wässrigen Flotte beim Trocknen zu vermeiden. Somit wird das geschützte Isocyanat erst beim Kondensieren deblockiert und für die Vernetzungsreaktion freigegeben. Auf die Verwendung eines Katalysator kann bei dieser Synthese verzichtet werden, da Amine (N-(tert.-Butylamino)ethyl-methacrylat) etwa 4000 mal nukleophiler als OH-Gruppen (vgl. 2. Stufe bei p-EMKOTDI-o-HEMA-Synthese) sind. Dies erhöht generell die Lagerstabilität der späteren, wässrigen Polymerdispersion, da Katalysatorspuren vermieden werden können. Die Reaktion findet zudem in Substanz, d.h. lösungsmittelfrei statt, was die gesamte Synthese und Aufarbeitung zusätzlich vereinfacht. Literaturquelle: Degussa AG - Coatings & Colorants, VESTANAT IPDI - Eigenschaften & Handling, Produktinformation 43.01.062d/02.06/500/jd/g3, 2009, 1-16; Knebel, J., Breiner, C., Schmitt, B., "Neues polymerisierbares Isocyanat und Polymere, enthaltend dieses Isocyanat", WO 2009/024493 A2.

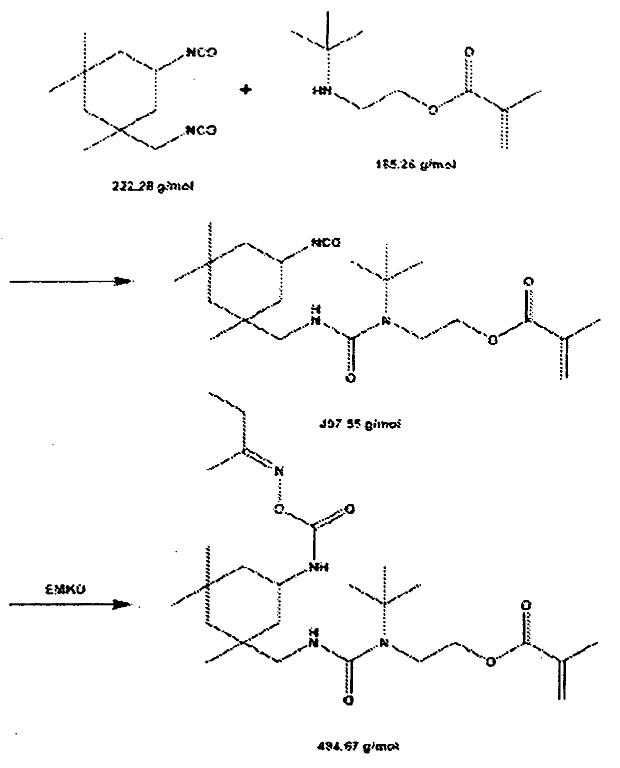

**Zusammenfassung:** Die radikalische Polymerisation für das Vernetzerscreening mit dem EMKO-Alkenylisocyanat-Vernetzermonomer verlief erfolgreich. Es konnte ein innert kürzester Zeit sehr gut quellbares Polymer synthetisiert werden.

### Polymerverbindung P-059

mPEG-methacrylate haben ein Optimum zwischen Wasserabsorption und Bruchdehnung bei n = 3 Ethylenoxideinheiten. Die Verbindung P-059 wird analog zu P-042 hergestellt, mit Ethyltriglykol-methacrylat (ETMA) anstelle von mPEG-35o-Methacrylat (n = 8) als hydrophiles Monomer.

Ethyltriglykol-methacrylat (ETMA) hat gegenüber Methyltriglykol-methacrylat den Vorteil, dass es kommerziell in grossen Mengen erhältlich ist. Literaturquelle: Kumakura, M., Kaetsu, I., Physical characterization and molecular structure of hydrophilic polymers obtained by radiation cast-polymerization of methoxypolyethyleneglycole Methacrylate monomers for biomedical applications, Journal of Materials Science (18), 1983, 2430-2436.

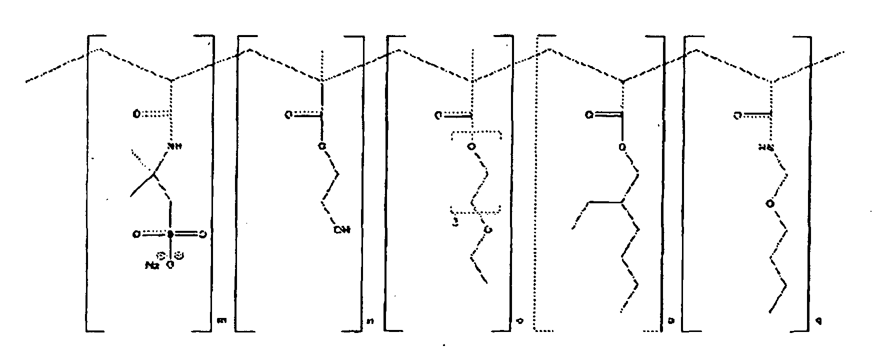

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | Visiomer ETMA: Ethyltriglykol-methacrylat (ETMA), >97%, Evonik Industries, M = 246.30 g/mol, d = 1.02 g/ml, bp = 292 °C |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 0.83 g | Cylink NBMA Monomer: N-(Butoxymethyl)-acrylamid >82%, Cytec Industries |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 4.5 g | Wasser deionisiert |

Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst. Die Permanenz ist schlecht, das Polymer fällt körnig (brüchig) auseinander.

**Zusammenfassung:** Die radikalische Polymerisation mit Ethyltriglykol-methacrylat (ETMA) anstelle von mPEG-350-Methacrylat als hydrophiles Monomer verlief erfolgreich. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-060

Basierend auf der Zusammensetzung von P-042 wird versucht, die Natrium-Gegenionen von AMPS durch Lithium-Ionen zu ersetzen. Dies aufgrund der Vermutung, dass die Natrium-Gegenionen die Kristallinität und die Brüchigkeit der Polymere erhöhen, wodurch die Polymere weniger elastisch werden. Dazu wird das AMPS-Natriumsalz durch reines AMPS ersetzt und dieses mit Lithiumhydroxid neutralisiert. Hierbei soll untersucht werden, ob durch die kleineren und mobileren Lithium-Gegenionen die Brüchigkeit der Polymere beim Quellen mit Wasser abnimmt, was die Waschpermanenz positiv beeinflussen sollte. Die Polymerisation wird unverdünnt durchgeführt.

Bei der Durchführung der AMPS-Neutralisationen ist generell zu beachten, dass diese unter Kühlung (<10 °C) stattfinden müssen, da AMPS unter erhöhten Temperaturen zur Autopolymerisation neigt und Basen eine Michaeladdition an den Acrylatmonomeren auslösen können. Aus denselben Gründen muss daher auch ein pH-Bereich zwischen 7.0-7.5 eingehalten werden, da bei pH < 7.0 die Autopolymerisation und bei pH > 7.5 die Michaeladdition begünstigt wird.

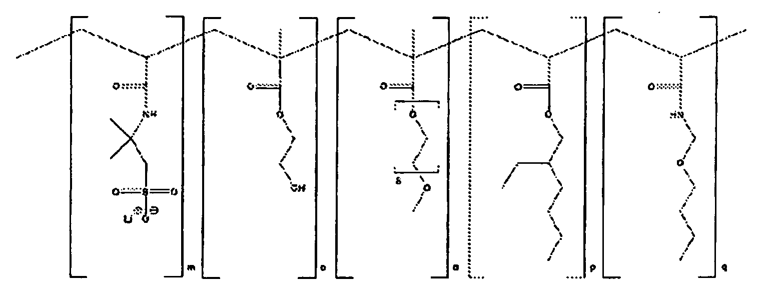

### AMPS-Lithium-Lösung:

| *Einwaage* | *Produktbezeichnung* |
|---|---|
| 4.52 g, 21.81 mmol | AMPS 2401 Monomer: 2-Acrylamido-2-methylpropansulfonsäure, >99.0%, Lubrizol, M = 207.25 g/mol |
| 0.92 g | Lithiumhydroxid Monohydrat, ≥99%, Fluka |
| 4.56 g | Wasser deionisiert |

3 g Wasser werden vorgelegt. Unter Rühren wird nun das AMPS hinzugegeben und mit dem Lithiumhydroxid Monohydrat neutralisiert. Zur Vermeidung der Autopolymerisation und Michaeladdition wird mittels Kühlbad die stark exotherme Neutralisationsreaktion bei einer Reaktionstemperatur von maximal 10 °C gehalten. Abschliessend wird die Lösung mit dem restlichen Wasser verdünnt. Es ergeben sich 10 g Lösung, pH = 7.

### Eduktlösung:

| *Einwaage* | *Produktbezeichnung* |
|---|---|
| 4.96 g | AMPS-Lithium-Lösung: (äquimolar zu AMPS aus P-042) |
| 2.48 g | mPEG-350-Methacrylat, >95%, Evonik Industries |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 0.83 g | Cylink NBMA Monomer: N-(Butoxymethyl)-acrylamid >82%, Cytec Industries |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, bp = 90-95 °C/1 mmHg |
| 4.5 g | Wasser deionisiert |

Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst. Das Polymer zerfällt in kleine Stücke, die Permanenz scheint schlecht zu sein.

**Zusammenfassung:** Die radikalische Polymerisation mit AMPS-Lithium anstelle von AMPS-Natrium verlief erfolgreich. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-061

Basierend auf der Zusammensetzung von P-042 wird versucht, die Natrium-Gegenionen von AMPS durch Ammonium-Ionen zu ersetzen. Dazu wird reines AMPS mit Ammoniumhydroxid neutralisiert, um Ammonium-Gegenionen zu erzeugen.

### AMPS-Ammonium-Lösung:

| *Einwaage* | *Produktbezeichnung* |
|---|---|
| 4.52 g, 21.81 mmol | AMPS 2401 Monomer: 2-Acrylamido-2-methylpropansulfonsäure, >99.0%, Lubrizol, M = 207.25 g/mol |
| 1.49 g | Ammoniumhydroxid-Lösung, 25% NH₃ in Wasser, Fluka |
| 3.99 g | Wasser deionisiert |

3 g Wasser werden vorgelegt. Unter Rühren wird nun das AMPS hinzugegeben und mit mit der Ammonium-Lösung neutralisiert. Zur Vermeidung der Autopolymerisation und Michaeladdition wird mittels Kühlbad die stark exotherme Neutralisationsreaktion bei einer Reaktionstemperatur von maximal 10 °C gehalten. Abschliessend wird die Lösung mit dem restlichen Wasser verdünnt. Es ergeben sich 10 g Lösung, pH = 7.

Eduktlösung wie P-059, mit 4.96 g AMPS-Ammonium-Lösung anstelle der AMPS-Lithium-Lösung. Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst. Das Polymer zerfällt zwar in grobe Stücke, die Permanenz scheint dennoch schlecht zu sein.

**Zusammenfassung:** Die radikalische Polymerisation mit AMPS-Ammonium anstelle von AMPS-Natrium verlief erfolgreich. Der Umsatz liegt nach einer Reaktionszeit von 4 h bei 99.5%. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-062

Basierend auf der Zusammensetzung von P-042 wird versucht, die Natrium-Gegenionen von AMPS durch Triethylammonium-Ionen zu ersetzen. Dazu wird reines AMPS mit Triethylamin neutralisiert, um den Einfluss der relativ voluminösen Ethylgruppen von Triethylammonium gegenüber den Lithium-, Natrium- und Ammonium-Gegenionen zu untersuchen.

### AMPS-Triethylammonium-Lösung:

| *Einwaage* | *Produktbezeichnung* |
|---|---|
| 4.52 g, 21.81 mmol | AMPS 2401 Monomer: 2-Acrylamido-2-methylpropansulfonsäure, >99.0%, Lubrizol, M = 207.25 g/mol |
| 2.21 g | Triethylamin, ≥98%, Fluka |
| 3.27 g | Wasser deionisiert |

3 g Wasser werden vorgelegt Unter Rühren wird nun das AMPS hinzugegeben und mit Triethylamin neutralisiert. Zur Vermeidung der Autopolymerisation und Michaeladdition wird mittels Kühlbad die stark exotherme Neutralisationsreaktion bei einer Reaktionstemperatur von max. 70 °C gehalten. Abschliessend wird die Lösung mit dem restlichen Wasser verdünnt. Es ergeben sich 10 g Lösung, pH = 7.

Eduktlösung wie P-059, mit 4.96 g AMPSTriethylammonium-Lösung anstelle der AMPS-Lithium-Lösung. Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst. Das Polymer zerfällt zwar in grobe Stücke, die Permanenz scheint dennoch schlecht zu sein.

**Zusammenfassung:** Die radikalische Polymerisation mit AMPS-Triethylammonium anstelle von AMPS-Natrium verlief erfolgreich. Der Umsatz liegt nach einer Reaktionszeit von 4 h bei 93.0%. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-063

Basierend auf der Zusammensetzung von P-042 wird versucht, die Natrium-Gegenionen von AMPS durch 1-Methylimidazolium-lonen zu ersetzen. Dazu wird reines AMPS mit 1-Methylimidazol neutralisiert, um 1-Methylimidazolium-Gegenionen zu erzeugen.

### AMPS-1-Methylimidazolium-Lösung:

| *Einwaage* | *Produktbezeichnung* |
|---|---|
| 4.52 g, 21.81 mmol | AMPS 2401 Monomer 2-Acrylamido-2-methylpropansulfonsäure, >99.0%, Lubrizol, M = 207.25 g/mol |
| 1.79 g | 1-Methylimidazol, ≥99%, Fluka |
| 3.69 g | Wasser deionisiert |

3 g Wasser werden vorgelegt. Unter Rühren wird nun das AMPS hinzugegeben und mit 1-Methylimidazolium neutralisiert. Zur Vermeidung der Autopolymerisation und Michaeladdition wird mittels Kühlbad die stark exotherme Neutralisationsreaktion bei einer Reaktionstemperatur von maximal 10 °C gehalten. Abschliessend wird die Lösung mit dem restlichen Wasser verdünnt. Es ergeben sich 10 g Lösung, pH = 7.

Eduktlösung wie P-059, mit 4.96 g AMPS-1-Methylimidazolium-Lösung anstelle der AMPS-Lithium-Lösung. Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst Das Polymer zerfällt zwar in grobe Stücke, die Permanenz scheint dennoch schlecht zu sein.

**Zusammenfassung:** Die radikalische Polymerisation mit AMPS-1-Methylimidazolium anstelle von AMPS-Natrium verlief erfolgreich. Der Umsatz liegt nach einer Reaktionszeit von 4 h bei 93.2%. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-064

Basierend auf der Zusammensetzung von P-042 wird versucht, die Na+-Gegenionen von AMPS durch 4-Methylmorpholinium-Ionen zu ersetzen. Dazu wird reines AMPS mit 4-Methylmorpholin neutralisiert, um 1-Methylimidazolium-Gegenionen zu erzeugen.

### AMPS-4-Methylmorpholinium-Lösung:

| *Einwaage* | *Produktbezeichnung* |
|---|---|
| 4.52 g, 21.81 mmol | AMPS 2401 Monomer: 2-Acrylamido-2-methylpropansulfonsäure, >99.0%, Lubrizol, M = 207.25 g/mol |
| 1.79 g | 4-Methylmorpholin, 99%, Acros Organics |
| 3.27 g | Wasser deionisiert |

3 g Wasser werden vorgelegt. Unter Rühren wird nun das AMPS hinzugegeben und mit 4-Methylmorpholin neutralisiert. Zur Vermeidung der Autopolymerisation und Michaeladdition wird mittels Kühlbad die stark exotherme Neutralisationsreaktion bei einer Reaktionstemperatur von maximal 10 °C gehalten. Abschliessend wird die Lösung mit dem restlichen Wasser verdünnt. Es ergeben sich 10 g Lösung, pH = 7.

Eduktlösung wie P-059, mit 4.96 g AMPS-4-Methylmorpholin-Lösung anstelle der AMPS-Lithium-Lösung. Apparatur, Versuchsablauf, Synthese, Quellversuche etc. sind analog zu P-054. Initiatorlösung wie in P-017. Quellverhalten: Etwas getrocknetes Polymer wird mit Wasser übergossen, wodurch das Polymer innert Minuten anquellt und sich ablöst. Das Polymer zerfällt zwar in grobe Stücke, die Permanenz scheint dennoch schlecht zu sein.

**Zusammenfassung:** Die radikalische Polymerisation mit AMPS-4-Methylimidazolium anstelle von AMPS-Natrium verlief erfolgreich. Der Umsatz liegt nach einer Reaktionszeit von 4 h bei 92.7%. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-065

Basierend auf der Zusammensetzung von P-054 wird die Polymerisation anstelle des NBMA-Vernetzers mit 1% N, N'-Methylen-bis-acrylamid (MBAm) wiederholt Hierbei soll untersucht werden, ob die Brüchigkeit der Polymere beim Quellen mit Wasser abnimmt, was die Waschpermanenz positiv beeinflussen sollte. Die Polymerisation wird unverdünnt durchgeführt.

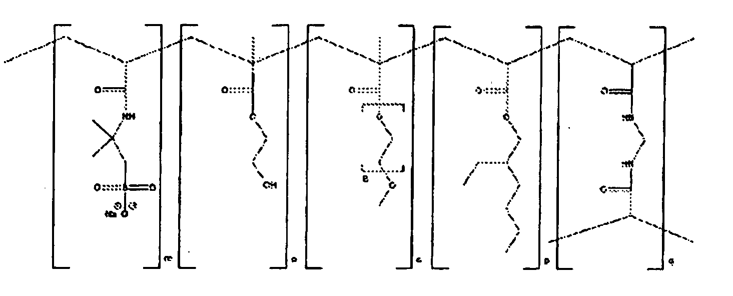

### Eduktlösung:

| *Einwaage* | *Produktbezeichnung* |
|---|---|
| 4.96 g | 2403 Monomer: 2-Acrylamido-2-methylpropan-natriumsulfonat |
| 2.48 g | AMPS-Na mPEG-350-Methacrylat, >95%, Evonik Industries |
| 0.83 g | 2-Hydroxyethyl-methacrylat (HEMA), 97%, stabilisiert, Acros Organics |
| 2.4 g | 2-Ethylhexyl-acrylat, 99%, stabilisiert, Acros Organics |
| 0.08 g | N, N'-Methylen-bis-acrylamid (MBAm), ≥99%, Fluka, M = 154.17 g/mol |
| 5.0 g | 2-Propanol, Schweizerhall, M = 60.10 g/mol, d = 0.785 g/ml, bp = 82 ° C |
| 10.0 g | Dipropylenglykol, d = 1.023 g/ml, b p = 9 0-9 5 °C/1 mmHg |
| 4.5 g | Wasser deionisiert |

Apparatur, Versuchsablauf, Synthese, Quellversuche etc. sind analog zu P-054. Quellverhalten: Etwas getrocknetes Polymer wird mit Wasser übergossen, wodurch das Polymer innert Minuten filmmässig anquellt und sich kaum ablöst. Das Polymer zerfällt nicht in grobe Stücke, es scheint visuell recht gut vernetzt zu sein.

**Zusammenfassung:** Die radikalische Polymerisation mit 1% N, N'-Methylen-bis-acrylamid (MBAm) verlief erfolgreich. Allerdings erhöhte sich die Viskosität der Reaktionslösung durch das zweifach funktionalisierte N, N'-Methylen-bis-acrylamid aufgrund der Netzwerkbildung während des Polymerisationsprozesses deutlich. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Polymerverbindung P-066

Basierend auf der Zusammensetzung von P-042 wird die Polymerisation mit 9% NBMA-Vernetzer und 1% N, N'-Methylen-bis-acrylamid (MBAm) wiederholt. Hierbei soll untersucht werden, ob die Brüchigkeit der Polymere beim Quellen mit Wasser abnimmt, was die Waschpermanenz positiv beeinflussen sollte. Die Polymerisation wird unverdünnt durchgeführt.

### Eduktlösung:

| *Einwaage* | *Produktbezeichnung* |
|---|---|
| 4.96 g | AMPS-Na 2403 Monomer: 2-Acrylamido-2-methylpropan-natriumsulfonat |
| 2.48 g | mPEG-350-Methacrylat, >95%, Evonik Industries |
| 0.83 g | 2-Hydroxyethyl-methacrylat (HEMA), 97%, stabilisiert, Acros Organics |
| 1.65 g | 2-Ethylhexyl-acrylat, 99%, stabilisiert, Acros Organics |
| 0.74 g | Cylink NBMA Monomer: N-(Butoxymethyl)-acrylamid >82%, Cytec Industries |
| 0.08 g | N, N'-Methylen-bis-acrylamid (MBAm), ≥99%, Fluka, M = 154.17 g/mol |
| 5.0 g | 2-Propanol, Schweizerhall, M = 60.10 g/mol, d = 0.785 g/ml, bp = 82 °C |
| 10.0 g | Dipropylenglykol, d = 1.023 g/ml, b p = 90-95 °C/1 mmHg |
| 4.5 g | Wasser deionisiert |

Apparatur, Versuchsablauf, Synthese, Quellversuche etc. sind analog zu P-054. Quellverhalten: Etwas getrocknetes Polymer wird mit Wasser übergossen, wodurch das Polymer innert Minuten anquellt und sich ablöst. Das Polymer zerfällt in feine Stücke, die Permanenz scheint sehr schlecht zu sein.

**Zusammenfassung:** Die radikalische Polymerisation mit 9% N-(Butoxymethyl)acrylamid und 1% N, N'-Methylen-bis-acrylamid (MBAm) als Vernetzermonomere verlief erfolgreich. Allerdings erhöhte sich die Viskosität der Reaktionslösung durch das zweifach funktionalisierte N, N'-Methylen-bis-acrylamid aufgrund der Netzwerkbildung während des Polymerisationsprozesses derart, dass eine gelartige Lösung entstand. Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Scale-up P-042 Nr. 1

Die Polymerisation wird gegenüber dem Versuch P-042 nicht als 12%-ige Lösungspolymerisation, sondern als 30%-ige radikalische Emulsionspolymerisation in einer 1 I-Glasapparatur mit V-50 als Initiator bei einer Reaktionstemperatur von 70 °C durchgeführt. Zudem wird Disponil AFX und Natriumdodecylbenzolsulfonat als zusätzlicher Emulgator hinzugefügt. Es soll untersucht werden, ob sich P-042 als 0.5 kg Scale-up mittels Emulsionspolymerisation problemlos herstellen lässt.

### Eduktlösung:

| *Einwaage* | *Produktbezeichnung* |
|---|---|
| 90.0 g | AMPS-Na 2403 Monomer: 2-Acrylamido-2-methylpropan-natriumsulfonat |
| 45.0 g | mPEG-350-Methacrylat, >95%, Evonik Industries |
| 15.0 g | 2-Hydroxyethyl-methacrylat (HEMA), 97%, stabilisiert, Acros Organics |
| 30.0 g | 2-Ethylhexyl-acrylat, 99%, stabilisiert, Acros Organics |
| 15.0 g | Cylink NBMA Monomer: N-(Butoxymethyl)-acrylamid, 81 %, Cytec Industries |
| 4.5 g | Disponil AFX 1080, 80%, Cognis |
| 3.0 g | Natriumdodecylsulfat (SDS), p.A., Serva Feinbiochemika, M = 288.38 g/mol |
| 5.0 g | Natriumdodecylbenzolsulfonat-Lösung, 30% in Wasser, M = 348.48 g/mol |
| 2 5.0 g | Dipropylenglykol, d = 1.023 g/ml, bp = 90-95 °C/1 mmHg |
| 222.8 g | Wasser deionisiert |

Initiatorlösung wie in P-017. Vorhomogenisation: Die Eduktlösung (pH = 5.7) wird fünf Mal bei 600 bar durch den Hochdruck-Homogenisierer gefördert, wodurch eine Emulsion entsteht. Apparatur: 1 I-Vierhalsrundkolben mit Rührwerk, Rückflusskühler, Septum und Temperaturfühler. Ein Abgang mit Hahn auf dem Rückflusskühler dient dem Evakuieren und Belüften mit Stickstoff.

Synthese: 455 g homogenisierte Eduktlösung wird in der Apparatur vorgelegt und mittels Heizpilz auf 70 °C erhitzt. Bei Erreichen von etwa 60 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 25 g Initiatorlösung durch das Septum zudosiert. Es ist nur eine leichte Exothermie feststellbar. Die Reaktionstemperatur wird bei 70 °C konstant gehalten. Nach etwa 10 min wird die Reaktionsmischung hochviskos und polymerisiert dann schlagartig komplett durch, wobei das gesamte Wasser im Ansatz vom gebildeten Polymer absorbiert wird und somit ein fester, gelartiger Polymerblock im Reaktor zurückbleibt. Die 30%-ige radikalische Emulsionspolymerisation ist zu konzentriert.

### Scale-up P-042 Nr. 2

Synthese analog zum Scale-up P-042 Nr. 1, jedoch als 15%-ige radikalische Emulsionspolymerisation. Die Initiatorlösung wird doppelt so konzentriert eingesetzt (gleiche Menge Initiatorlösung bei halber Menge Monomere), um die Reaktionsgeschwindigkeit ohne grosse Einbussen aufrecht zu erhalten.

### Eduktlösung:

| *Einwaage* | *Produktbezeichnung* |
|---|---|
| 45.0 g | MPS-Na 2403 Monomer: 2-Acrylamido-2-methylpropan-natriumsulfonat |
| 22.5 g | mPEG-350-Methacrylat, >95%, Evonik Industries |
| 7.5 g | 2-Hydroxyethyl-methacrylat (HEMA), 97%, stabilisiert, Acros Organics |
| 15.0 g | 2-Ethylhexyl-acrylat, 99%, stabilisiert, Acros Organics |
| 7.5 g | Cylink NBMA Monomer: N-(Butoxymethyl)-acrylamid, 81%, Cytec Industries |
| 4.5 g | Disponil AFX 1080, 80%, Cognis |
| 3.0 g | Natriumdodecylsulfat (SDS), p.A., Serva Feinbiochemika, M = 288.38 g/mol |
| 5.0 g | Natriumdodecylbenzolsulfonat-Lösung, 30% in Wasser, M = 348.48 g/mol |
| 25.0 g | Dipropylenglykol, d = 1.023 g/ml, bp = 90-95 °C/1 mmHg |
| 222.8 g | Wasser deionisiert |

### Initiatorlösung wie in P-017. Vorhomogenisation, Apparatur wie Scale-up P-042 Nr. 1.

Synthese: 455 g homogenisierte Eduktlösung wird in der Apparatur vorgelegt und mittels Heizpilz auf 70 °C erhitzt Bei Erreichen von etwa 60 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 25 g Initiatorlösung durch das Septum zudosiert. Es ist nur eine leichte Exothermie feststellbar. Die Reaktionstemperatur wird bei 70 °C konstant gehalten. Nach 30 min werden wiederum mittels Spritze die restlichen 27 g Initiatorlösung zugegeben (keine Nachexothermie feststellbar). Es entsteht eine leicht rötlich schimmernde Emulsion, auf welcher während der ganzen Reaktion eine etwa 1-2 cm dicke Schaumschicht liegt Quellverhalten: Etwas getrocknetes Polymer wird mit Wasser übergossen, wodurch das Polymer innert Minuten um ein Vielfaches anquellt und sich ablöst. Die Permanenz scheint schlecht zu sein.

Zusammenfassung: Die 15%-ige radikalische Emulsionspolymerisation als 0.5 kg Scale-up verlief erfolgreich, obschon der Umsatz nach einer Reaktionszeit von 4 h nur 91.7% beträgt. Da sich am Reaktionskolben kein Koagulum bildete, liegt dies wahrscheinlich mitunter am Verlust von Eduktlösung beim Homogenisieren (Totvolumen). Die Bestimmung der Partikelgrössenverteilung mittels Photonenkorrelationsspektroskopie (PCS) ergibt eine multidisperse Emulsionstropfenverteilung mit hydrodynamischen Tropfendurchmessern von 100 nm (3.9%-Vol.), 1094 nm (92.2%-Vol.) und 4602 nm (3.9%-Vol.). Um die anschliessenden Appreturversuche zu vereinfachen, wurde die Reaktionslösung mit Wasser auf eine Polymer-Konzentration von 12.0% verdünnt. Es konnte ein innert kürzester Zeit sehr gut quellbares Polymer synthetisiert werden.

### Scale-up P-044 Nr. 1

Scale-up von P-044 auf 0.5 kg zur Herstellung von Poly(2-Acrylamido-2-methylpropan-natriumsulfonat-stat-2-Ethylhexyl-acrylat-stat-mPEG-350-Methacrylat-stat-N-(Butoxymethyl)-acrylamid).

Da das bisherige Emulsionssystem nicht besonders stabil ist, wurde nach Alternativen gesucht. Bei der Eduktlösung handelt es sich um ein vielseitiges Monomersystem mit hydrophilen und hydrophoben als auch um nichtionische sowie ionische Monomere, wodurch bisher im ungerührten System nach etwa 10 Minuten eine Phasentrennung auftrat. Dies kann bei einer Polymerisation im Grossreaktor Probleme verursachen, da die obere, schwimmende Monomerphase als Massepolymerisation durchgehen könnte. Daher wurde zusätzlich mit folgenden, nichtionischen Emulgatoren Vorversuche durchgeführt: Marlipal 013/30, Marlipal 013/50, Mulsifan RT110, Hostapur OS Liquid, Marlosol OL7 und Marlowet R 40. Mit 3-5% Marlowet R 40 entstand die stabilste Emulsion. Es zeigte sich, dass der Zusatz von Natriumdodecylsulfat (SDS) und Natriumdodecylbenzolsulfonat zur erneuten Phasentrennung führt. Daher wird nur Marlowet R 40 als Alleinemulgator eingesetzt. Dieser bildet aber nur eine stabile Emulsion, wenn zuerst die Monomere in der Apparatur vorgelegt werden, Marlowet R 40 zugegeben und erst am Schluss Wasser zugesetzt wird. Wird hingegen zuerst das Wasser und dann der Emulgator zugegeben, entsteht eine instabile Emulsion.

Die Polymerisation basiert auf P-042 Scale-up Nr. 2, wird jedoch mit nur 1 % V-50 als Initiator (statt 2% wie bei Scale-up P-042 Nr. 2) bei einer Reaktionstemperatur von 70 °C durchgeführt. Das Monomer AMPS-Na 2405 wird anstelle von AMPS-Na 2403 für die Produktion eingesetzt. AMPS-Na 2405 ist sowohl für Lebensmittel als auch bei Hautapplikation zugelassen, da der Acrylamid- und Acrolynitrilgehalt unter 0.05% liegt. Ausserdem wird die Polymerisation ohne Vorhomogenisation durchgeführt und als Basis wird die Rezeptur von P-044 (ohne HEMA) herangezogen, da diese bei den Appreturversuchen die besten Ergebnisse zeigte. Es soll untersucht werden, ob sich P-044 als 0.5 kg Scale-up mittels Emulsionspolymerisation problemlos herstellen lässt.

### Eduktlösung:

| *Einwaage* | *Produktbezeichnung* |
|---|---|
| 45.0 g | AMPS-Na 2405 Monomer: 2-Acrylamido-2-methylpropan-natriumsulfonat (enthält ca. 50% Wasser), Lubrizol, M = 229.23 q/mol, d = 1.21 g/ml |
| 30.0 g | mPEG-350-Methacrylat, >95%, Evonik Industries |
| 15.0 g | 2-Ethylhexyl-acrylat, 99%, stabilisiert, Acros Organics |
| 7.5 g | Cylink NBMA Monomer: N-(Butoxymethyl)-acrylamid 81%, Cytec Industries |
| 2.3 g | Marlowet R 40: PEG-40-Rizinusöl, 83%, Sasol, M = 2695 g/mol, d = 1.06 g/ml, mp = 17 °C |
| 15.0 g | Dipropylenglykol, d = 1.023 q/ml, bp = 9 0-9 5 °C/1 mmHg |
| 375.2 g | Wasser deionisiert |

### Initiatorlösung wie in P-017. Vorhomogenisation, Apparatur wie Scale-up P-042 Nr. 1.

Synthese: In die Apparatur werden 97.5 g Monomere sowie 2.3 g Marlowet R 40 und 15.0 g Dipropylenglykol vorgelegt Dann werden unter intensivem Rühren langsam 375.2 g Wasser hinzugetropft, wodurch das System emulgiert (weisse Emulsion). Unter Rühren wird die Monomeremulsion mittels Heizpilz auf 70 °C erhitzt. Bei Erreichen von etwa 60 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels einer Spritze 5 g Initiatorlösung durch das Septum zudosiert. Es ist nur eine leichte Exothermie feststellbar. Die Reaktionstemperatur wird bei 70 °C konstant gehalten. Nach 30 min werden wiederum mittels Spritze die restlichen 5 g Initiatorlösung zugegeben (keine Nachexothermie feststellbar). Es entsteht eine weisse, viskose Emulsion. Quellverhalten: Etwas getrocknetes Polymer wird mit Wasser übergossen, wodurch das Polymer innert Minuten um ein Vielfaches anquellt und sich ablöst. Die Permanenz scheint schlecht zu sein.

Zusammenfassung: Die 15%-ige radikalische Emulsionspolymerisation von P-044 als 0.5 kg Scale-up verlief erfolgreich. Der Umsatz mit 1 % V-50 als Initiator liegt nach einer Reaktionszeit von 4 h bei 98.0%. Trotz neuem Emulgatorsystem und ohne Vorhomogenisation bildete sich wiederum kein Koagulum am Reaktionskolben. Die Bestimmung der Partikelgrössenverteilung mittels Photonenkorrelationsspektroskopie (PCS) ergibt eine monodisperse Emulsionstropfengrösse mit einem hydrodynamischen Durchmesser von 1265 nm. Es konnte ein innert kürzester Zeit sehr gut quellbares Polymer synthetisiert werden.

### Scale-up P-067

Basierend auf dem Scale-up P-044, 40% mPEG-350-Methacrylat und 20% 2-Ethylhexyl-acrylat werden die Monomeranteile umgekehrt eingesetzt. Das Polymer P-067 besitzt somit folgende Grund-Zusammensetzung: 30%-Gew. AMPS-Natriumsalz, 20%-Gew. mPEG-350-Methacrylat, 40%-Gew. 2-Ethylhexyl-acrylat, 10%-Gew. NBMA-Vernetzer.

Bei Desorptionsversuchen kann damit die Desorptionsrate der Wirkstoffe in Abhängigkeit des amphiphilen Charakters der polymeren Donorschicht untersucht werden. Werden die Wirkstoffe zu schnell an die Haut abgeben, muss die Donorschicht lipophiler gestaltet werden, und umgekehrt hydrophiler bei zu niedriger Wirkstoff-Desorption.

### Eduktlösung:

| *Ein waaqe* | *Produktbezeichnung* |
|---|---|
| 45.0 g | AMPS-Na 2405 Monomer: 2-Acrylamido-2-methylpropan-natriumsulfonat |
| 15.0 g | mPEG-350-Methacrylat, >95%, Evonik Industries |
| 30.0 g | 2-Ethylhexyl-acrylat, 99%, stabilisiert, Acros Organics |
| 7.5 g | Cylink NBMA Monomer: N-(Butoxymethyl)-acrylamid 81%, Cytec Industries |
| 2.3 g | Marlowet R 40: PEG-40-Rizinusöl, 83%, Sasol |
| 15.0 g | Dipropylengykol, d = 1.023 g/ml, bp = 90-95 °C/1 mmHg |
| 375.2 g | Wasser deionisiert |

Initiatorlösung, Vorhomogenisation, Apparatur, Synthese wie Scale-up P-044 Nr. 1. Quellverhalten: Etwas getrocknetes Polymer wird mit Wasser übergossen, wodurch das Polymer innert Minuten um ein Vielfaches anquellt und sich nur leicht ablöst. Die Permanenz scheint gut zu sein.

### Scale-up P-044 Nr. 2

Scale-up von P-044 auf 4.5 kg zur Herstellung von Poly(2-Acrylamido-2-methylpropan-natriumsulfonat-stat-2-Ethylhexyl-acrylat-stat-mPEG350-Methacrylat-stat-N-(Butoxymethyl)-acrylamid).

Die Polymerisation wird in einem 6 I-Vierhalsrundkolben mit V-50 als Initiator bei einer Reaktionstemperatur von 70 °C durchgeführt. Da bei der Emulsionspolymerisation von Scale-up P-067 ohne Vorhomogenisation visuell grobe Partikel in der verdünnten Probe festgestellt werden konnten, wird diese Polymerisation vorhomogenisiert. Um Zeit zu sparen wird die Eduktlösung jedoch nur drei Mal bei 600 bar durch den Hochdruck-Homogenisierer gefördert. Es soll untersucht werden, ob sich P-044 als 4.5 kg Scale-up mittels Emulsionspolymerisation problemlos herstellen lässt.

### Eduktlösung:

| *Einwaage* | *Produktbezeichnung* |
|---|---|
| 405.0 g | AMPS-Na 2405 Monomer_2-Acrylamido-2-methylpropan-natriumsulfonat |
| 270.0 g | PEG-350-Methacrylat, >95%, Evonik Industries |
| 135.0 g | 2-Ethylhexyl-acrylat, 99%, stabilisiert, Acros Organics |
| 67.5 g | Cylink NBMA Monomer: N-(Butoxymethyl)-acrylamid 81%, Cytec Industries |
| 20.5 g | Marlowet R 40: PEG40-Rizinusöl, 83%, Sasol |
| 135.0 g | Dipropylenglykol, d = 1.023 g/ml, bp = 90-95 °C/1 mmHg |
| 3367.0 g | Wasser deionisiert |

Initiatorlösung wie in P-017. Apparatur: 6 I-Vierhalsrundkolben mit Rührwerk, Rückflusskühler, 250 ml-Zulauftrichter mit Druckausgleich und Temperaturfühler. Ein Abgang mit Hahn auf dem Rückflusskühler dient dem Evakuieren und Belüften mit Stickstoff. Vorhomogenisation: Die Eduktlösung (pH = 6.1) wird drei Mal bei 600 bar durch den Hochdruck-Homogenisierer gefördert, wodurch eine Emulsion entsteht. 1 g homogenisierte Eduktlösung wird mit 19 g Wasser verdünnt (1:20). Viskosität: 0.98 mPa · s. Partikelgrössenverteilung (PCS-Messung): Peak 1: d(H) = 1224 nm - 77.0%-Vol.; Peak 2: d(H) = 95 nm - 23.0%-Vol.; Peak 3: d(H) = Kein Signal vorhanden.

Synthese: 4400 g homogenisierte Eduktlösung wird in der Apparatur vorgelegt und mittels Heizpilz auf 70 °C erhitzt. Anschliessend werden 100 g Initiatorlösung in den Zulauftrichter auf der Apparatur eingefüllt. Bei Erreichen von etwa 60 °C wird jeweils drei Mal evakuiert und mit Stickstoff belüftet, um den Luftsauerstoff (Inhibitor) zu entfernen. Damit während der gesamten Polymerisation ein Druckausgleich gewährleistet ist, wird die Apparatur mit Stickstoff hinterleitet. Nun werden unter gutem Rühren mittels Zulauftrichter 50 g Initiatorlösung zudosiert. Es ist eine Exothermie feststellbar, wodurch sich die Reaktionslösung auf 80 °C erhitzt. Daher wird der Heizpilz vorübergehend abgesenkt, um ein besseres Abkühlen zu gewährleisten. Die Reaktionstemperatur wird bei 70 °C konstant gehalten. Nach 30 min werden die restlichen 50 g Initiatorlösung zugegeben (keine Nachexothermie feststellbar). Es entsteht eine weisse, viskose Emulsion, auf welcher eine Schaumschicht liegt. Koagulum ist keines entstanden. Quellverhalten: Etwas getrocknetes Polymer wird mit Wasser übergossen, wodurch das Polymer innert Minuten um ein Vielfaches anquellt und sich ablöst. Die Permanenz scheint schlecht zu sein.

Zusammenfassung: Die 15%-ige radikalische Emulsionspolymerisation von P-044 als 4.5 kg Scale-up verlief erfolgreich. Der Umsatz liegt nach einer Reaktionszeit von 4 h bei 97.6%. Am Reaktionskolben bildete sich wiederum kein Koagulum. Die Bestimmung der Partikelgrössenverteilung mittels Photonenkorrelationsspektroskopie (PCS) ergibt eine multidisperse Emulsionstropfenverteilung mit hydrodynamischen Tropfendurchmessern von 1126 nm (92.3%-Vol.), 202 nm (4.9%-Vol.) und 5472 nm (2.8%-Vol.), auch wenn dieses Messresultat ungenau ist. Dies könnte daran liegen, dass die Eduktlösung nur drei Mal anstatt wie bisher fünf Mal bei 600 bar durch den Hochdruck-Homogenisierer gefördert wurde. Es konnte ein innert kürzester Zeit sehr gut quellbares Polymer synthetisiert werden.

Die in diesem Abschnitt aufgeführten Beispiele für erfindungsgemässe Polymerverbindungen bilden in Verbindung mit einem geeigneten Binder auf Textilien waschpermanente Ausrüstungen. Entsprechende Verfahren sind im nächsten Abschnitt gezeigt. Es wurde gefunden, dass P-044 und P-067 textile Ausrüstungen bilden können, welche 100 Wäschen bei 60 °C während 50-55 min ohne wesentliche Beeinträchtigung der Donorschicht überstehen.

### B. Ausrüstung von textilen Flächen

Die Permanenz der Ausrüstung von Geweben mit den erfindungsgemässen Polymeren und die effektiv zur Verfügung stehende Oberflächenladurig der Ausrüstungsschichten wurden untersucht.

### Versuchsreihe 1

**Ausrüstung der Gewebe:** Für die Appreturversuche wurde jeweils ein Gewebe aus 100% Polyamid (PA) verwendet (Charmeuse vorfixiert, Flächengewicht 135 g/m²; Fussenegger Textilveredelung GmbH, AT-6850 Dornbirn; Trikothersteller: Huber Tricot GmbH, AT6841 Mäder; Prod. Nr. 11065). Die Flottenansätze bestanden aus 200 g Polymer/kg wässriger Flotte. Dazu kamen optional 100 g Vernetzungskatalysator-Stammlösung/kg Flotte. Die genannte Katalysator-Stammlösung bestand aus 50 g MgCl₂ x 6 H₂O/kg Stammlösung und 20 g L-(+)-Weinsäure/kg Stammlösung. Die Ausrüstung erfolgte durch Foulardieren des PA-Gewebes mit der Polymer-Dispersion-Flotte (Walzendruck 15 bar, Warengeschwindigkeit 2 m/min), anschliessendem Trocknen (Umlufttemperatur 100 °C, 3 min), und Kondensieren/Fixieren der Ausrüstung (Umlufttemperatur 150 °C, 5 min).

**Permanenz der Ausrüstung:** Die Permanenz der Ausrüstung wurde durch Soxleth-Extraktion bestimmt. Dazu wurden jeweils zwei Proben ausgerüsteten Textilgewebes à 12.5 g während 3 h mit Methanol extrahiert. Die Resultate für die verschiedenen untersuchten Ausrüstungsvarianten ergaben:

**Tabelle: Permanenz der Ausrüstung**

| *Ausrüstung* | *Polymer* | *pH der Polymerdispersion* | *Vernetzungskatalysator verwendet* | *Auflagemenge der Ausrüstung (%-Gew. Gewebeprobe)* | *Verbleibende Auflagemenge der Ausrüstung nach Extraktion* |
|---|---|---|---|---|---|
| A-001 | P-002 | 3.3 | Nein | 3.35% | 89% |
| A-002 | P-002 | 3.3 | Ja | 3.46% | 90% |
| A-003 | P-004 | 8.5 | Nein | 3.73% | 68% |
| A-004 | P-004 | 8.5 | Ja | 3.76% | 77% |
| A-007 | P-005 | ca. 7 | Ja | 4.44% | 7% |
| A-008 | P-008 | ca. 7 | Ja | 1.00% | 0% |
| A-009 | P-009 | ca. 7 | Ja | 1.76% | 24% |

### Oberflächenladung der Ausrüstungsschicht

Die Oberflächenladung der ausgerüsteten Gewebemuster wird mittels Ladungstitration mit einem Charge Analysing System (CAS) bestimmt (AFG Analytic GmbH, Leipzig, DE; Modell Nr. B390/B422/B490). Probenvorbereitung: 0.5 g Stoffprobe werden mit einer 20 mm-Mahlkugel aus Wolframcarbid in einem verschraubbaren 25 ml-Mahlbecher aus gehärtetem Spezialstahl bei -196 °C (flüssiger Stickstoff) während 2x2 min bei 30 Hz vermahlen (Kugelmühle Retsch MM400).

Zur Bestimmung der Oberflächenladung werden in die PTFE-Messzelle des CAS 4.8 g Wasser vorgelegt und vom zermahlenen Stoffmuster 0.2 g Probe zugegeben und verrührt. Während 2 min wird die PTFE-Messzelle im Ultraschallbad suspendiert. Anschliessend wird der Messkolben in die Messzelle eingefügt und danach die Ladungstitration mit kationischer Polyelektrolytlösung, 0.001 N PolyDADMAC (Poly(Diallyldimethyl-ammoniumchlorid)) durchgeführt.

**Tabelle: Resultate Ladungstitration**

| *Ausrüstung Nr.* | *Polymer, Katalysator, Lagerung* | *Verbrauch Titrationslösung [ml]* | *Oberflächenladung [µmol neg. Ladung*/*g Ware]****)* | | |
|---|---|---|---|---|---|
| | | | *total* | *aufgrund Ausrüstungsschicht *)* | *theoretisch* |
| A-001 | P-002 ohne Kat. | 0.30 | 1.50 | 0.87 | 86.02 |
| A-001 | P-002 ohne Kat., 4 Wochen gelagert | 0.59 | 2.95 | 2.32 | 86.02 |
| A-002 | P-002 mit Kat. | 0.71 | 3.55 | 2.92 | 88.93 |
| A-002 | P-002 mit Kat., 4 Wochen gelagert | 0.65 | 3.25 | 2.62 | 88.93 |
| A-003 | P-004 ohne Kat., 4 Wochen gelagert | 6.95 | 34.75 | 34.12 | 31.03 |
| A-004 | P-004 mit Kat. | 0.87 | 4.35 | 3.72 | 33.06 |
| A-004 | P-004 mit Kat., 1 Woche gelagert | 5.13 | 25.65 | 25.02 | 33.06 |
| A-004 | P-004 mit Kat., 4 Wochen gelagert | 6.97 | 34.85 | 34.22 | 33.06 |
| A-007 | P-005 mit Kat. | 14.81 | 74.05 | 73.42 | 75.43 |
| A-008 | P-008 mit Kat. | 1.36 | 6.80 | 6.17 | 13.06 |
| A-009 | P-009 mit Kat. | 2.8 | 14.00 | 13.37 | 23.54 |

| | | | | | |
|---|---|---|---|---|---|
| *) Für das unbehandelte Gewebe wurde eine Oberflächenladung von 0.63 µmol/g Ware bestimmt. **) Die Oberflächenladung in mol kann durch Multiplikation mit der Faradaykonstante C = 96485.309 C/mol in Coulomb umgewandelt werden | | | | | |

**A-001, A-002:** Die Permanenz der Ausrüstungsschichten mit P-002 ist gut. Jedoch ist die zur Verfügung stehende negative Oberflächenladung ungenügend. Die Ausrüstungsschicht nimmt keine Feuchtigkeit auf, so dass sich die effektiv zur Verfügung stehende Oberflächenladung nicht verbessert.

**A-003, A-004:** Unmittelbar nach dem Ausrüsten der Stoffmuster liegt die analytisch erfassbare Oberflächenladung nur bei 3.7 µmol/g Ware, von theoretisch möglichen 33 µmol/g Ware. Jedoch steigt diese bereits nach einer Lagerzeit von einer Woche auf 25 µmol/g Ware an und ist nach vier Wochen mit 34 µmol/g Ware im Wesentlichen komplett zugänglich. Gegenüber A-001, A-002 fördern die negativen Sulfonatgruppen des Polymers P-004 die Quellbarkeit der Ausrüstungsschicht. Diese sollte jedoch schneller quellbar sein. Die Permanenz der Ausrüstungsschicht ist ausreichend, wobei wie zu Erwarten die Verwendung des Katalysators für die säurekatalysierte Fixierung die Permanenz verbessert.

**A-007:** Das Polymer gewährleistet aufgrund seines schnellen Quellungsvermögens eine sofortige Zugänglichkeit der Ladungsträger nach der Ausrüstung. Hingegen ist die Permanenz der Ausrüstung unzureichend, was gegebenenfalls mit den voluminösen mPEG-1000-Methacrylat-Monomeren zusammenhängen kann. Hier kann durch eine geeignete Optimierung in Bezug auf den Vernetzer und die Fixierungsparameter die Permanenz verbessert werden.

**A-008, A-009:** Die Oberflächenladungen sind nach der Ausrüstung des textilen Produkts noch nicht vollständig zugänglich, jedoch ist bei A-009 die Permanenz gegenüber A-007 verbessert.

Durch Optimierung der Anteile an hydrophilen Monomeren kann ein Kompromiss gefunden werden zwischen der Permanenz einer erfindungsgemässen Ausrüstungsschicht, und der Geschwindigkeit, mit welcher die Ausrüstungsschicht nach der Ausrüstung Wasser aufnehmen kann, so dass die Oberflächenladungen zugänglich werden.

### Versuchsreihe 2

Appreturlösungen mit verschiedenen Polymerverbindungen der Screeningreihe P-018 bis P-053 wurden für die Ausrüstung von Gewebe getestet. Die PA-Gewebe werden dazu im Imprägnieransatz getaucht, im Foulard abgepresst und im Labortrocker getrocknet und kondensiert. Zur Vernetzung wird p-Toluolsulfonsäure als Katalysator eingesetzt. Anschliessend wird die Waschpermanenz der Polymere mittels Ladungstitration (CAS) untersucht.

**Gewebe:** Für die Appreturversuche wurde jeweils ein Gewebe aus 100% Polyamid (PA) verwendet (Charmeuse vorfixiert, Flächengewicht 135 g/m²; Fussenegger Textilveredelung GmbH, AT-6850 Dornbirn; Trikothersteller: Huber Tricot GmbH, AT-6841 Mäder; Prod. Nr. 11065).

**Ausrüstungsflotte:** Die wässrige Flotte bestand jeweils aus 13.2 g Polymerlösung und 10.8 g Katalysatorlösung. Die Polymerlösung entspricht den Reaktionslösungen der Polymerisationsversuche, verdünnt mit Wasser auf eine Polymer-Konzentration von 12.0% (vgl. Abschnitt A). Die Katalysatorlösung besteht aus 2.44 g p-Toluolsulfonsäure Monohydrat (98.5%, Fluka, M = 190.22 g/mol, mp = 103-105 °C) auf 997.56 g Wasser.

**Ausrüstung:** Die Reaktionslösung (verdünnt auf 12.0% Polymeranteil) wird in einer Glasschale unter Rühren mit Katalysator-Lösung verdünnt. In der resultierenden Flotte werden pro Imprägnieransatz jeweils sechs Stoffmuster (ausgestanzt auf 11x11 cm) in der jeweiligen Imprägnierlösung während einigen Minuten von Hand getaucht und im Foulard ein Mal abgepresst (Walzendruck 15 bar, Warengeschwindigkeit 2 m/min). Die Flottenaufnahme bezogen auf das Trockengewicht lag zwischen 57% und 64%-Gew., was einer Auflagemenge von durchschnittlich 4%-Gew. Polymer entspricht. Anschliessend folgte das Trocknen (Umlufttemperatur 100 °C, 3 min), und Kondensieren/Fixieren der Ausrüstung (Umlufttemperatur 150 °C, 5 min).

**Permanenz der Ausrüstung:** Zur Überprüfung der Waschmaschinenpermanenz der Ausrüstung wurde jeweils ein appretiertes PA-Stoffmuster halbiert und dieses einer Maschinenwäsche (MW) unterzogen. Eine handelsübliche europäische Haushalts-Waschmaschine wird mit einem Wäschesack, der die appretierten Stoffmuster und eine Polyester- und Polyamidbeiladung (Gesamtmenge 2 kg) enthält, befüllt. Für den Hauptspülgang werden ca. 15 g Waschpulver (Perwoll Wolle & Feines) verwendet. Nach 1 Maschinenwäsche bei 60 °C (für Unterwäsche) während 50-55 min werden die gewaschenen Stoffmuster bei Raumtemperatur an der Luft getrocknet und während mindestens 24 h im Normklima (20 ± 2 °C, 65 ± 5% relative Luftfeuchtigkeit) gelagert.

**Oberflächenladung der Ausrüstungsschicht:** Probenvorbereitung und Ladungstitration mit Charge Analysing System (CAS) wie in Versuchsreihe 1. Aufgrund des Verbrauchs an Titrationslösung und nach Abzug des Blindwerts für unbehandeltes Gewebe ergibt sich die verbliebene Auflagemenge

**Tabelle: Relative Ladungspermanenz nach 1 Maschinenwäsche**

| *Ausrüstung* | *Polymer* | *Ausrüstungsauflage, berechnet aus Flottengehalt vor Trocknen*/*%-Gew.* | *Relative Ladungspermanenz nach 1 MW, normiert auf 4%-Gew. Auflage vor MW* |
|---|---|---|---|
| *Screeningreihe 2.1 - Polymer mit 0% Vernetzer* | | | |
| A-018 | P-018 | 4.19 | 2 |
| A-019 | P-019 | 4.25 | 3 |
| A-020 | P-020 | 4.19 | 2 |
| A-021 | P-021 | 4.09 | 2 |
| A-022 | P-022 | 4.05 | 1 |
| A-02 3 | P-023 | 4.18 | 1 |
| A-024 | P-024 | 3.78 | 1 |
| A-025 | P-025 | 4.07 | 2 |
| A-026 | P-026 | 3.85 | 1 |

| *Screeningreihe 2.2 - Polymer mit 5% Vernetzer* | | | |
|---|---|---|---|
| A-027 | P-027 | 3.95 | 35 |
| A-028 | P-028 | 3.92 | 35 |
| A-029 | P-029 | 4.07 | 29 |
| A-030 | P-030 | 4.09 | 32 |
| A-031 | P-031 | 3.94 | 16 |
| A-032 | P-032 | 3.91 | 19 |
| A-033 | P-033 | 3.89 | 23 |
| A-034 | P-034 | 3.73 | 14 |
| A-035 | P-035 | 3.79 | 15 |

| *Screeningreihe 2.3 - Polymer mit 10% Vernetzer* | | | |
|---|---|---|---|
| A-036 | P-036 | 4.18 | 21 |
| A-037 | P-037 | 4.10 | 38 |
| A-038 | P-038 | 4.02 | 37 |
| A-039 | P-039 | 3.94 | 36 |
| A-040 | P-040 | 3.91 | 36 |
| A-041 | P-041 | 3.85 | 35 |
| A-042 | P-042 | 3.84 | 27 |
| A-043 | P-043 | 3.85 | 27 |
| A-044 | P-044 | 3.85 | 29 |

| *Screeningreihe 2.4 - Polymer mit 15% Vernetzer* | | | |
|---|---|---|---|
| A-045 | P-045 | 4.09 | 41 |
| A-046 | P-046 | 4.13 | 42 |
| A-047 | P-047 | 4.12 | 32 |
| A-048 | P-048 | 3.87 | 36 |
| A-049 | P-049 | 3.82 | 39 |
| A-050 | P-050 | 3.83 | 28 |
| A-051 | P-051 | 3.85 | 35 |
| A-052 | P-052 | 3.85 | 35 |
| A-053 | P-053 | 3.94 | 14 |

### Versuchsreihe 3

Mit dem gleichen Versuchsablauf wie in Versuchsreihe 2 wurden verschiedene Appreturlösungen auf ihre Waschpermanenz getestet, unter Verwendung der Polymerlösungen der Vernetzerscreenings P-054 bis P-059.

**Tabelle: Relative Ladungspermanenz nach 1 Maschinenwäsche**

| *Ausrüstung* | *Polymer* | *Ausrüstungsauflage, berechnet aus Flottengehalt vor Trocknen*/*%-Gew.* | *Relative Ladungspermanenz nach 1 MW, normiert auf 4%-Gew. Auflage vor MW* |
|---|---|---|---|
| A-054 | P-054 (1% NMBA) | 3.84 | 7 |
| A-05 | P-055 (eq. NMA) | 4.18 | 13 |
| A-056 | P-056 (eq. GMA) | 4.05 | 8 |
| A-057 | P-057 (eq. p-EMKO-...) | 3.96 | 18 |
| A-058* | P-058 (eq. EMKO-Alkenyl...) | 3.98, 4.07 | 7,9 |
| A-059 | P-059 (NBMA, ETMA) | 3.62 | 19 |

| | | | |
|---|---|---|---|
| * mehrere Versuchsergebnisse | | | |

**Zusammenfassung:** Die Polyelektrolytverbräuche der Screeningreihe A-054 bis A-059 mit untschiedlichen Vernetzern (Vernetzergehalte jeweils auf eine äquimolare Einsatzmenge von 1 % NBMA bezogen) weisen im ungewaschenen Zustand unterschiedliche Ladungszugänglichkeiten auf. Die PolyDADMAC-Verbräuche der gewaschenen Stoffmuster (1 MW bei 60 °C während 50-55 min) liegen allerdings bei sämtlichen Schichten im Bereich von 1 ml 0.001 N PolyDADMAC. Durch den Einsatz der unterschiedlichen Vernetzer, als auch durch den Ersatz von mPEG-35GMethacrylat durch Ethyltriglykol-methacrylat (A-059), kann keine Verbesserung der Waschmaschinenpermanenz erzielt werden.

### Versuchsreihe 4

Mit dem gleichen Versuchsablauf wie in Versuchsreihe 2 wurden verschiedene Appreturlösungen auf ihre Waschpermanenz getestet, unter Verwendung der Polymerlösungen der Gegenionenscreenings P-060 bis P-064. Getestet wird die Permanenz nach 1 und 5 Maschinenwäschen.

**Tabelle: Relative Ladungspermanenz nach 1 bzw. 5 Maschinenwäschen**

| *Ausrüstung* | *Polymer* | *Ausrüstungsauflage, berechnet aus Flottengehalt vor Trocknen*/*%-Gew.* | *Relative Ladungspermanenz, normiert auf 4%-Gew. Auflage vor MW* | |
|---|---|---|---|---|
| | | | *1 MW* | *5 MW* |
| A-060 | P-060 (Lithium) | 4.22, 4.13 | 21 | 14 |
| A-061 | P-061 (Ammonium) | 4.15,3.97 | 99 | 91 |
| A-062 | P-062 (Triethylammonium) | 3.97.4.11 | 56 | 86 |
| A-063 | P-063 (1-Methylimidazolium) | 4.00, 4.04 | 51 | 60 |
| A-064 | P-064 (4-Methylmorpholinium) | 3.91, 4.07 | 73 | 136 |

**Zusammenfassung:** Die Polyelektrolytverbräuche der Screeningreihe A-060 bis A-064 mit unterschiedlichen AMPS-Gegenionen weisen mit Ausnahme von A-060 mit AMPS-Lithium, welches im ungewaschenen Zustand einen Verbrauch von etwa 8.5 ml 0.001 N PolyDADMAC hat, Verbräuche zwischen 2 ml und 4 ml 0.001 N PolyDADMAC auf. Aufgrund der geringeren Quellbarkeit der Donorschichten steigen die Werte der relativen Ladungspermanenz an. Die PolyDADMAC-Verbräuche der gewaschenen Stoffmuster (1 und 5 MW bei 60 °C während 50-55 min) liegen in demselben Bereich wie die Screeningreihen A-027 bis A-053, womit ebenfalls keine wesentliche Verbesserung betreffend den Ladungszugänglichkeiten festgestellt werden kann.

### Versuchsreihe 5

Mit dem gleichen Versuchsablauf wie in Versuchsreihe 2 wurden verschiedene Appreturlösungen auf ihre Waschpermanenz getestet, nach 1 und 5 Maschinenwäschen.

Versuchsweise wurden die erfindungsgemässen Ausrüstungsformulierungen zusammen mit einer Polyurethan-Dispersion (Lamethan NKS-AF) in Kombination mit einem alkylmodifizierten Melamin-/Formaldehyd-Derivat (Knittex CHN) als Bindersystem getestet. Zum einen kann dies die Schichtpermanenz der Donorschicht weiter steigern, und zum anderen kann so aufgezeigt werden, dass die erfindungsgemässen Polymerverbindungen mit anderen Polymeren mischbar und vernetzbar sind.

Ausrüstungsflotte A-065, A-066: wie in Versuchsreihe 2. Ausrüstungsflotte A-067: Die wässrige Flotte bestand aus 13.2 g Polymerlösung, 2.6 g Dicrylan PGS (7753) (60%, Erba AG, d = 1.10 g/ml), 0.2 g Knittex CHN (Erba AG, d = 1.18 g/ml) und 8.0 g Katalysatorlösung. Ausrüstungsflotte A-068: Die wässrige Flotte bestand aus 13.2 g Polymerlösung, 3.3 g Lamethan NKS-AF (48%, CHT R. Beitlich GmbH, d = 1.0 g/ml), 0.2 g Knittex CHN, 7.2 g Katalysatorlösung und zusätzlich 0.1 g p-Toluolsulfonsäure Monohydrat. Die Polymerlösung entspricht den Reaktionslösungen der Polymerisationsversuche, verdünnt mit Wasser auf eine Polymer-Konzentration von 12.0% (vgl. Abschnitt A). Katalysatorlösung wie in Versuchsreihe 2.

**Tabelle: Relative Ladungspermanenz nach 1 bzw. 5 Maschinenwäschen**

| *Ausrüstung* | *Polymer* | *Ausrüstungsauflage, berechnet aus Flottengehalt vor Trocknen*/*%-Gew.* | *Relative Ladungspermanenz nach 1 MW, normiert auf 4%-Gew. Auflage vor MW* | |
|---|---|---|---|---|
| | | | *1 MW* | 5 *MW* |
| A-065 | P-065 | 3.91 | 3 | |
| A-066 | P-066 | 3.93, 3.93 | 69 | 27 |
| A-067 | P-042, Dicrylan PGS, Knittex CHN | 4.24, 4.09 | 51 | 66 |
| A-068 | P-042, Lamethan NKS-AF, Knittex CHN | 3.97, 3.84 | 68 | 77 |

**Zusammenfassung:** Die Polyelektrolytverbräuche der Versuche A-065 bis A-068 liegen im ungewaschenen Zustand bei rund 5 ml 0.001 N PolyDADMAC. Der PolyDADMAC-Verbrauch des gewaschenen Stoffmusters A-065 (1 MW bei 60 °C während 50-55 min) mit N, N'-Methylen-bis-acrylamid (MBAm) als Vernetzer liegt mit etwa 0.4 ml 0.001 N PolyDADMAC im Bereich der Screeningreihe A-018 bis A-026. Wird der bisherige NBMA-Vernetzer allerdings mit MBAm kombiniert (A-066), steigt der PolyDADMAC-Verbrauch des gewaschenen Textils erstmalig auf knapp 3.5 ml 0.001 N PolyDADMAC an. Die relative Ladungspermanenz ist mit 69% ebenfalls gut. Allerdings sinkt diese nach 5 MW auf 27% ab. Durch Kombination des Polymers mit den Bindersystemen Dicrylan PGS/Knittex CHN (A-067) und Lamethan NKS-AF/Knittex CHN (A-068) kann die relative Ladungspermanenz auch nach fünfmaligem Waschen erhalten werden.

### Versuchsreihe 6

Mit dem gleichen Versuchsablauf wie in Versuchsreihe 5 wurden verschiedene Appreturlösungen auf ihre Waschpermanenz getestet, nach 1 und 5 Maschinenwäschen. Anstelle des formaldehydhaltigen Knittex CHN-Vernetzers wird ein oximblockierter Polyisocyanat-Vernetzer (Phobol XAN) eingesetzt, um Formaldehyd in der Donorschicht zu vermeiden.

Ausrüstungsflotte A-069: Die wässrige Flotte bestand aus 13.2 g Polymerlösung, 2.6 g Dicrylan PGS (7753) (60%, Erba AG, d = 1.10 g/ml), 0.2 g Phobol XAN (Erba AG, d = 1.03-1.08 g/ml) und 8.0 g Katalysatorlösung. Ausrüstungsflotte A-070: Die wässrige Flotte bestand aus 13.2 g Polymerlösung, 3.3 g Lamethan NKS-AF (48%, CHT R. Beitlich GmbH, d = 1.0 g/ml), 0.2 g Phobol XAN, 7.2 g Katalysatorlösung und zusätzlich 0.1 g p-Toluolsulfonsäure Monohydrat.

**Tabelle: Relative Ladungspermanenz nach 1 bzw. 5 Maschinenwäschen**

| *Ausrüstung* | *Polymer* | *Ausrüstungsauflage, berechnet aus Flottengehalt vor Trocknen*/*%-Gew.* | *Relative Ladungspermanenz nach 1 MW, normiert auf 4%-Gew. Auflage vor MW* | |
|---|---|---|---|---|
| | | | *1 MW* | *5 MW* |
| A-069 | P-042, Dicrylan PGS, Phobol XAN | 4.05, 3.93 | 37 | 43 |
| A-070 | P-042, Lamethan NKS-AF, Phobol XAN | 4.10,4.09 | 83 | 96 |

Zusammenfassung: Die Versuche A-069 und A-070 zeigen, dass der Knittex CHN-Vernetzer problemlos durch den formaldehydfreien Polyisocyanat-Vernetzer Phobol XAN ersetzt werden kann.

### Versuchsreihe 7

Mit dem gleichen Versuchsablauf wie in Versuchsreihe 5 wurden verschiedene Appreturlösungen auf ihre Waschpermanenz getestet. Um das Quellungsvermögen und damit die Ladungszugänglichkeit der Schicht zu erhöhen, werden die Anteile an zusätzlichem Polymer reduziert.

Ausrüstungsflotte A-071: Die wässrige Flotte bestand aus 79.2 g Polymerlösung, 7.8 g Dicrylan PGS (7753), 1.2 g Phobol XAN und 55.8 g Katalysatorlösung. A-072: Die wässrige Flotte bestand aus 79.2 g Polymerlösung, 9.9 g Lamethan NKS-AF, 1.2 g Phobol XAN, 53.3 g Katalysatorlösung, 0.4 g p-Toluolsulfonsäure Monohydrat. A-071: Die wässrige Flotte bestand aus 13.2 g Polymerlösung, 0.7 g Dicrylan PGS (7753), 0.2 g Phobol XAN und 9.9 g Katalysatorlösung. A-074: Die wässrige Flotte bestand aus 13.2 g Polymerlösung, 0.8 g Lamethan NKS-AF, 0.2 g Phobol XAN, 9.7 g Katalysatorlösung, 0.1 g p-Toluolsulfonsäure Monohydrat. A-075: Die wässrige Flotte bestand aus 13.2 g Polymerlösung, 0.2 g Phobol XAN, 10.6 g Katalysatorlösung.

**Tabelle: Relative Ladungspermanenz**

| *Ausrüstung* | *Anzahl MW* | *Ausrüstungsauflage, berechnet aus Flottengehalt vor Trocknen*/*%-Gew.* | *Relative Ladungspermanenz in %, normiert auf 4%-Gew. Auflage vor MW* |
|---|---|---|---|
| *Polymer: Scale-up P-042 Nr. 2, Dicrylan PGS (halbe Menge wie in A-069), Phobol XAN* | | | |
| A-071 | 1 | 4.14,3.93 | 55, 53 |
| | 5 | 4.09 | 51 |
| | 25 | 4.06 | 26 |
| | 50 | 3.85 | 18 |

| *Polymer: Scale-up P-042 Nr. 2, Lamethan NKS-AF (halbe Menge wie in A-070), Phobol XAN* | | | |
|---|---|---|---|
| A-072 | 1 | 4.07 | 71 |
| | 5 | 4.05 | 78 |
| | 25 | 3.97 | 64 |
| | 50 | 3.64 | 30 |

| *Polymer: Scale-up P-042 Nr. 2, Dicrylan PGS (ein Viertel von A-069), Phobol XAN* | | | |
|---|---|---|---|
| A-073 | 1 | 4.05 | 60 |
| | 5 | 4.05 | 61 |
| | 25 | 4.05 | 45 |
| | 50 | 4.13 | 11 |

| *Polymer: Scale-up P041 Nr. 2, Lamethan NKS-AF (ein Viertel von A-070), Phobol XAN* | | | |
|---|---|---|---|
| A-074 | 1 | 4.32 | 70 |
| | 5 | 4.11 | 62 |
| | 25 | 4.12 | 58 |
| | 50 | 3.80 | 22 |

| *Polymer: Scale-up P-042 Nr. 2, Phobol XAN* | | | |
|---|---|---|---|
| A-075 | 1 | 4.16 | 64 |
| | 5 | 4.20 | 63 |
| | 25 | 4.00 | 29 |
| 50 | | 4.17 | 15 |

**Zusammenfassung:** Um das Quellungsvermögen und damit die Ladungszugänglichkeit der Schicht zu erhöhen, wurde bei A-071 der Gehalt von Dicrylan PGS gegenüber A-069 halbiert. Die Polyelektrolytverbräuche steigen dadurch bei den ungewaschenen Stoffmustern auf 9 ml 0.001 N PolyDADMAC an. Die PolyDADMAC-Verbräuche der gewaschenen Stoffmuster (1 und 5 MW bei 60 °C während 50-55 min) können ebenfalls auf fast 5 ml 0.001 N PolyDADMAC gesteigert werden, woraus sich eine relative Ladungspermanenz von 53% ergibt. Erst nach 25 und 50 MW findet ein Einbruch der relativen Ladungspermanenz auf 26% bzw. 18% statt. Bei A-072 wurde der Gehalt von Lamethan NKS-AF ebenso und als Ergebnis eine zu A-071 vergleichbare Permanenz erhalten. Die Reduktion von Dicrylan PGS bei A-073 auf einen Viertel der ursprünglich eingesetzten Menge erbringt keine bedeutsame Verbesserung der bestimmten Schichtparameter. Bei A-074 wurde der Gehalt von Lamethan NKS-AF ebenso auf einen Viertel der ursprünglich eingesetzten Menge reduziert und als Ergebnis eine zu A-072 vergleichbare Permanenz erhalten. Interessanterweise wurde bei A-075 festgestellt, dass ohne jeglichen Einsatz von Dicrylan PGS oder Lamethan NKS-AF, jedoch zusammen mit dem im erfindungsgemässen Polymer eingebauten NBMA-Vernetzer und 1 % Phobol XAN eine ebenso vergleichbare Permanenz wie bei den Versuchen A-071 bis A-074 erzielt werden kann.

### Versuchsreihe 8

Mit dem gleichen Versuchsablauf wie in Versuchsreihe 5 wurden verschiedene Appreturlösungen auf ihre Waschpermanenz getestet.

Um die Zusammensetzung der erfindungsgemässen Ausrüstungsformulierung weiter zu optimieren, werden basierend auf A-072 die weiteren Versuche mit Lamethan NKS-AF anstelle von Dicrylan PGS durchgeführt. Obschon bei den bisherigen Versuchen Lamethan NKS-AF und Dicrylan PGS vergleichbare Waschpermanenzen aufwiesen, ist Lamethan NKS-AF günstiger im Anschaffungspreis, elastischer und zeigt einen besseren, d.h. angenehmeren Griff (Haptik). Dicrylan PGS hingegen ist für seine hohe Hydrolysebeständigkeit bekannt ist.

Ausrüstungsflotte: Die wässrige Flotte bestand aus jeweils 13.2 g Polymerlösung, 1.7 g Lamethan NKS-AF, 0.2 g Phobol XAN, 8.8 g Katalysatorlösung, 0.1 g p-Toluolsulfonsäure Monohydrat.

**Tabelle: Relative Ladungspermanenz**

| *Ausrüstung* | *Anzahl MIN* | *Ausrustungsauflage, berechnet aus Flottengehalt vor Trocknen*/*%.Gew.* | *Relative Ladungspermanenz in %, normiert auf 4%-Gew. Auflage vor MW* |
|---|---|---|---|
| *P-036, Lamethan NKS-AF, Phobol XAN* | | | |
| A-076 | 1 | 4.23 | 113 |
| | 5 | 4.44 | 125 |

| *P-044, Lamethan NKS-AF, Phobol XAN* | | | |
|---|---|---|---|
| A-077 | 1 | 4.07 | 75 |
| | 5 | 4.10 | 71 |
| | 25 | 4.01 | 86 |
| | 50 | 3.95 | 55 |

| *P-033, Lamethan NKS-AF, Phobol XAN* | | | |
|---|---|---|---|
| A-078 | 1 | 3.94 | 55 |
| | 5 | 3.89 | 61 |
| | 25 | 4.51 | 35 |
| | 50 | 3.93 | 13 |

| *P-024, Lamethan NKS-AF, Phobol XAN* | | | |
|---|---|---|---|
| A-079 | 1 | 4.03 | 27 |
| | 5 | 4.02 | 24 |

Basierend auf Versuch A-072 wurde bei A-076 auf mPEG-350-Methacrylat verzichtet, dafür aber 40% HEMA eingesetzt Bei A-077 hingegen wurden 40% mPEG-350-Methacrylat und 0% HEMA verwendet. Bei beiden Ausrüstungen wurden gute relative Ladungspermanenzen erzielt, allerdings ist dass Quellungsvermögen der Schicht bei A-076 mit einem Polyelektrolytverbrauch im Bereich von 2.5 ml 0.001 N PolyDADMAC stark reduziert. Bei A-077 hingegen kann der PolyDADMAC-Verbrauch der gewaschenen Stoffmuster (1, 5 und 25 MW bei 60 °C während 50-55 min) auf über 6 ml 0.001 N PolyDADMAC gesteigert werden. Und auch nach 50 MW liegt der Verbrauch noch immer bei 5 ml 0.001 N PolyDADMAC. A-077 ist damit die bisher beste Ausrüstungsformulierung, weist sie doch ein hohes Quellungsvermögen als auch eine hohe relative Ladungspermanenz auf. Um das Quellungsvermögen und damit die Ladungszugänglichkeit der Schicht zu erhöhen, wurde bei A-078, ebenfalls auf A-072 basierend, der Gehalt des im Polymer eingebauten NBMA-Vernetzers auf 5% reduziert, wodurch sich jedoch die Permanenz gegenüber A-072 besonders nach 25 und 50 MW reduzierte. In A-079 enthält das Polymer P-024 kein NBMA, aber wie P-042 10% HEMA, mit welchem das oximblockierte Polyisocyanat Phobol XAN ebenfalls vernetzen kann und die Permanenz somit gewährleistet bleiben sollte.

### Versuchsreihe 9

Mit dem gleichen Versuchsablauf wie in Versuchsreihe 5 wurden verschiedene Appreturlösungen auf ihre Waschpermanenz getestet. Die Formulierungen entsprechen A-072 mit verschiedenen Anteilen Phobol XAN.

Ausrüstungsflotte: Die wässrige Flotte bestand aus jeweils 13.2 g Polymerlösung, 1.7 g Lamethan NKS-AF, sowie 0.2 g (A-080), 0.1 g (A-081), 0.0 g (A-082), 0.7 g (A-083) Phobol XAN, 8.7 g (A-080), 8.9 g (A-081), 9.0 g (A-082), 8.3 g (A-083) Katalysatorlösung, 0.1 g p-Toluolsulfonsäure Monohydrat.

**Tabelle: Relative Ladungspermanenz**

| *Ausrüstung* | *Anzahl MW* | *Ausrüstungsauflage, berechnet aus Flottengehalt vor Trocknenl%-Gew,* | *Relative Ladungspermanenz in %, normiert auf 4%-Gew. Auflage vor MW* |
|---|---|---|---|
| *Scale-up P-042 Nr. 2, Lamethan NKS-AF, Phobol XAN 1.5%* | | | |
| A-080 | 1 | 4.05 | 71 |
| | 5 | 4.08 | 72 |
| | 25 | 3.89 | 81 |
| | 50 | 4.02 | 39 |

| *Scale-up P-042 Nr. 2, Lamethan NKS-AF, Phobol XAN 0.5%* | | | |
|---|---|---|---|
| A-081 | 1 | 4.21 | 99 |
| | 5 | 4.05 | 95 |
| | 25 | 4.09 | 93 |
| | 50 | 4.01 | 34 |

| *Scale-up P-042 Nr. 2, Lamethan NKS-AF, kein Phobol XAN* | | | |
|---|---|---|---|
| A-082 | 1 | 4.13 | 72 |
| | 5 | 4.06 | 69 |
| | 25 | 4.02 | 45 |
| | 50 | 3.83 | 21 |

| *P-026, Lamethan NKS-AF, Phobol XAN 3%* | | | |
|---|---|---|---|
| A-083 | 1 | 4.1 | 21 |
| | 5 | 4.20 | 16 |

Basierend auf Versuch A-072 wurde bei A-079 der Gehalt des im Polymer eingebauten NBMA-Vernetzers auf 0% reduziert. Wie erwartet verschlechterte sich dadurch die Permanenz. Bei A-080 und A-081, ebenfalls auf A-072 basierend, wurde der Vernetzergehalt von Phobol XAN auf 1.5 bzw. 0.5% Phobol XAN verändert. Als Ergebnis kann gegenüber A-072 kaum ein Unterschied festgestellt werden. Basierend auf Versuch A-072 wurde bei A-082 der Vernetzergehalt auf 0% Phobol XAN reduziert, wobei sich interessanterweise (vergleiche Versuch A-075) herausstellte, dass entweder auf Phobol XAN oder auf den Binder Dicrylan PGS bzw. Lamethan NKS-AF verzichtet werden kann. In A-083 wurde P-026 ohne HEMA und ohne NBMA-Vernetzer mit 3% Phobol XAN eingesetzt. Der Versuch zeigt keine Verbesserung der bisherigen Ergebnisse.

### Versuchsreihe 10

Produktionsversuch basierend auf A-077 und A-080 bis A-082 zur anionischen Gewebe-Imprägnierung mit einer 15%-igen Polymer-Dispersion von P-044 (Scale-up Nr. 2). Gemäss den Permanenzuntersuchungen von A-080 bis A-082 wird auf die Verwendung von Phobol XAN als oximblockierter Polyisocyanat-Vernetzer verzichtet, da dieser keinen massgeblichen Einfluss auf die Permanenz zeigte. Die Gewebe werden foulardiert und anschliessend direkt im Laborspannrahmen getrocknet und kondensiert. Zur Unterstützung der Vernetzung werden Magnesiumchlorid und Weinsäure als Katalysatoren eingesetzt. Anschliessend wird die Waschpermanenz der Polymere mittels Ladungstitration (CAS) untersucht.

**Gewebe:** PA: 100% PA wie in Versuchsreihe 1 und 2. PES: 100% PES, Christian Eschler AG, CH-9055 Bühler, Eyelet (Rundstrickware), 130-145 g/m², Farbe 100020 ausgewaschen. PES/PUE: 83% PES/17% PUE (Elastan), Christian Eschler AG, CH-9055 Bühler, Charmeuse (Kettwirkware), 130-140 g/m², Farbe 100202 continue ausgewaschen. CO: 100% CO, Gestrick, Greuter-Jersey AG, CH-8583 Sulgen, 167 g/m².

Katalysator-Stammlösung: 125 g Magnesiumchlorid x 6 H₂O, ≥98.0%, Fluka; 50 g L-(+)-Weinsäure ≥ 99.5%, Fluka; 2325 g Wasser (deionisiert).

**Gewebe-Imprägnieransatz:** 1320 g Scale-up P-044 Nr. 2 Reaktionslösung; 210 g Lamethan NKS-AF, CHT R. Beitlich GmbH, 1170 g Wasser (nicht deionisiert); 300 g Katalysator-Stammlösung. Der Katalysator wird erst unmittelbar vor dem Foulardieren zugegeben.

**Ausrüstung:** Nach dem Ansetzen wird die Appreturflotte gut durchgerührt und in den Tauchtrog am Foulard eingefüllt Anschliessend werden jeweils 6 Laufmeter unter Produktionsbedingungen foulardiert (4.5 bar Walzendruck, 4 m/min) und danach direkt im Laborspannrahmen getrocknet/kondensiert (170 °C, 2 min). Um den Griff der Ware nach der Ausrüstung zu verbessern, werden die ausgerüsteten Gewebe 60 min im Wäschetrockner (Tumbler) behandelt Abschliessend werden jeweils drei Stoffmuster (zugeschnitten auf 37x50 cm) foulardiert und die durchschnittlichen Flottenaufnahmen bezogen auf das Trockengewicht bestimmt. Waschmaschinenpermanenz: Wie in Versuchsreihe 2. Ladungstitration: Probenvorbereitung und Ladungstitration mit Charge Analysing System (CAS) wie in Versuchsreihe 1 und 2.

**Tabelle: Relative Ladungspermanenz**

| *Ausrüstung* | *Gewebe* | *Anzahl MM* | *Ausrüstungsauflage, berechnet aus Flottengehalt vor Trocknen*/*%-Gew.* | *Relative Ladungspermanenz in %, normiert auf 4%-Gew. Auflage vor MM* |
|---|---|---|---|---|
| A-084-I | 100% PA | 1 | 3.69 | 80 |
| A-084-I | | 5 | 3.69 | 106 |
| A-084-I | | 25 | 3.69 | 106 |
| A-084-IV | | 50 | 3.69 | 104 |
| A-084-V | | 100 | 3.69 | 106 |
| A-085-I | 100% PES | 1 | 4.82 | 48 |
| A-085-II | | 5 | 4.82 | 55 |
| A-085-III | | 25 | 4.82 | 45 |
| A-085-IV | | 50 | 4.82 | 40 |
| A-085-V | | 100 | 4.82 | 21 |
| A-086-I | 83% PES/17% PUE | 1 | 3.48 | 61 |
| A-086-I | | 5 | 3.48 | 56 |
| A-086-III | | 25 | 3.48 | 63 |
| A-086-IV | | 50 | 3.48 | 73 |
| A-086-V | | 100 | 3.48 | 106 |
| A-087-I | 100% CO | 1 | 5.56 | 103 |
| A-087-I | | 5 | 5.56 | 109 |
| A-087-III | | 25 | 5.56 | 139 |
| A-087-IV | | 50 | 5.56 | 164 |
| A-087-V | | 100 | 5.56 | 174 |

**Zusammenfassung:** Die durchschnittlichen Flottenaufnahmen der Stoffmuster liegen für PA bei 55% (A-084), für PES bei 72% (A-085), für PES/PUE bei 52% (A-086) und für CO bei 83% (A-087). Dadurch werden unterschiedliche Auflagen erreicht, und zwar rund 3.7% Polymer auf den PA-Geweben, rund 4.8% Polymer-Auflage auf den PES-Geweben, rund 3.5% Polymer-Auflage auf den PES/PUE-Geweben und rund 5.6% Polymer-Auflage auf den CO-Geweben.

Vergleich der Produktionsversuche A-084 bis A-087: Der Produktionsversuch A-084 weist sowohl eine hervorragende Ladungszugänglichkeit als auch relative Ladungspermanenz auf, was eine hohe Wirkstoff-Beladungskapazität ermöglicht. Die Polyelektrolytverbräuche liegen bei den ungewaschenen Stoffmustern bei 7 ml 0.001 N PolyDADMAC. Der PolyDADMAC-Verbrauch des gewaschenen Stoffmusters (1 MW bei 60 °C während 50-55 min) liegt bei 5.5 ml 0.001 N PolyDADMAC. Die Verbräuche nach 5, 25, 50 und 100 MW steigen auf fast 8 ml 0.001 N PolyDADMAC an, was mitunter durch das Auswaschen unvernetzter Polymeranteile verursacht wird. Dadurch steigt die relative Ladungspermanenz auf über 100% an. Der Produktionsversuch A-085 ist ebenfalls über 100 Mal waschbeständig. Allerdings kann aufgrund der fehlenden Ankergruppen des Polyesters deutlich weniger Donorschicht aufgebracht werden, was sich durch Polyelektrolytverbräuche der gewaschenen Stoffmuster im Bereich von 2 ml 0.001 N PolyDADMAC äussert. Die relative Ladungspermanenz liegt bei rund 45%. Der Produktionsversuch A-086 ist ebenso über 100 Mal waschbeständig. Die Polyelektrolytverbräuche liegen bei den ungewaschenen Stoffmustern (1, 5 und 25 MW bei 60 °C während 50-55 min) bei 4 ml 0.001 N PolyDADMAC. Danach steigen nach 50 und 100 MW die PolyDADMAC-Verbräuche auf 5 ml bzw. 7 ml an. Offenbar nimmt die Elastizität der Donorschicht mit der Zeit zu, wodurch sich auch die analytisch erfassbare Oberflächenladung erhöht. Auch der Produktionsversuch A-087 ist über 100 Mal waschbeständig. Wie bei A-086 steigt auch hier mit zunehmender Quellzyklenzahl die Ladungszugänglichkeit. Nach 100 MW erreicht der PolyDADMAC-Verbrauch mit 11.4 ml den maximalen, theoretisch möglichen Verbrauch von 11.6 ml 0.001 N PolyDADMAC. Folglich entspricht die relative Ladungspermanenz von 174% nach 100 MW für CO der absoluten Schichtpermanenz, da beim Waschen nichts von der Donorschicht verloren geht.

### Versuchsreihe 11

Produktionsversuch basierend auf A-084 bis A-087. Imprägnierung mit einer 15%-igen Polymer-Dispersion von P-067 (Scale-up). Gegenüber P-044 mit 40% mPEG-350-Methacrylat und 20% 2-Ethylhexyl-acrylat enthält P-067 nur 20% mPEG-350-Methacrylat, dafür aber 40% 2-Ethylhexyl-acrylat und ist damit deutlich lipophiler.

Versuchablauf analog zu Versuchsreihe 3. Gewebe analog zu Versuchsreihe 1 und 2. Katalysator-Stammlösung analog zu Versuchsreihe 3.

**Gewebe-Imprägnieransatz:** 330 g Scale-up P-067 Reaktionslösung; 53 g Lamethan NKS-AF, CHT R. Beitlich GmbH, 292 g Wasser (nicht deionisiert); 75 g Katalysator-Stammlösung. Der Katalysator wird erst unmittelbar vor dem Foulardieren zugegeben.

Waschmaschinenpermanenz: Wie in Versuchsreihe 2. Ladungstitration: Probenvorbereitung und Ladungstitration mit Charge Analysing System (CAS) wie in Versuchsreihe 1 und 2.

**Tabelle:Ladungspermanenz**

| *Ausrüstung* | *Gewebe* | *Anzahl MW* | *Ausrüstungsauflage, berechnet aus Flottengehalt vor Trocknen*/*%-Gew.* | *Relative Ladungspermanenz in* %, *normiert auf 496-Gew. Auflage vor MW* |
|---|---|---|---|---|
| A-088-I | 100% PA | 1 | 3.36 | 54 |
| A-088-II | | 5 | 3.36 | 71 |
| A-088-III | | 25 | 3.36 | 73 |
| A-088-IV | | 50 | 3.36 | 74 |
| A-088-V | | 100 | 3.36 | 59 |

**Zusammenfassung:** Die durchschnittliche Flottenaufnahme der Stoffmuster liegt im Bereich von 49%, wodurch eine Auflage von rund 3.4% Polymer auf den PA-Geweben erreicht wird. Der Produktionsversuch A-088 weist ebenso wie A-084 eine hervorragende Ladungszugänglichkeit auf. Aufgrund des lipophileren Charakters der amphiphilen Donorschicht liegen die Polyelektrolytverbräuche der gewaschenen Stoffmuster (1, 5, 25, 50 und 100 MW bei 60 °C während 50-55 min) gegenüber A-084 mit rund 6 ml 0.001 N PolyDADMAC etwas tiefer. Die relative Ladungspermanenz liegt im Bereich von 70%.

### C. Zytotoxizitätstest

Um die Verträglichkeit von erfindungsgemäss ausgerüsteten Textilien zu prüfen, wurde eine Gewebeprobe einem Zytotoxizitätstest nach DIN EN ISO 10993-5 ("Biologische Beurteilung von Medizinprodukten - Teil 5: Prüfungen auf In-vitro-Zytotoxizität") unterzogen. Bei der Gewebeprobe handelt es sich um 100%PA-Gewebe, welches mit Ausrüstungsformulierung Nr. A-084 ausgerüstet worden war. Die Gewebeprobe wurde vorher ein Mal gewaschen (60 °C während 50-55 min). Das Gewebe war nicht mit einer Emulsion bzw. Wirkstoffen beladen.

Die Prüfung auf Zytotoxizität nach DIN EN ISO 10993-5 ist als Basis für alle Medizinprodukte anerkannt und erforderlich. Durch den Einsatz von Zellkulturen ist es möglich, aus den geprüften Produkten herauslösbare toxische Substanzen nachzuweisen. Die Freisetzung toxischer Substanzen aus einem Textilprodukt mit Hautkontakt ist Voraussetzung für die Entstehung einer Hautirritation. Die Prüfung auf Zytotoxizität erlaubt die Beurteilung eines Gefahrenpotenzials zur Hautirritation. Dieses wird als Summenparameter erfasst.

**Methode**: Zelllinie: L 929 Zellen (ATCC Nr. CCL1, NCTC Klon 929 L (DSMZ)), Passagenzahl: 31. Kulturmedium: DMEM mit 10% FCS. Extraktionsverfahren: Inkubation des Untersuchungsgutes mit saurer Schweisslösung nach Norm DIN EN ISO 105-E04 für 24 h unter leichtem Schütteln bei 37 °C; die Schweisslösung wird auf pH 7.3-7.4 eingestellt und sterilfiltriert. Inkubation der Zellkultur: 68-72 h mit Schweisslösung in Verdünnungsstufen von 33, 3% bis 9, 9%. Untersuchung der Zytotoxizität Nach Inkubation der Zellen wird der Proteingehalt als Mass für das Zellwachstum mit dem der Kontrollen verglichen (Biol. Toxicol. 1984, 1(1), 55-65). Eine Positiv- und eine Negativkontrolle wird im Experiment mitgeführt, um die Validität des Testsystems zu bestätigen. In Gegenwart zelltoxischer Substanzen zeigen sich veränderte Proliferations- und Teilungsraten der Zellen (Wachstumshemmungs-Test). Prüfmaterial: Konzentrationen des Prüfmaterials in Kulturmedium: 9.9%, 14.8%, 22.2% und 33.3%.

**Resultat:** Im Schweissextrakt wurde mit blossem Auge keine Verfärbung beobachtet. Es wurde am Probenmaterial bzw. Schweissextrakt ein schwacher süsslicher Geruch festgestellt Am Schweissextrakt wurde eine Erhöhung des pH-Wertes von 5.5 auf 6.0 beobachtet. Es ergaben sich folgende Werte:

| Konz. Prüfmaterial/%.-Vol. | Wachstumshemmung/% |
|---|---|
| 33.3 | 18 |
| 22.2 | 13 |
| 14.8 | 10 |
| 9.0 | 9 |

Nach DIN EN ISO 10993-5 wird eine Wachstumshemmung von mehr als 30% im Vergleich zur Lösungsmittelkontrolle als eine zytotoxische Wirkung angesehen. Mit der beschriebenen Prüfmethodik zeigte der Schweissextrakt der Probe eine Wachstumshemmung von 18% im Zytotoxizitätstest. Ausgehend vom Prüfmaterial konnte eine dosisabhängige Wachstumshemmung der L929 Zellen beobachtet werden, jedoch wurde die Signifikanzgrenze von 30% nicht überschritten. Unter Berücksichtigung der Beurteilungskriterien zeigt die Probe keine biologische Aktivität Daraus ist zu schliessen, dass unter den angegebenen Bedingungen aus dem Untersuchungsgut keine zelltoxischen Substanzen freigesetzt werden, die bei Hautkontakt zu Irritationen führen können.

### D. Emulsionen

Um ein effizientes Aufbringen der meist lipophilen Wirkstoffe aus einer verdünnten Emulsion auf die negativ geladene erfindungsgemässe Ausrüstungsschicht zu ermöglichen, wird ein Emulgator verwendet, welcher über entsprechende positive Ladungen verfügt. Es resultieren Wirkstoff-Emulsionen mit wirkstoffhaltigen Ölphasentröpfchen, die sich aufgrund der vorhandenen positiven Oberflächenladung gut in die mit negativen Ladungsstellen versehene Matrix der erfindungsgemässen Ausrüstungsschicht einlagern. Der Wirkstoff kann nach dem Beladen der Ausrüstungsschicht mit der Emulsion aus den emulgierten Öltröpfchen in die lipophilen Bereiche der Polymermatrix diffundieren. Aus der Ölphase der adsorbierten Emulsionspartikel und/oder der lipophilen Phasen der Polymermatrix kann anschliessend der Wirkstoff abgegeben werden.

Für den offenbarungsgemässen Einsatz geeignet sind Phosphatidylcholin-Lecithine, wie beispielsweise 1-Palmityl-2-oleyl-sn-glycero-3-phosphatidylcholin (POPC, Palmityloleylphosphatidylcholin). Die positive Ladung an der Oberfläche der emulgierten Öltröpfchen interagiert aufgrund der Amingruppe mit den negativen Ladungsstellen der erfindungsgemässen Polymerausrüstungen. 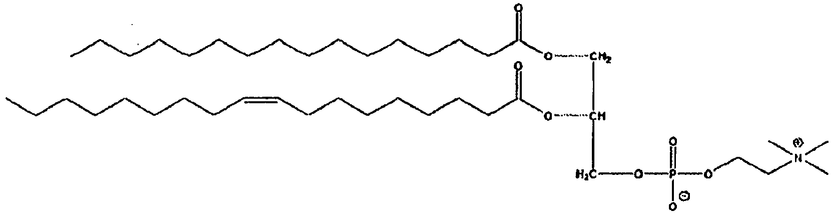 lecithinhaltige Öl/Wasser-Mikroemulsionen sind bekannt. Beispielhaft sei dabei auf DE 198 59 427 A1 verwiesen. In der Regel wird dabei eine Makroemulsion durch Hochdruckhomogenisierung in eine Mikroemulsion oder Miniemulsion umgewandelt.

Ebenfalls geeignet für Mikroemulsionen ist zum Beispiel Behenyltrimethylammonium-methosulfat:

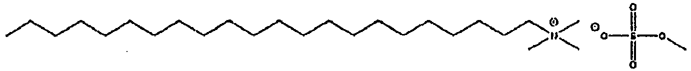

Behenyltrimethylammonium-methosulfat in Kombination mit Cetylalkohol 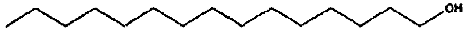 als Coemulgator ist erhältlich als Varisoft BTMS Flake (Evonik Goldschmidt GmbH), mit 25%-Gew. BTMS und 75%-Gew. Cetylalkohol. Der Cetylalkohol weist in diesem Fall jedoch die Tendenz auf, sich beim Beladen der erfindungsgemässen Ausrüstungsschicht abzusetzen.

Als besonders geeigneter Emulgator für eine offenbarungsgemässe Emulsion hat sich Ethyl-N^{alpha}-Lauroyl-L-Arginat/HCl erwiesen (nachfolgend Lauroylarginat).

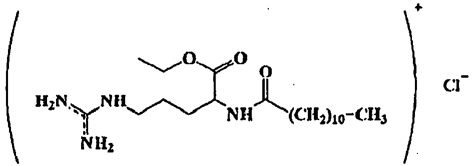

Die Synthese von Lauroylarginat (CAS-Nr. 60372-77-2) wird beschrieben in WO 01/94292 A1, und ist erhältlich von Laboratorios Miret SA, Barcelona, Spanien. Lauroylarginat ist ein weisser, hygroskopischer Feststoff, der bis zu 247 g/kg in Wasser dispergierbar ist. Der Schmelzpunkt liegt bei 50 °C bis 58 °C.

Lauroylarginat wird verwendet als antimikrobieller Konservierungsstoff, wobei seine antimikrobiellen Eigenschaften auf seinen Eigenschaften als kationisches Tensid beruhen. Lauroylarginat ist wirksam gegen ein breites Spektrum an Gram-negativen und -positiven Bakterien sowie Hefen und Schimmelpilzen. Es unterdrückt das Wachstum von Bakterienkolonien, führt jedoch nicht zur Lysis von Zellen. Es wird für die Verwendung in kosmetischen Produkten (Scientific Committee on Consumer Products SCCP, Opinion on Ethyl lauroyl arginate HCl, SSCP/1106/07, 15 April. 2008) und in Nahrungsmitteln (Food Standards Australia New Zealand, Application A1015, Ethyl Lauroyl Arginate as a food additive - assessment report, 6 May 2009) als zur Zulassung empfohlen.

Aus WO 02/087328 A2 ist die Verwendung von Lauroylarginat in Kombination mit Sorbinsäure, Kaliumsorbat oder Natriumsorbat bekannt. In WO 2008/014824 A1 wird die antivirale Aktivität von Lauroylarginat postuliert. Aus WO 2007/014580 A1 wiederum sind weitere, ähnliche antimikrobiell wirksame Tenside bekannt. Die Offenbarung der vorgenannten Dokumente soll hiermit als Bestandteil dieser Beschreibung gelten.

Es ist aus dem Stand der Technik nicht bekannt, Lauroylarginat als Emulgator einzusetzen. Die Verwendung von Lauroylarginat zu diesem Zweck hat zum einen den Vorteil, dass für eine entsprechende Emulsion keine weiteren Konservierungsmittel mehr notwendig sind. Zum anderen ist gleichzeitig die gesundheitliche Unbedenklichkeit einer solchen Emulsion gegeben.

Im Weiteren findet eine Übertragung der antimikrobiellen Eigenschaften der Emulsion auf das Textil statt, wodurch dieses selbst bakteriostatische Eigenschaften annimmt. Dies aufgrund der geringen Wirkkonzentrationen im µg/ml-Bereich von Lauroylarginat gegen unterschiedlichste Mikroorganismen und der relativ hohen Einsatzkonzentration von bis zu 0.8% dieses kationischen Emulgators in der Emulsion. Eine besonders hohe Wirksamkeit der mit der Emulsion beladenen Gewebe wird durch den vorgängig beschriebenen Einsatz von Lauroylarginat in Kombination mit z.B. Sorbaten oder Benzoaten erzielt, wodurch ein synergistischer Effekt dieser Wirksubstanzen zum Tragen kommt.

Als Beispiel eines Trägermediums bzw. Wirkstoffes, mit welchem eine erfindungsgemässe Ausrüstungsschicht beladbar ist, wird Nachtkerzenöl verwendet. Die Samen der gemeinen Nachtkerze (Oenothera biennis) enthalten ungefähr 7-10%-Gew. Linolensäure. Das entsprechende Öl der Nachtkerze wirkt beispielsweise schmerzlindernd bei prämenstruellem Syndrom, und hat einen heilenden Effekt bei Hautkrankheiten wie Psoriasis (Schuppenflechte) und Neurodermitis. Nachtkerzenöl besteht aus 71%-Gew. Linolsäure, 10%-Gew. gamma-Linolensäure, 7%-Gew. Ölsäure, 2%-Gew. Stearinsäure, 7%-Gew. Palmitinsäure und 3%-Gew. weiteren Substanzen. Der aktive Wirkstoff ist gamma-Linolensäure.

Alternativ können als Trägermedium für lipophile aktive Wirksubstanzen auch andere lipophile Medien, z.B. auf Paraffin- oder Paraffinölbasis, verwendet werden. Diese sollten natürlich vorzugsweise hautverträglich sein. Hydrophile Wirksubstanzen wiederum können im Inneren von Wasser-in-Öl-in-Wasser-Emulsionen oder Liposomen auf erfindungsgemässe Ausrüstungsschichten beladen werden.

WO 2007/050580 A2 und US 5474783 offenbaren eine Reihe von aktiven Wirkstoffen, welche für eine transdermale Aufnahme geeignet sind. Der Offenbarungsgehalt dieser Schriften bildet einen integralen Bestandteil dieser Beschreibung.

Zusätzlich zu den eigentlichen aktiven Wirksubstanzen können auch noch Hilfsstoffe bereitgestellt werden, die beispielsweise die Aufnahme der Wirksubstanz in die Haut verbessern (sogenannte "Penetration Enhancers") oder z.B. osmophore oder chromophore Gruppen enthalten, die als Duftstoffe bzw. Farbstoffe den sensorischen Konsumentenbedürfnissen Rechnung tragen.

Als weitere Hilfs- bzw. Zusatzstoffe sind zudem sämtliche Substanzen geeignet, die z.B. das Einfrieren der Emulsion verhindern oder generell die Emulsionsstabilität über einen weiten Temperaturbereich und Zeitraum gewährleisten, indem diese sowohl die chemische, als auch die physikalische und biologische Stabilität derselben erhöhen. Des Weiteren kann die Emulsion Bitterstoffe enthalten, um die unkontrollierte Einnahme derselben durch Kinder zu verhindern.

**Tabelle: Ausführungsbeispiele für Emulsionen**

| *Emulsion Nr.* | *E-018* | *E-019* | *E-020* | *E-021* | *E-022* |
|---|---|---|---|---|---|
| Nachtkerzenöl raffiniert (Wirkstoff), Carl Roth GmbH + Co. KG, Prod. Nr. 3691.2 [%Gew.] | 25.5 | 25.5 | 25.5 | 25.5 | 25.5 |
| Lecithin 97%, Carl Roth GmbH + Co. KG, Prod. Nr. 9812.1 [%-Gew.] | 2.0 | - | - | - | 1.5 |
| Lipoid S40 (Lecithinemulsion), Lipoid GmbH, Prod. Nr. 740046 [%-Gew.] | - | 2.0 | - | - | - |
| Phosal 50 PG (enthält 40% PG), Phospholipid GmbH, Prod. Nr. 368214 [%-Gew.] | - | - | 2.0 | - | - |
| Varisoft BTMS Flake (25% BTMS, 75% Cetylalkohol), Evonik Goldschmidt GmbH, Prod. Nr. 204109 [%-Gew.] | - | - | - | 2.0 *) | 2.0 *) |
| Propylenglykol > = 99.5% (GC), Fluka [%-Gew.] | 6.5 | 6.5 | 6.5 | 6.5 | 6.5 |
| Wasser deionisiert [%-Gew.] | 65.5 | 65.5 | 65.5 | 65.5 | 65.5 |

| | | | | | |
|---|---|---|---|---|---|
| *) entspricht 0.5%-Gew. kationischem Emulgator | | | | | |

### Emulsion E-018

Herstellung: In einem 250 ml Erlenmeyerkolben werden 50.0 g Nachtkerzenöl vorgelegt. Dann werden unter Rühren 4.0 g Lecithin zugegeben. Die Mischung wird auf dem Magnetrührer gerührt, und nachdem das Lecithin gleichmässig aufgequollen ist, werden unter intensivem Rühren langsam 13.0 g Propylenglykol und 133.0 g Wasser hinzugetropft und während 3 h weitergerührt (schwer mischbar). Bestimmung der Homogenität: 1 g Emulsionslösung werden mit 19 g Wasser verdünnt (1:20). Viskosität: 1.04 mPa · s (Bei allen Viskositätsmessungen: Viskosimeter der CBC Materials Co., Ldt., Tokio, Japan. Modell: Viscomate VM-10A-L, Messfühler: PR-10-L). Partikelgrössenverteilung (PCS-Messung): Peak 1: d(H) = 1350 nm - 70.2%-Vol.; Peak 2: d(H) = 5510 nm - 27.0%-Vol.; Peak 3: d(H) = 141 nm - 2.8%-Vol.

Homogenisation: Die Emulsionslösung wird fünf Mal bei 600 bar durch den Hochdruck-Homogenisierer (APV Products, DK-2620 Alberstlund, Modell Nr. APV-2000) gefördert. Es resultieren 200 g dünnflüssige, weiss-gelbliche Emulsion. Bestimmung der Homogenität: 1 g homogenisierte Emulsionslösung wird mit 19 g Wasser verdünnt (1:20). Viskosität: 0.99 mPa · s. Partikelgrössenverteilung (PCS-Messung): Peak 1: d(H) = 568 nm - 92.0%-Vol.; Peak 2: d(H) = 4870 nm - 8.0%-Vol.; Peak 3: Kein Signal vorhanden.

### Emulsion E-019

Herstellung: In einem 250 ml-Erlenmeyerkolben werden 50.0 g Nachtkerzenöl vorgelegt. Dann werden unter Rühren 4.0 g Lipoid S 40 (Lipoid GmbH) zugegeben. Die Mischung wird auf dem Magnetrührer gerührt und nachdem das Lecithin gleichmässig aufgequollen ist, werden unter intensivem Rühren langsam 13.0 g Propylenglykol und 133.0 g Wasser hinzugetropft und während 2 h weitergerührt (gut mischbar). Es resultieren 200 g dünnflüssige, weiss-gelbliche Emulsion. Bestimmung der Homogenität: 1 g Emulsionslösung wird mit 19 g Wasser verdünnt (1:20). Viskosität: 1.03 mPa · s. Partikelgrössenverteilung (PCS-Messung): Peak 1: d(H) = 110 nm - 13.3%-Vol.; Peak 2: d(H) = 207 nm - 15.1%-Vol.; Peak 3: d(H) = 875 nm - 71.6%-Vol.

**Homogenisation**: Diese Emulsionslösung wird fünf Mal bei 600 bar durch den Hochdruck-Homogenisierer gefördert. Es resultieren 200 g dünnflüssige, weiss-gelbliche Emulsion. 1 g homogenisierte Emulsionslösung werden mit 19 g Wasser verdünnt (1:20). Viskosität: 0.99 mPa · s. Partikelgrössenverteilung (PCS-Messung): Peak 1: d(H) = 335 nm - 100%-Vol.; Peak 2: d(H) = Kein Signal vorhanden.

**Oberflächenladung:** Mittels CAS wurde eine Oberflächenladung von 6.65 µmol neg. Ladung/g Emulsion bestimmt. Notwendig wäre jedoch eine positive Oberflächenladung. Dies liegt daran, dass bei der Herstellung des Lecithins (Isolation aus Soja oder Eiern) teilweise die Cholingruppe abgespalten wird, womit die negativ geladene Phosphatgruppe zurückbleibt. Dies führt zu einer Reduktion der Oberflächenladung der Emulsionspartikel und damit eventuell zu einer neutralen oder gar schwach negativen Oberflächenladung der emulgierten Partikel.

### Emulsion E-020

**Herstellung:** In einem 250 ml-Erlenmeyerkolben werden 50.0 g Nachtkerzenöl vorgelegt Dann werden unter Rühren 6.7 g Phosal 50 PG (Phospholipid GmbH) zugegeben. Der Mischung werden unter intensivem Rühren auf dem Magnetrührer langsam 10.3 g Propylenglykol und 133.0 g Wasser hinzugetropft und während 2 h weitergerührt (enthält Öltropfen). Bestimmung der Homogenität: 1 g Emulsionslösung wird mit 19 g Wasser verdünnt (1:20). Viskosität: 0.98 mPa · s. Partikelgrössenverteilung (PCS-Messung): Peak 1: d(H) = 1150 nm - 82.6%-Vol.; Peak 2: d(H) = 215 nm - 12.8%-Vol.; Peak 3: d(H) = 5500 nm - 4.6%-Vol.

Homogenisation: Die Emulsionslösung wird fünf Mal bei 600 bar durch den Hochdruck-Homogenisierer gefördert. Es resultieren 200 g dünnflüssige, weiss-gelbliche Emulsion mit Öltropfen. Bestimmung der Homogenität 1 g homogenisierte Emulsionslösung werden mit 19 g Wasser verdünnt (1:20). Viskosität: 0.99 mPa · s. Partikelgrössenverteilung (PCS-Messung): Peak 1: d(H) = 679 nm - 100%-Vol.; Peak 2: d(H) = Kein Signal vorhanden.

**Oberflächenladung:** Beim CAS (Titration mit 0.01 N HCl) konnte ein positives elektrisches Potential erreicht werden. Damit ist prinzipiell gezeigt, dass die emulgierten Tröpfchen eine positive Oberflächenladung aufweisen. Die Langzeitstabilität der Emulsion ist jedoch ungenügend.

### Emulsion E-021

**Herstellung:** In einem 250 ml-Erlenmeyerkolben werden 25.0 g Nachtkerzenöl vorgelegt Dann werden unter Rühren 2.0 g Varisoft BTMS Flake zugegeben. Das Gemisch wird im Ölbad auf 75 °C erwärmt und während 2 h weitergerührt. Es entsteht eine viskose, weisse Mischung. In einen zweiten 250 ml-Erlenmeyerkolben werden 66.5 g Wasser vorgelegt und 6.5 g Propylenglykol hinzugegeben. Das Gemisch wird auf 60 °C erwärmt und anschliessend unter intensivem Rühren in die warme Ölphase (27 g) hinzugetropft Unter Rühren lässt man die Emulsionslösung auf Raumtemperatur abkühlen. Es resultieren 100 g hochviskose, weiss-gelbliche Emulsion. pH = 4.2. Bestimmung der Homogenität: 1 g Emulsionslösung wird mit 99 g Wasser verdünnt (1:100). Viskosität: 1.14 mPa · s. Partikelgrössenverteilung (PCS-Messung): Peak 1: d(H) = 618 nm - 100%-Vol.; Peak 2: d(H) = Kein Signal vorhanden.

**Oberflächenladung:** Die CAS Messungen (Titration mit 0.001 N Poly(Vinylsulfonsäure-Natriumsalz)) ergab eine Oberflächenladung der emulgierten Tröpfchen von 1.03 µmol pos. Ladung/g Emulsion.

Der Einsatz von Behenyltrimethylammonium-methosulfat als kationischen Emulgator anstelle von Lecithin zeigt, dass zwar eine hochviskose, aber stabile Emulsion erzeugt werden kann. Die Partikelgrössenverteilung ergibt eine monodisperse Emulsionstropfengrösse mit einem hydrodynamischen Durchmesser von 618 nm.

### Emulsion E-022

**Herstellung:** In einem 250 ml-Erlenmeyerkolben werden 25.0 g Nachtkerzenöl vorgelegt. Dann werden unter Rühren 2.0 g Varisoft BTMS Flake zugegeben. Das Gemisch wird im Ölbad auf 75 °C erwärmt und während 2 h weitergerührt. Nun werden 1.5 g Lecithin hinzugefügt und während 10 min weitergerührt. Es entsteht eine viskose, weiss-gelbe Mischung. In einem zweiten 250 ml-Erlenmeyerkolben werden 65.0 g Wasser vorgelegt und 6.5 g Propylenglykol hinzugegeben. Das Gemisch wird auf 60 °C erwärmt und anschliessend unter intensivem Rühren in die warme Ölphase (28.5 g) hinzugetropft. Unter Rühren lässt man die Emulsionslösung auf Raumtemperatur abkühlen. Es resultieren 100 g hochviskose, weiss-gelbe Emulsion. pH = 5.2. Bestimmung der Homogenität: 1 g Emulsionslösung wird mit 99 g Wasser verdünnt (1:100). Viskosität: 1.18 mPa · s. Partikelgrössenverteilung (PCS-Messung): Peak 1: d(H) = 755.nm - 100%-Vol.; Peak 2: d(H) = Kein Signal vorhanden.

**Oberflächenladung:** Die CAS Messungen (Titration mit 0.001 N Poly(Vinylsulfonsäure-Natriumsalz)) ergab eine Oberflächenladung der emulgierten Tröpfchen von 0.29 µmol pos. Ladung/g Emulsion.

Die Zugabe von Lecithin reduziert wie erwartet die Oberflächenladung der Emulsionstropfen im Vergleich zu E-021. Die Partikelgrössenverteilung ergibt wie bei E-021 eine monodisperse Emulsionstropfengrösse mit einem hydrodynamischen Durchmesser von 755 nm. Dies bedeutet, dass sowohl das Lecithin als auch das Behenyltrimethylammonium-methosulfat in denselben Emulsionströpfchen eingelagert sind.

### Emulsion E-023

Die Lecithinemulsion wird mit einer Calciumchlorid-Lösung versetzt um zu überprüfen, ob die Phosphatgruppen zweier Lecithinmoleküle mit CaCl₂ das schwerlösliche Calciumphosphat bilden. Die dabei freigesetzten Chloridionen stehen danach den Trialkylammoniumgruppen des Lecithins als Gegenionen zur Verfügung. Durch die damit verbundene Abschirmung der stark anionischen Phosphatgruppen soll die Lecithinemulsion eine kationische Oberflächenladung erhalten. Bei einer Lecithin-Molmasse von M = ca. 760 g/mol entsprechen 4.0 g Lecithin 5.3 mmol. D.h. es muss halb soviel Calciumchlorid eingesetzt werden, um die gesamten Phosphatgruppen des eingesetzten Lecithins abzuschirmen. Dadurch wird ebenfalls ein Salzüberschuss vermieden, welcher die Emulsion destabilisieren würde.

**Herstellung:** In einem 250 ml-Erlenmeyerkolben werden 50.0 g Nachtkerzenöl vorgelegt. Dann werden unter Rühren 4.0 g Lecithin zugegeben. Die Mischung wird auf dem Magnetrührer gerührt, und nachdem das Lecithin gleichmässig aufgequollen ist, werden unter intensivem Rühren langsam 13.0 g Propylenglykol und 133.0 g Calciumchlorid-Lösung (0.3 g CaCl₂ in 132.7 g Wasser) hinzugetropft und während 4 h weitergerührt. Es bildet sich eine niedrigviskose, weiss-gelbliche, zweiphasige Mischung. Beim Stehenlassen bildet sich sofort eine obere, weiss-gelbliche Suspensionsphase und eine untere, klare, wässrige Phase. Homogenisation: Die Emulsionslösung wird 15 Mal bei 600 bar durch den Hochdruck-Homogenisierer (APV Products, DK-2620 Alberstlund, Modell Nr. APV-2000) gefördert. Es resultiert eine dünnflüssige, weiss-gelbliche nicht stabile Emulsion. Fazit: Die Elektrolytzugabe bei E-018 und E-023 verursacht im Bereich des Ladungsnullpunktes eine instabile Emulsion, wodurch diese bricht.

### Emulsion E-026

Die Elektrolytzugabe bei E-018 und E-023 verursacht im Bereich des Ladungsnullpunktes eine instabile Emulsion, wodurch diese bricht. Um dies zu vermeiden, wird das Lecithin im nichtwässrigen System umgeladen, um anschliessend mit dem dann kationischen Emulgator die Emulsion herzustellen. Dadurch muss der Ladungsnullpunkt der Emulsionslösung nicht durchschritten werden, da die Emulsion erst nachträglich mit dem bereits kationischen Lecithin erzeugt wird. Dazu wird mittels einer Aluminiumchlorid-Cosolvent-Lösung und einem zur Emulsionsstabilisierung geeigneten Coemulgator im nichtwässrigen System das Lecithin umgeladen, um mit dem kationischen Lecithin die Emulsion herzustellen.

Bei einer Lecithin-Molmasse von M = ca. 760 g/mol entsprechen 4.0 g Lecithin 5.3 mmol. D.h. es muss ein Drittel soviel Aluminiumchlorid eingesetzt werden, um die gesamten Phosphatgruppen des eingesetzten Lecithins abzuschirmen. Dadurch wird ein Salzüberschuss vermieden, welcher die Emulsion destabilisieren würde.

**Herstellung Lecithin-Ölphase:** In einem 250 ml-Erlenmeyerkolben werden 400 g Nachtkerzenöl vorgelegt. Dann werden unter Rühren 32 g Lecithin zugegeben. Die Mischung wird auf dem Magnetrührer gerührt bis das Lecithin gleichmässig aufgequollen ist. Es ergeben sich 432 g gelbe Lecithin-Ölphase.

**Herstellung E-026-A mit 0 eq. Aluminiumchlorid in Ethylenglykol (Blindwert):** In einem 1 I-Erlenmeyerkolben werden 54 g Lecithin-Ölphase vorgelegt und anschliessend unter intensivem Rühren langsam 16.7 g Cosolvent-Coemulgator-Lösung (12.7 g Ethylenglykol und 4 g Marlipal 013/30) hinzugetropft Bei Marlipal® 013/30 handelt es sich um ein Alkoholethoxylat mit einer C₁₃-Kette und 3 Ethylenoxidgruppen, welches bei der Firma Sasol erhältlich ist.

Wiederum werden unter intensivem Rühren langsam 129 g Wasser (inkl. 400 µl 37%-ige Formaldehyd-Lösung zur Konservierung) hinzugetropft und über Nacht weitergerührt. Es ergeben sich 200 g weiss-gelbe Mischung (keine Phasentrennung). Diese Emulsionslösung wird fünf Mal bei 600 bar durch den Hochdruck-Homogenisierer gefördert Es ergeben sich 150 g dünnflüssige, weisse Emulsion; pH = 6.3; Leitfähigkeit = 455 µS/cm. 1 g homogenisierte Emulsionslösung wird mit 39 g Wasser verdünnt (1:40). Es ergibt sich eine Viskosität von 1.06 mPa · s. Partikelgrössenverteilung: Peak 1: d(H) = 153 nm - 100%-Vol. (PCS-Messung); Peak 2: d(H) = Kein Signal vorhanden.

**Herstellung E-026-8 mit 0.5 eq. Aluminiumchlorid in Ethylenglykol:** In einem 250 ml-Erlenmeyerkolben werden 54 g Lecithin-Ölphase vorgelegt und anschliessend unter intensivem Rühren langsam 16.8 g Aluminiumchlorid-Cosolvent-Coemulgator-Lösung (0.12 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und während 4 h weitergerührt. Es ergeben sich 200 g weiss-gelbliche Mischung (keine Phasentrennung). Diese Emulsionslösung wird fünf Mal bei 600 bar durch den Hochdruck-Homogenisierer gefördert. Es ergeben sich 150 g dünnflüssige, weisse Emulsion; pH = 3.0 (Aluminiumchlorid reagiert sauer in wässriger Phase); Leitfähigkeit = 1234 µS/cm. 1 g homogenisierte Emulsionslösung wird mit 39 g Wasser verdünnt (1:40); Viskosität: 0.98 mPa · s; Partikelgrössenverteilung: Peak 1: d(H) = 527 nm - 100%-Vol.; (PCS-Messung); Peak 2: d(H) = Kein Signal vorhanden.

**Herstellung E-026-C mit 0.75 eq. Aluminiumchlorid in Ethylenglykol**: In einem 250 ml-Erlenmeyerkolben werden 54 g Lecithin-Ölphase vorgelegt und anschliessend unter intensivem Rühren langsam 16.9 g Aluminiumchlorid-Cosolvent-Coemulgator-Lösung (0.18 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und während 4 h weitergerührt. Es ergeben sich 200 g weiss-gelbliche Mischung (Phasentrennung über Nacht). Diese Emulsionslösung wird fünf Mal bei 600 bar durch den Hochdruck-Homogenisierer gefördert. Es ergeben sich 150 g dünnflüssige, weisse Emulsion; pH = 2.6 (Aluminiumchlorid reagiert sauer in wässriger Phase); Leitfähigkeit = 1807 µS/cm. 1 g homogenisierte Emulsionslösung wird mit 39 g Wasser verdünnt (1:40). Viskosität: 0.98 mPa · s; Partikelgrössenverteilung: Peak 1: d(H) = 792 nm - 100%-Vol. (PCS-Messung); Peak 2: d(H) = Kein Signal vorhanden.

**Herstellung E-026-D mit 1.0 eq. Aluminiumchlorid in Ethylenglykol:** In einem 250 ml-Erlenmeyerkolben werden 54 g Lecithin-Ölphase vorgelegt und anschliessend unter intensivem Rühren langsam 16.9 g Aluminiumchlorid-Cosolvent-Coemulgator-Lösung (0.23 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und während 4 h weitergerührt. Es ergeben sich 200 g weiss-gelbliche Mischung (keine Phasentrennung). Diese Emulsionslösung wird fünf Mal bei 600 bar durch den Hochdruck-Homogenisierer gefördert. Es ergeben sich 150 g dünnflüssige, weisse Emulsion; pH = 2.6 (Aluminiumchlorid reagiert sauer in wässriger Phase); Leitfähigkeit = 2160 µS/cm. 1 g homogenisierte Emulsionslösung wird mit 39 g Wasser verdünnt (1:40); Viskosität: 0.99 mPa · s; Partikelgrössenverteilung: Peak 1: d(H) = 899 nm - 93.8%-Vol. (PCS-Messung); Peak 2: d(H) = 5126 nm - 6.2%-Vol.; Peak 3: d(H) = Kein Signal vorhanden.

**Herstellung E-026-E mit 1.25 eq. Aluminiumchlorid in Ethylenglykol:** In einem 250 ml-Erlenmeyerkolben werden 54 g Lecithin-Ölphase vorgelegt und anschliessend unter intensivem Rühren langsam 17.0 g Aluminiumchlorid-Cosolvent-Coemulgator-Lösung (0.29 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und während 4 h weitergerührt. Es ergeben sich 200 g weiss-gelbliche Mischung (keine Phasentrennung). Diese Emulsionslösung wird fünf Mal bei 600 bar durch den Hochdruck-Homogenisierer gefördert. Es ergeben sich 150 g dünnflüssige, weisse Emulsion; pH = 2.6 (Aluminiumchlorid reagiert sauer in wässriger Phase); Leitfähigkeit = 2420 µS/cm. 1 g homogenisierte Emulsionslösung wird mit 39 g Wasser verdünnt (1:40); Viskosität: 0.99 mPa · s; Partikelgrössenverteilung: Peak 1: d(H) = 1055 nm - 100%-Vol. (PCS-Messung); Peak 2: d(H) = Kein Signal vorhanden.

**Herstellung E-026-F mit 1.5 eq. Aluminiumchlorid in Ethylenglykol:** In einem 250 ml-Erlenmeyerkolben werden 54 g Lecithin-Ölphase vorgelegt und anschliessend unter intensivem Rühren langsam 17.1 g Aluminiumchlorid-Cosolvent-Coemulgator-Lösung (0.35 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und über Nacht weitergerührt. Es ergeben sich 200 g weiss-gelbliche Mischung (keine Phasentrennung). Diese Emulsionslösung wird fünf Mal bei 600 bar durch den Hochdruck-Homogenisierer gefördert Es ergeben sich 150 g dünnflüssige, weisse Emulsion; pH = 2.7 (Aluminiumchlorid reagiert sauer in wässriger Phase); Leitfähigkeit = 2860 µS/cm. 1 g homogenisierte Emulsionslösung wird mit 39 g Wasser verdünnt (1:40); Viskosität 0.97 mPa · s; Partikelgrössenverteilung: Peak 1: d(H) = 835 nm - 97.0%-Vol. (PCS-Messung); Peak 2: d(H) = 5351 nm - 3.0%-Vol.; Peak 3: d(H) = Kein Signal vorhanden.

**Herstellung E-026-G mit 2.0 eq. Aluminiumchlorid in Ethylenglykol:** In einem 250 ml-Erlenmeyerkolben werden 54 g Lecithin-Ölphase vorgelegt und anschliessend unter intensivem Rühren langsam 17.2 g Aluminiumchlorid-Cosolvent-Coemulgator-Lösung (0.47 g Aluminiumchlorid in 12.7 g Ethylenglykol und 4 g Marlipal 013/30) hinzugetropft. Wiederum werden unter intensivem Rühren langsam 129 g Wasser (inkl. 400 µl 37%-ige Formaldehyd-Lösung zur Konservierung) hinzugetropft und während 4 h weitergerührt. Es ergeben sich 200 g weiss-gelbliche Mischung (keine Phasentrennung). Diese Emulsionslösung wird fünf Mal bei 600 bar durch den Hochdruck-Homogenisierer gefördert Es ergeben sich 150 g dünnflüssige, weisse Emulsion; pH = 2.8 (Aluminiumchlorid reagiert sauer in wässriger Phase); Leitfähigkeit = 3330 µS/cm. 1 g homogenisierte Emulsionslösung wird mit 39 g Wasser verdünnt (1:40); Viskosität 0.97 mPa · s; Partikelgrössenverteilung: Peak 1: d(H) = 854 nm - 100%-Vol. (PCS-Messung); Peak 2: d(H) = Kein Signal vorhanden.

pH-Titration mit Charge Analysing System (CAS): Zur CAS-pH-Titration wurde 0.1 N HCl Titrierlösung verwendet. Zur Vorbereitung, d.h. Reinigung der PTFE-Messzelle des CAS wird diese vor jeder Messung der Reihe nach mit deionisiertem Wasser, 2-Propanol, deionisiertem Wasser und Aceton (Spritzflaschen) gespült und anschliessend mit Druckluft ausgeblasen. Von Zeit zu Zeit wird die PTFE-Messzelle zusätzlich mit 65%-iger Salpetersäure gereinigt. 1.0 g Emulsionslösung wird in die PTFE-Messzelle des CAS eingewogen und mit 9.0 g Wasser aufgefüllt (Verdünnung 1:10). Dann wird der Messkolben in die Messzelle eingefügt und die CAS-pH-Titration am CAS gestartet.

**Tabelle: Oberflächenladung**

| *Ladungstitration* | *Anfangspotential *)* | *Isoelektrischer Punkt (pH bei CAS)* | *pH-Wert 1:10 verdünnt* | *Leitfähigkeit 1:10 verdunnt* |
|---|---|---|---|---|
| E-02.6-A (0eq.) | -321 mV | - | 5.5 | 76 µS/cm |
| E-026-B (0.5 eq.) | -122 mV | 4.0 | 3.8 | 227 µS/cm |
| E-026-C (0.75 eq.) | -29 mV | 4.8 | 3.6 | 345 µS/cm |
| E-026-D (1.0 eq.) | +38 mV | 5.0 | 3.5 | 412 µS/cm |
| E-026-E (1.25 eq.) | +55 mV | 6.8 | 3.5 | 462 µS/cm |
| E-026-F (1.5 eq.) | +70 mV | 6.4 | 3.6 | 545 µS/cm |
| E-026-G (2.0 eq.) | +99 mV | 6.5 | 3.5 | 590 µS/cm |

| | | | | |
|---|---|---|---|---|
| *) Die gemessenen Anfangspotentiale schwanken je nach Messung mehr oder weniger. | | | | |

**Zusammenfassung E-026-Emulsionen:** Es konnten sehr gute, d.h. dank dem Coemulgator Marlipal 013/30 stabile Emulsionen mit unterschiedlichen Äquivalenten Aluminiumchlorid hergestellt werden. Die Messresultate zeigen sehr schön, dass die Emulsionen mit weniger als 1.0 eq. AlCl₃ wie erwartet eine negative Oberflächenladung aufweisen. Die Lecithinemulsionen mit 1.0 eq. und mehr Äquivalenten AlCl₃ haben ein kationisches Oberflächenpotential. Damit konnte erfolgreich eine Serie umgeladener Lecithinemulsionen erzeugt werden. Zusätzlich wurden von all diesen Emulsionen CAS-pH-Titrationen durchgeführt. Die hierbei gemessenen Anfangspotentiale bestätigen die bereits zuvor gemessenen Anfangspotentiale. Im Weiteren zeigen diese Messungen sehr gut, wie die pH-Werte der isoelektrischen Punkte der Emulsionen mit steigendem Aluminiumchloridgehalt in Richtung neutral wandern. Er findet bei 1.25 eq. AlCl₃ (E-026-E) ein Maximum bei pH = 6.8 und nimmt bei höheren AlCl₃-Equivalenten wieder leicht auf pH = 6.5 ab (E-026-F und E-026-G). Der isoelektrische Punkt kann somit offenbar nicht weiter in den basischen pH-Bereich verschoben werden. Zudem wurden von allen Emulsionen die pH-Werte und die Leitfähigkeiten gemessen. Dies gilt nicht nur für die unverdünnten Emulsionen, sondern ebenfalls für die mit deionisiertem Wasser 1:10 verdünnten Emulsionen. Demnach liegt die maximale Leitfähigkeit der 1:10 verdünnten Proben mit 590 µS/cm bei E-026-G (2.0 eq. AlCl₃). Im Weiteren wurden auch noch CAS-pH-Titrationen mit unterschiedlich verdünnten Proben durchgeführt. So bleibt E-026-G mit 2.0 eq. AlCl₃ unverdünnt, 1:10 und 1:50 verdünnt immer kationisch.

### Emulsion E-027 - Kationische Emulsion mit LAE/Maltodextrin

Basierend auf Rezeptur E-021, jedoch mit 66% N^{alpha}-Lauroyl-L-arginin-ethylester-monohydrochlorid (LAE) mit 34% Maltodextrin als kationischem Emulgator. Die entsprechende Lösung ist unter dem Namen Mirenat-D von Vedeq SA erhätlich. LAE stabilisiert die Emulsion zugleich gegen mikrobiellen Abbau.

Herstellung: In einem 250 ml-Erlenmeyerkolben werden 50.0 g Nachtkerzenöl vorgelegt. Dann werden unter Rühren 6.0 g Mirenat-D (entspricht 2.0%-Gew. kationischem Emulgator) zugegeben, welches sich allerdings nicht im Öl löst. Die Mischung wird auf dem Magnetrührer gerührt und unter intensivem Rühren langsam 144.0 g Wasser hinzugetropft und während 2 h weitergerührt (gut mischbar). Es ergeben sich 200 g dünnflüssige, weiss-gelbliche Emulsion; pH = 3.4. Homogenisation: Diese Emulsionslösung wird fünf Mal bei 600 bar durch den Hochdruck-Homogenisierer gefördert. Es ergeben sich 150 g dünnflüssige, weiss-gelbliche Emulsion. 1 g homogenisierte Emulsionslösung wird mit 19 g Wasser verdünnt (1:20); Viskosität: 0.98 mPa · s; Partikelgrössenverteilung: Peak 1: d(H) = 228 nm - 100%-Vol. (PCS-Messung); Peak 2: d(H) = Kein Signal vorhanden.

Zusammenfassung: Der Einsatz von N^{α}-Lauroyl-L-arginin-ethylester-monohydrochlorid (LAE) in Maltodextrin als kationischem Emulgator anstelle von Behenyltrimethylammonium-methosulfat zeigt, dass eine dünnflüssige, weiss-gelbliche, stabile Emulsion erzeugt werden kann. Die Partikelgrössenverteilung ergibt eine monodisperse Emulsionstropfengrösse mit einem hydrodynamischen Durchmesser von 228 nm. Die CAS-pH-Titrationen zeigen, dass das Strömungspotential der Emulsion über den gesamten pH-Bereich von 3.0 bis 10.0 stets grösser als +343 mV ist.

### Emulsion E-028 - Kationische Emulsion mit LAE/Maltodextrin und Lecithin

Basierend auf Rezeptur E-022/E-027, jedoch mit Lecithin als zusätzlichen Emulgator.

Herstellung: In einem 250 ml-Erlenmeyerkolben werden 50.0 g Nachtkerzenöl vorgelegt Dann werden unter Rühren 4.0 g Lecithin (Carl Roth GmbH + Co. KG) zugegeben. Die Mischung wird auf dem Magnetrührer gerührt bis das Lecithin gleichmässig aufgequollen ist. Nun werden 6.0 g Mirenat-D zugegeben, welches sich allerdings nicht im Öl löst. Unter intensivem Rühren werden langsam 140.0 g Wasser hinzugetropft und während 2 h weitergerührt (gut mischbar). Es ergeben sich 200 g dünnflüssige, weiss-gelbliche Emulsion; pH = 3.9. Homogenisation: Diese Emulsionslösung wird fünf Mal bei 600 bar durch den Hochdruck-Homogenisierer gefördert Es ergeben sich 150 g dünnflüssige, weiss-gelbliche Emulsion. 1 g homogenisierte Emulsionslösung wird mit 19 g Wasser verdünnt (1:20); Viskosität: 1.00 mPa · s; Partikelgrössenverteilung: Peak 1: d(H) = 95 nm - 19.0%-Vol. (PCS-Messung); Peak 2: d(H) = 346 nm - 81.0%-Vol.; Peak 3: d(H) = Kein Signal vorhanden.

Zusammenfassung: Die Zugabe von Lecithin als zusätzlichen Emulgator funktioniert problemlos. Es entsteht eine dünnflüssige, weiss-gelbliche, stabile Emulsion. Die Partikelgrössenverteilung ergibt Emulsionstropfen mit hydrodynamischen Durchmessern von 95 nm (19.0%-Vol.) und 346 nm (81.0%-Vol.). Die CAS-pH-Titrationen zeigen, dass das Strömungspotential der Emulsion über den gesamten pH-Bereich von 3.0 bis 10.0 stets grösser als +229 mV ist.

### Emulsion E-029 - Kationische Emulsion mit LAE/Glycerin

Basierend auf Rezeptur E-027, jedoch mit N^{α}-Lauroyl-L-arginin-ethylester-monohydrochlorid (LAE) in Glycerin als kationischem Emulgator. Diese Lösung ist erhältlich als Aminat-G von der Vedeq SA, und enthält 20% N^{α}-Lauroyl-L-arginin-ethylester-monohydrochlorid (LAE) in 80% Glycerin.

Herstellung: In einem 250 ml-Erlenmeyerkolben werden 50.0 g Nachtkerzenöl vorgelegt. Dann werden unter Rühren 20.0 g Aminat-G zugegeben (entspricht 2.0%-Gew. kationischem Emulgator), welches sich allerdings nicht richtig mit dem Öl vermischt. Die Mischung wird auf dem Magnetrührer gerührt und unter intensivem Rühren langsam 130.0 g Wasser hinzugetropft und während 2 h weitergerührt (gut mischbar). Es ergeben sich 200 g dünnflüssige, weiss-gelbliche Emulsion; pH = 4.0. Homogenisation: Diese Emulsionslösung wird fünf Mal bei 600 bar durch den Hochdruck-Homogenisierer gefördert. Es ergeben sich 150 g dünnflüssige, weiss-gelbliche Emulsion. 1 g homogenisierte Emulsionslösung wird mit 19 g Wasser verdünnt (1:20); Viskosität: 0.99 mPa · s; Partikelgrössenverteilung: Peak 1: d(H) = 248 nm - 100%-Vol. (PCS-Messung); Peak 2: d(H) = Kein Signal vorhanden. Die Emulsion verdickt nach einigen Tagen und bildet Klumpen. Durch Schütteln lässt sich die Emulsion aber wieder verflüssigen.

Zusammenfassung: Der Einsatz von LAE in Glycerin als kationischem Emulgator anstelle von LAE in Maltodextrin zeigt, dass ebenfalls eine dünnflüssige, weiss-gelbliche Emulsion erzeugt werden kann. Allerdings verdickt die Emulsion nach einigen Tagen und bildet Klumpen. Durch Schütteln lässt sich die Emulsion aber wieder verflüssigen. Die Partikelgrössenverteilung ergibt eine monodisperse Emulsionstropfengrösse mit einem hydrodynamischen Durchmesser von 248 nm. Die CAS-pH-Titrationen zeigen, dass das Strömungspotential der Emulsion über den gesamten pH-Bereich von 3.0 bis 10.0 stets grösser als +386 mV ist.

### Emulsion E-030 - Kationische Emulsion mit LAE/Glycerin und Lecithin

Basierend auf Rezeptur E-028/029, jedoch mit Lecithin als zusätzlichen Emulgator.

Herstellung: In einem 250 ml-Erlenmeyerkolben werden 50.0 g Nachtkerzenöl vorgelegt. Dann werden unter Rühren 4.0 g Lecithin (Carl Roth GmbH + Co. KG) zugegeben. Die Mischung wird auf dem Magnetrührer gerührt bis das Lecithin gleichmässig aufgequollen ist. Nun werden 20.0 g Aminat-G zugegeben, welches sich allerdings nicht richtig mit dem Öl vermischt. Unter intensivem Rühren werden langsam 140.0 g Wasser hinzugetropft und während 2 h weitergerührt (gut mischbar). Es ergeben sich 200 g dünnflüssige, weiss-gelbliche Emulsion; pH = 4.3. Homogenisation: Diese Emulsionslösung wird fünf Mal bei 600 bar durch den Hochdruck-Homogenisierer gefördert. Es ergeben sich 150 g dünnflüssige, weiss-gelbliche Emulsion. 1 g homogenisierte Emulsionslösung wird mit 19 g Wasser verdünnt (1:20); Viskosität: 0.95 mPa · s; Partikelgrössenverteilung: Peak 1: d(H) = 233 nm - 100%-Vol. (PCS-Messung); Peak 2: d(H) = Kein Signal vorhanden.

Zusammenfassung: Die Zugabe von Lecithin als zusätzlichen Emulgator funktioniert problemlos. Es entsteht eine dünnflüssige, weiss-gelbliche, stabile Emulsion. Die Partikelgrössenverteilung ergibt eine monodisperse Emulsionstropfengrösse mit einem hydrodynamischen Durchmesser von 233 nm. Die CAS-pH-Titrationen zeigen, dass das Strömungspotential der Emulsion über den gesamten pH-Bereich von 3.0 bis 10.0 stets grösser als +306 mV ist.

### Emulsion E-031 - Kilo-Batch von kationischer Emulsion mit LAE/Glycerin, Lecithin und β-Carotin als Pseudowirkstoff

Basierend auf Rezeptur E-030, jedoch mit β-Carotin als Pseudowirkstoff und DL-alpha-Tocopherolacetat (Antioxidans) zur oxidativen Stabilisierung des Nachtkerzenöls. Die Konzentration von LAE als kationischer Emulgator wird auf 0.4% reduziert, da dies die maximal zulässige Konzentration von LAE in kosmetischen Produkten ist (gilt nicht für Produkte, die als Spray eingesetzt werden). Ebenso wird der Lecthingehalt auf 1.6% reduziert.

Herstellung: In einem 1 I-Erlenmeyerkolben werden 150.0 g Nachtkerzenöl vorgelegt. Dann werden unter Rühren 9.6 g Lecithin (Carl Roth GmbH + Co. KG), 3 mg DL-alpha-Tocopherolacetat (97.0%, HPLC, Fluka) und 322 mg β-Carotin (97.0%, UV, Fluka) zugegeben. Die Mischung wird im verschlossenen, mit Stickstoff belüfteten Erlenmeyerkolben auf dem Magnetrührer über Nacht gerührt bis sich alles komplett gelöst hat. In einem zweiten 1 I-Erlenmeyerkolben werden 428.1 g Wasser vorgelegt und 12.0 g Aminat-G zugegeben (entspricht 0.4% kationischem Emulgator und 1.6% Glycerin). Das Gemisch wird anschliessend unter intensivem Rühren in die 160 g Ölphase hinzugetropft und während 2 h weitergerührt. Es ergeben sich 600 g dünnflüssige, karottenrote Emulsion; pH = 5.2. Homogenisation: Diese Emulsionslösung wird fünf Mal bei 600 bar durch den Hochdruck-Homogenisierer gefördert. Es ergeben sich 500 g dünnflüssige, orange-weissliche Emulsion. 1 g homogenisierte Emulsionslösung wird mit 19 g Wasser verdünnt (1:20); Viskosität: 0.98 mPa · s; Partikelgrössenverteilung: Peak 1: d(H) = 82 nm - 4.9%-Vol. (PCS-Messung); Peak 2: d(H) = 631 nm - 95.1%-Vol.; Peak 3: d(H) = Kein Signal vorhanden. Bei der CAS-pH-Titration mit 0.01 N Natriumhydroxid wurde ein isolelektrischer Punkt von pH = 6.6 gefunden.

Zusammenfassung: Die Produktion der Emulsion verlief erfolgreich, es konnte eine dünnflüssige, orange-weissliche Emulsion mit β-Carotin als Pseudowirkstoff hergestellt werden. Die Partikelgrössenverteilung ergibt Emulsionstropfen mit hydrodynamischen Durchmessern von 82 nm (4.9%-Vol.) und 631 nm (95.1%-Vol.). Die CAS-pH-Titrationen zeigen, dass das Strömungspotential der Emulsion im sauren pH-Bereich, d.h. unterhalb von pH = 6.0 grösser als +200 mV ist. Die Emulsion hat allerdings bei pH = 6.6 einen isoelektrischen Punkt, was darauf hindeutet, dass 0.4% N^{α}-Lauroyl-L-arginin-ethylester-monohydrochlorid (LAE) als kationischer Emulgator nicht ausreicht, wenn die Lecithinemulsion über den gesamten pH-Bereich kationisch sein soll.

### Emulsion E-032 - Kilo-Batch von kationischer Emulsion mit LAE/Glycerin, Lecithin und β-Carotin als Pseudowirkstoff

Basierend auf Rezeptur E-031, jedoch mit 0.8% LAE als kationischem Emulgator anstatt nur 0.4%. 0.8% ist die maximal zulässige Konzentration von LAE in Seifen und Antischuppen-Shampoos (gilt nicht für Produkte, die als Spray eingesetzt werden).

Herstellung: Wie E-031, mit 416.1 g Wasser vorgelegt und 24.0 g Aminat-G. Es ergeben sich 600 g dünnflüssige, karottenrote Emulsion; pH = 4.6. Homogenisation: Wie E-031. Es ergeben sich 500 g dünnflüssige, orange-weissliche Emulsion. 1 g homogenisierte Emulsionslösung wird mit 19 g Wasser verdünnt (1:20); Viskosität: 0.96 mPa · s; Partikelgrössenverteilung: Peak 1: d(H) = 126 nm - 8.3%-Vol. (PCS-Messung); Peak 2: d(H) = 827 nm - 80.6%-Vol.; Peak 3: d(H) = 5208 nm - 11.0%-Vol.

Eine Ladungstitration in 1:10 Verdünnung mit Charge Analysing System (CAS) mit anionischer Polyelektrolytlösung (0.001 N Poly(Vinyisulfonsäure-Natriumsalz) ergab eine gemessene Oberflächenladung von 12.05 µmol pos. Ladung/g Emulsion (+96.5 mC/g Emulsion).

Zusammenfassung: Die Produktion der Emulsion mit 0.8% LAE verlief erfolgreich. Es konnte eine dünnflüssige, orange-weissliche Emulsion mit β-Carotin als Pseudowirkstoff hergestellt werden. Die Partikelgrössenverteilung ergibt Emulsionstropfen mit hydrodynamischen Durchmessern von 126 nm (8.3%-Vol.), 827 nm (80.6%-Vol.) und 5208 nm (11.0%-Vol.). Gemäss Ladungstitration beträgt die Oberflächenladung der kationischen Emulsion 12.05 µmol pos. Ladung/g Emulsion. Die CAS-pH-Titrationen zeigen, dass das Strömungspotential der Emulsion über den gesamten pH-Bereich von 3.0 bis 10.0 stets grösser als +238 mV ist. Die Emulsion weist folglich in diesem pH-Bereich keinen isoelektrischen Punkt auf. 0.8% N^{α}-Lauroyl-L-arginin-ethylester-monohydrochlorid (LAE) als kationischer Emulgator genügt somit, um die Lecithinemulsion über den genannten pH-Bereich kationisch zu stellen. Dadurch kann die Beladung der Textilien auch in kalkhaltigem, leicht alkalischem Leitungswasser erfolgen, ohne dieses jeweils z.B. mit Zitronensäure leicht ansäuern zu müssen.

### Emulsion E-033 - 1.2 kg Batch von kationischer, wirkstofffreier Emulsion mit LAE/Glycerin und Lecithin

Herstellung analog zu E-032, jedoch ohne das β-Carotin als Pseudowirkstoff. Mit dieser kationischen Emulsion werden die ausgerüsteten Textilien für die Sorptions- und Desorptionsuntersuchungen beladen. Mit dieser kationischen Emulsion werden die ausgerüsteten Textilien für die Sorptions- und Desorptionsuntersuchungen beladen.

Die Produktion der wirkstofffreien Emulsion verlief erfolgreich, es konnte eine dünnflüssige, weiss-gelbliche Emulsion hergestellt werden. Die Partikelgrössenverteilung ergibt eine monodisperse Emulsionstropfengrösse mit einem hydrodynamischen Durchmesser von 303 nm. Gemäss Ladungstitration beträgt die Oberflächenladung der kationischen Emulsion 12.12 µmol pos. Ladung/g Emulsion. Die pH-Titrationen zeigen, dass das Strömungspotential der Emulsion über den gesamten pH-Bereich von 3.0 bis 10.0 stets grösser als +198 mV ist.

### E. Aufbringen der Wirkstoff-Emulsion auf die Ausrüstungsschicht; Sorption

### Versuch 1

0.2 g Emulsion E-021 werden in 6 ml Wasser verdünnt und vollständig auf 4.4 g ausgerüstetes Stoffmuster A-007 (mit mPEG-1000-Methacrylat) aufgesprüht. Die Probe wird nach vollständigem Trocknen extrahiert und methyliert. Die GC-Analyse ergibt 423.6 µg Palmitinsäure-methylester/g Ware, was 6 mg Nachtkerzenöl/g Ware entspricht.

### Versuch 2 (S-004)

Sorptionsversuch: In eine 100 ml-Schottflasche werden 39.2 g Wasser vorgelegt und 0.8 g kationische BTMS-Emulsion E-021 zugegeben. Anschliessend wird ein 4.0 g Stoffmuster A-009 in die Flasche gelegt, geschüttelt und während 16 h bei RT stehengelassen. Danach wird das Stoffmuster während 1 h bei 50 °C im Trockenofen getrocknet. Optisch ist ein ausziehen feststellbar.

Quantifizierung: In 2 ml-Probenvials werden mit einer Pinzette jeweils drei Mal 50 mg Stoffmuster S-004 eingewogen. Dann werden mit der Mikropipette jeweils 1000 µl INT-STD-01 (Toluol-Standardlösung mit 1 mg/ml Nonadecansäure-methylester) zum Extrahieren und jeweils 100 µl 5%-ige, methanolische meta-Katalysatorlösung dazu pipettiert. Die 2 ml-Probenvials werden verschlossen, 5 min auf der Schüttelmaschine extrahiert und während mindestens 30 min bei RT stehen gelassen. Zum Entfernen des Katalysators (Schutz der GC-Säule) werden den Mischungen jeweils 50 ± 5 mg Amberlyst 15 zugegeben und kurz geschüttelt. Dann wird zentrifugiert, die Überstände in GC-Vials überführt und die Gehaltsbestimmungen am GC/MS durchgeführt. Die GC-Analyse ergibt Nachtkerzenöl 267 µg/cm², was 35 mg Nachtkerzenöl/g Ware entspricht.

### Versuch 3

0.4 g Emulsion E-021 werden in 40 ml Wasser verdünnt. 4.0 g ausgerüstetes Stoffmuster A-009 (mit HEMA) werden eingetaucht und geschüttelt. Die Wirkstoffemulsion zieht sofort auf das Gewebe aus.

### Versuch 4 (S-005, S-006)

Sorptionsversuche von E-033 (LAE-Emulsion) auf A-084 (Ausrüstung mit P-044, mit 40% mPEG-350-Methacrylat und 20% 2-Ethylhexyl-acrylat) und auf A-088 (Ausrüstung mit P-067, mit 20% mPEG-350-Methacrylat und 40% 2-Ethylhexyl-acrylat) während 30 min zur Bestimmung der Beladbarkeit (Sorptionsvermögen). Die auf der Haut applizierten Mengen an Hautpflegeprodukten liegen in der Dermatologie typischerweise bei 2 mg/cm² mit einem Wirkstoffgehalt zwischen 1-3% und einer Kontaktzeit von normalerweise 16 Stunden. Dies wird bei den nachfolgenden Sorptionsversuchen berücksichtigt. Stoffmuster: A-084: Anionisches PA-Gewebe mit Poly(2-Acrylamido-2-methylpropan-natriumsulfonat-stat-2-Ethylhexyl-acrylat-stat-N-(Butoxymethyl)-acrylamid) mit P-044 nach einer Maschinenwäsche bei 60 °C während 50-55 min. A-088: Anionisches PA-Gewebe mit Poly(2-Acrylamido-2-methylpropan-natriumsulfonat-stat-2-Ethylhexyl-acrylat-stat-N-(Butoxymethyl)-acrylamid) mit P-067 nach einer Maschinenwäsche bei 60 °C während 50-55 min. PA-Gewebe: 100% PA-Gewebe (Trikotstoff) Fussenegger Textilveredelung GmbH, Charmeuse vorfixiert, Gewicht = 135 g/m², Breite = 140 cm, Farbe = 0109. Zur Probenvorbereitung werden die appretierten PA-Stoffmuster, nachdem diese einer Maschinenwäsche bei 60 °C während 50-55 min unterzogen wurden, auf ein Gewicht von jeweils 5.0 g (ca. 20x20 cm) zugeschnitten. In 150 ml-Metallbomben werden jeweils 71.0 g Wasser vorgelegt und jeweils 4.0 g kationische LAE-Emulsion E-033 zugegeben. Anschliessend werden jeweils 5.0 g Stoffmuster A-084 ( = > S-005), bzw. 5.0 g Stoffmuster A-088 ( = > S-006) in die Bomben gelegt, diese umgehend in einen Polymaten eingesetzt und derselbe bei 25 °C mit 50 U/min (Zyklus: 12 s rotieren, 3 s Pause, 12 s in Gegenrichtung rotieren, usw.) gestartet. Nach 30 min werden die Stoffmuster samt Flotte jeweils separat in der vorgängig mit Sprühreiniger, Schwamm, Waschlappen und Wasser gereinigten Wäscheschleuder bei RT mit 2800 U/min während exakt 1 min zentrifugiert und abschliessend bei RT an der Luft getrocknet. Der Blindwert wird auf dieselbe Weise angefertigt.

Es ergeben sich gravimetrisch folgende Beladungswerte mit Nachtkerzenöl (% des theoretischen Maximalwerts von 1 g): S-005-30min: 90%; S-006-30min: 91%; Blindwert: 8%.

Die offenbarten spezifischen Ausführungsformen sind nicht dazu geeignet, die vorliegende Erfindung in ihrem Umfang zu beschränken. Dem Fachmann ergeben sich aus der vorangehenden Beschreibung und den Zeichnungen verschiedene mögliche Abwandlungen und Modifikationen, zusätzlich zu den offenbarten Beispielen, die ebenfalls unter den Schutzbereich der Ansprüche fallen sollen.

## Patentansprüche

1. Polymerverbindung, umfassend ein Acrylsäure-Copolymer aus Acrylsäure-Derivaten und/oder Methacrylsäure-Derivaten, welches beinhaltet: a) mindestens ein Acrylsäure-Derivat und/oder Methacrylsäure-Derivat, substituiert mit einer Sulfonsäuregruppe; b) mindestens ein hydrophil substituiertes Acrylsäure-Derivat und/oder Methacrylsäure-Derivat; c) mindestens ein lipophil substituiertes Acrylsäure-Derivat und/oder Methacrylsäure-Derivat; und d) mindestens ein Acrylsäure-Derivat und/oder Methacrylsäure-Derivat, welches als Vernetzer wirkt.

2. Polymerverbindung nach Anspruch 1, **dadurch gekennzeichnet, dass** das Acrylsäure-Copolymer 2-Acryloyl-2-methyl-propansulfonsäure beinhaltet.

3. Polymerverbindung nach Anspruch 1 oder 2, **dadurch gekennzeichnet, dass** das Acrylsäure-Copolymer Ethyltriglykol-methacrylat, 2-Hydroxyethyl-methacrylat und/oder mPEG-methacrylat, insbesondere mPEG-1000-Methacrylat und/oder mPEG-350-Methacrylat, als hydrophiles Monomer beinhaltet.

4. Polymerverbindung nach einem der vorangehenden Ansprüche, **dadurch gekennzeichnet, dass** das Acrylsäure-Copolymer 2-Ethylhexyl-acrylat als lipophiles Monomer beinhaltet.

5. Polymerverbindung nach einem der vorangehenden Ansprüche, **dadurch gekennzeichnet, dass** das mindestens eine Vernetzer-Monomer des Acrylsäure-Copolymers ausgewählt ist aus einer Gruppe bestehend aus N-(Butoxymethyl)-acrylamid, N-(Methylol)-acrylamid, Glycidyl-methacrylat, p-EMKO-TDI-o-HEMA, EMKO-2-(N-(tert-Butyl){[(3-isocyanato-1, 5, 5-trimethylcyclohexyl)-methyl]amino}-carbonylamino)ethyl-methacrylat.

6. Polymerverbindung nach einem der vorangehenden Ansprüche, **dadurch gekennzeichnet, dass** die Polymerverbindung Polyethersulfone, Polyurethane, Polyesterurethane, Polyetherurethane, Polyamide oder Gemische davon enthält.

7. Ausrüstungsformulierung zur Ausrüstung eines textilen Produkts oder zur Beschichtung einer Wundauflage, **gekennzeichnet durch** eine Polymerverbindung nach einem der Ansprüche 1 bis 6.

8. Ausrüstungsschicht auf einem textilen Produkt oder einer Wundauflage, **gekennzeichnet durch** eine Polymerverbindung nach einem der Ansprüche 1 bis 6.

9. Textiles Produkt, **gekennzeichnet durch** eine Ausrüstung mit einer Polymerverbindung nach einem der Ansprüche 1 bis 6.

10. Textiles Produkt nach Anspruch 9, beladen mit einer Emulsion mit mindestens einer niedermolekularen Verbindung, wobei die mindestens eine niedermolekulare Verbindung in der dispergierten Phase der Emulsion enthalten ist, und wobei die Oberfläche der Partikel der dispergierten Phase eine positive Ladung aufweist.

11. Textiles Produkt nach Anspruch 10, wobei die Emulsion Lecithin, insbesondere Phosphatidylcholin-Lecithine, und/oder quartäre Ammoniumverbindungen mit einem oder zwei langkettigen lipophilen Resten, insbesondere Behenyltrimethylammonium, und/oder Ethyl-Nalpha-Lauroyl-L-Arginat/HCl als grenzflächenaktive Verbindung enthält.

12. Wundauflage, insbesondere Wundauflage eines Wundschnellverbandes, **gekennzeichnet durch** eine Ausrüstung mit einer Polymerverbindung nach einem der Ansprüche 1 bis 6.

13. Wundauflage nach Anspruch 12, beladen mit einer Emulsion mit mindestens einer niedermolekularen Verbindung, wobei die mindestens eine niedermolekulare Verbindung in der dispergierten Phase der Emulsion enthalten ist, und wobei die Oberfläche der Partikel der dispergierten Phase eine positive Ladung aufweist.

14. Wundauflage nach Anspruch 13, wobei die Emulsion Lecithin, insbesondere Phosphatidylcholin-Lecithine, und/oder quartäre Ammoniumverbindungen mit einem oder zwei langkettigen lipophilen Resten, insbesondere Behenyltrimethylammonium, und/oder Ethyl-Nalpha-Lauroyl-L-Arginat/HCl als grenzflächenaktive Verbindung enthält.

15. Kit, umfassend ein textiles Produkt nach Anspruch 9, und eine Emulsion mit mindestens einer niedermolekularen Verbindung, wobei die mindestens eine niedermolekulare Verbindung in der dispergierten Phase der Emulsion enthalten ist, und wobei die Oberfläche der Partikel der dispergierten Phase eine positive Ladung aufweist.

16. Kit nach Anspruch 15, wobei die Emulsion Lecithin, insbesondere Phosphatidylcholin-Lecithine, und/oder quartäre Ammoniumverbindungen mit einem oder zwei langkettigen lipophilen Resten, insbesondere Behenyltrimethylammonium, und/oder Ethyl-Nalpha-Lauroyl-L-Arginat/HCl als grenzflächenaktive Verbindung enthält.

## Claims

1. Polymer compound, comprising an acrylic acid copolymer composed of acrylic acid derivatives and/or methacrylic acid derivatives, containing: a) at least one acrylic acid derivative and/or methacrylic acid derivative substituted with a sulfonic acid group; b) at least one hydrophilically substituted acrylic acid derivative and/or methacrylic acid derivative; c) at least one lipophilically substituted acrylic acid derivative and/or methacrylic acid derivative; and d) at least one acrylic acid derivative and/or methacrylic acid derivative which acts as a crosslinking agent.

2. Polymer compound according to Claim 1, **characterized in that** the acrylic acid copolymer contains 2-acryloyl-2-methylpropanesulfonic acid.

3. Polymer compound according to Claim 1 or 2, **characterized in that** the acrylic acid copolymer contains ethyl triglycol methacrylate, 2-hydroxyethyl methacrylate, and/or mPEG methacrylate, in particular mPEG 1000 methacrylate and/or mPEG 350 methacrylate, as hydrophilic monomer.

4. Polymer compound according to one of Claims 1 through 3, **characterized in that** the acrylic acid copolymer contains 2-ethylhexyl acrylate as lipophilic monomer.

5. Polymer compound according to one of Claims 1 through 4, **characterized in that** the at least one crosslinking monomer of the acrylic acid copolymer is selected from a group composed of *N*-(butoxymethyl)acrylamide, *N*-(methylol)acrylamide, glycidyl methacrylate, *p*-EMKO-TDI-*o*-HEMA, and EMKO-2-(*N*-(*tert*-butyl){[(3-isocyanato-1,5,5-trimethylcyclohexyl)methyl]amino}carbonylamino)ethyl methacrylate.

6. Polymer compound according to one of Claims 1 through 5, **characterized in that** the polymer compound contains polyethersulfones, polyurethanes, polyester urethanes, polyether urethanes, polyamides, or mixtures thereof.

7. Finishing formulation for finishing a textile product or for coating a wound dressing, **characterized by** a polymer compound according to one of Claims 1 through 6.

8. Finishing layer on a textile product or a wound dressing, **characterized by** a polymer compound according to one of Claims 1 through 6.

9. Textile product, **characterized by** a finish containing a polymer compound according to one of Claims 1 through 6.

10. Textile product according to Claim 9, loaded with an emulsion with at least one low-molecular compound, the at least one low-molecular compound being contained in the dispersed phase of the emulsion, wherein the surface of the particles of the dispersed phase has a positive charge.

11. Textile product according to Claim 10, wherein the emulsion contains lecithin, in particular phosphatidylcholine lecithins, and/or quaternary ammonium compounds containing one or two long-chain lipophilic radicals, in particular behenyltrimethylammonium, and/or ethyl-N-alpha-lauroyl-L-arginate/HCl as surface-active compound.

12. Wound dressing, in particular a wound dressing of an adhesive bandage, **characterized by** a finish containing a polymer compound according to one of Claims 1 through 6.

13. Wound dressing according to Claim 12, loaded with an emulsion with at least one low-molecular compound, the at least one low-molecular compound being contained in the dispersed phase of the emulsion, wherein the surface of the particles of the dispersed phase has a positive charge.

14. Wound dressing according to Claim 13, wherein the emulsion contains lecithin, in particular phosphatidylcholine lecithins, and/or quaternary ammonium compounds containing one or two long-chain lipophilic substituents, in particular behenyltrimethylammonium, and/or ethyl-N-alpha-lauroyl-L-arginate/HCl as surface-active compound.

15. Kit comprising a textile product according to Claim 9, and an emulsion with at least one low-molecular compound, the at least one low-molecular compound being contained in the dispersed phase of the emulsion, wherein the surface of the particles of the dispersed phase has a positive charge.

16. Kit according to Claim 15, wherein the emulsion contains lecithin, in particular phosphatidylcholine lecithins, and/or quaternary ammonium compounds containing one or two long-chain lipophilic substituents, in particular behenyltrimethylammonium, and/or ethyl-N-alpha-lauroyl-L-arginate/HCl as surface-active compound.

## Revendications

1. Composé polymère, comprenant un copolymère d'acide acrylique à base de dérivés d'acide acrylique et/ou de dérivés d'acide méthacrylique, qui comporte : a) au moins un dérivé d'acide acrylique et/ou un dérivé d'acide méthacrylique, substitué(s) par un groupe d'acide sulfonique; b) au moins un dérivé d'acide acrylique et/ou un dérivé d'acide méthacrylique à substitution hydrophile; c) au moins un dérivé d'acide acrylique et/ou un dérivé d'acide méthacrylique à substitution lipophile; et d) au moins un dérivé d'acide acrylique et/ou un dérivé d'acide méthacrylique, qui agit en tant qu'agent de réticulation.

2. Composé polymère selon la revendication 1, **caractérisé en ce que** le copolymère d'acide acrylique comporte de l'acide 2-acryloyl-2-méthyl-propane-sulfonique.

3. Composé polymère selon la revendication 1 ou 2, **caractérisé en ce que** le copolymère d'acide acrylique comporte en tant que monomère hydrophile du méthacrylate d'éthyltriglycol, du méthacrylate de 2-hydroxyéthyle et/ou du méthacrylate de mPEG, en particulier du méthacrylate de mPEG-1000 et/ou du méthacrylate de mPEG-350.

4. Composé polymère selon l'une quelconque des revendications précédentes, **caractérisé en ce que** le copolymère d'acide acrylique comporte en tant que monomère lipophile de l'acrylate de 2-éthylhexyle.

5. Composé polymère selon l'une quelconque des revendications précédentes, **caractérisé en ce que** ledit au moins un monomère-agent de réticulation du copolymère d'acide acrylique est choisi dans un groupe constitué par le N-(butoxyméthyl)-acrylamide, le N-(méthylol)-acrylamide, le méthacrylate de glycidyle, le p-EMKO-TDI-o-HEMA, le méthacrylate d'EMKO-2-(N-tert-butyl){[(3-isocyanato-1,5,5-triméthylcyclohexyl)-méthyl]amino}carbonylamino)éthyle.

6. Composé polymère selon l'une quelconque des revendications précédentes, **caractérisé en ce que** le composé polymère comprend des polyéthersulfones, polyuréthanes, polyesteruréthanes, polyétheruréthanes, polyamides ou des mélanges de ceux-ci.

7. Composition d'apprêt pour l'apprêtage d'un produit textile ou pour le revêtement d'une compresse, **caractérisée par** un composé polymère selon l'une quelconque des revendications 1 à 6.

8. Couche d'apprêt sur un produit textile ou une compresse, **caractérisée par** un composé polymère selon l'une quelconque des revendications 1 à 6.

9. Produit textile, **caractérisé par** un apprêt comportant un composé polymère selon l'une quelconque des revendications 1 à 6.

10. Produit textile selon la revendication 9, chargé d'une émulsion comportant au moins un composé de faible masse moléculaire, ledit au moins un composé de faible masse moléculaire étant contenu dans la phase dispersée de l'émulsion, et la surface des particules de la phase dispersée présentant une charge positive.

11. Produit textile selon la revendication 10, dans lequel l'émulsion contient de la lécithine, en particulier des phosphatidylcholine-lécithines, et/ou des composés d'ammonium quaternaire comportant un ou deux radicaux lipophiles à longue chaîne, en particulier du béhényltriméthylammonium, et/ou du N-alpha-lauroyl-L-alginate d'éthyle/HCl en tant que composé tensioactif.

12. Pansement, en particulier pansement d'un pansement rapide, **caractérisée par** un apprêt comportant un composé polymère selon l'une quelconque des revendications 1 à 6.

13. Pansement selon la revendication 12, chargée d'une émulsion comportant au moins un composé de faible masse moléculaire, ledit au moins un composé de faible masse moléculaire étant contenu dans la phase dispersée de l'émulsion, et la surface des particules de la phase dispersée présentant une charge positive.

14. Pansement selon la revendication 13, dans laquelle l'émulsion contient de la lécithine, en particulier des phosphatidylcholine-lécithines, et/ou des composés d'ammonium quaternaire comportant un ou deux radicaux lipophiles à longue chaîne, en particulier du béhényltriméthylammonium, et/ou du N-alpha-lauroyl-L-alginate d'éthyle/HCl en tant que composé tensioactif.

15. Nécessaire, comprenant un produit textile selon la revendication 9, et une émulsion comportant au moins un composé de faible masse moléculaire, ledit au moins un composé de faible masse moléculaire étant contenu dans la phase dispersée de l'émulsion, et la surface des particules de la phase dispersée présentant une charge positive.

16. Nécessaire selon la revendication 15, dans lequel l'émulsion contient de la lécithine, en particulier des phosphatidylcholine-lécithines, et/ou des composés d'ammonium quaternaire comportant un ou deux radicaux lipophiles à longue chaîne, en particulier du béhényltriméthylammonium, et/ou du N-alpha-lauroyl-L-alginate d'éthyle/HCl en tant que composé tensioactif.
